# EUROPEAN PATENT APPLICATION

(11) **EP 4 707 356 A1**
(43) Date of publication of application: **11.03.2026**
(21) Application number: 25196624.8
(22) Date of filing: 19.08.2025
(51) Int. Cl.: C09K 11/06, C07D 471/04, H10K 50/16, H10K 50/17, H10K 85/60

(54) **HETEROCYCLIC COMPOUND AND ORGANIC LIGHT EMITTING DEVICE COMPRISING SAME**

(30) Priority: 09.09.2024 KR 20240122364
(71) Applicant: LT Materials Co., Ltd., Yongin-City 17118 (KR)
(72) Inventor: LEE, So-Yeon, 17118 Yongin-City (KR); JEONG, Won-Jang, 17118 Yongin-City (KR); LEE, Dong-Jin, 17118 Yongin-City (KR); KIM, Dong-Jun, 17118 Yongin-City (KR); CHOI, Dae-Hyuk, 17118 Yongin-City (KR)
(74) Representative: Meissner Bolte Partnerschaft mbB

(57) **Abstract**

Disclosed are a heterocyclic compound of Chemical Formula 1 and an organic light emitting device including the same.

## Description

### CROSS-REFERENCE TO RELATED APPLICATIONS

This application claims priority to and the benefit of Korean Patent Application No. 10-2024-0122364 filed in the Korean Intellectual Property Office on September 09, 2024 the entire contents of which are incorporated herein by reference.

### TECHNICAL FIELD

The present specification relates to a heterocyclic compound and an organic light emitting device including the same.

### BACKGROUND ART

An electroluminescence device is a kind of selfemitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multiple layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a hostdopant-based light emitting layer. In addition, as a material for the organic thin film, it is also possible to use a compound, which may play a role such as a hole injection, hole transport, electron blocking, hole blocking, electron transport or electron injection.

In order to improve the performance, service life, or efficiency of the organic light emitting device, there is a continuous need for developing a material for an organic thin film.

### [Related Art Documents]

### [Patent Documents]

(Patent Document 1) US Patent No. 4,356,429

### SUMMARY OF THE INVENTION

The present specification has been made in an effort to provide a heterocyclic compound and an organic light emitting device including the same.

An exemplary embodiment of the present invention provides a heterocyclic compound of the following Chemical Formula 1.

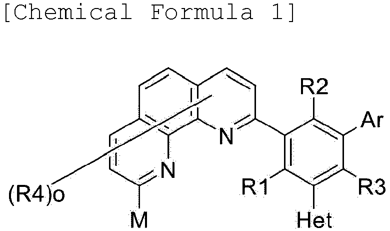

In Chemical Formula 1,
M is a substituted or unsubstituted C1 to C60 alkyl group; a substituted or unsubstituted C6 to C60 aryl group; or a substituted or unsubstituted C2 to C60 heteroaryl group,
Ar is hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
R1 to R3 are each independently hydrogen; deuterium; a halogen group; a cyano group; a substituted or unsubstituted C1 to C60 alkyl group; a substituted or unsubstituted C3 to C60 cycloalkyl group; or a substituted or unsubstituted C2 to C60 heterocycloalkyl group,
R4 is hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
o is an integer from 0 to 5, and when o is 2 or higher, R4's are the same as or different from each other,
Het is a group represented by any one of the following Chemical Formulae H-1 to H-3, 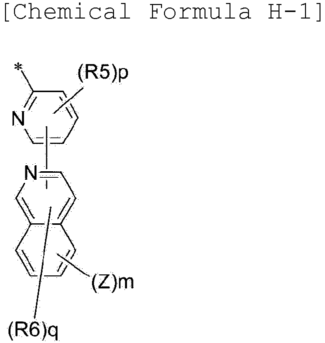 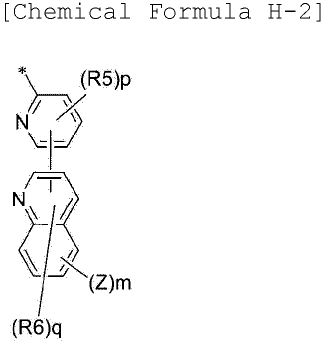 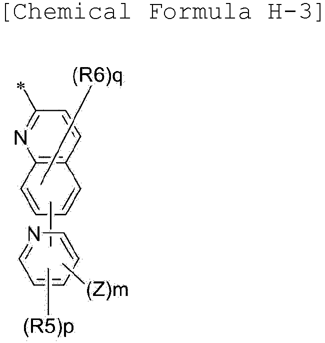 in Chemical Formulae H-1 to H-3,
   Z is hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
   m is an integer from 0 to 4, and when m is 2 or higher, Z's are the same as or different from each other,
   R5 and R6 are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, and
   p is an integer from 1 to 3, q is an integer from 1 to 5, and when p and q are each 2 or higher, R5 and R6 are the same as or different from each other.

Another exemplary embodiment provides an organic light emitting device including: a first electrode; a second electrode; and an organic material layer having one or more layers provided between the first electrode and the second electrode, in which one or more layers of the organic material layer include one or more of the compounds.

When used in an organic light emitting device, the heterocyclic compound described in the present specification can lower the driving voltage of the device, improve the light emitting efficiency, and improve the service life characteristics of the device.

Specifically, the heterocyclic compound includes a phenanthroline having a substituent at a specific position as a first substituent and a linking group of pyridine and quinoline as a second substituent, around a benzene ring, with the first and second substituents being substituted at the meta position of the benzene ring. When substituted at the meta position as described above, the amine of quinoline or pyridine forms a strong coordinate bond with a metal, increasing the bond energy with a metal to be doped (Li or Yb).

In addition, the linking group of pyridine and quinoline, which is the second substituent, is bonded to a benzene ring only at a specific position, a linking structure between pyridine and quinoline is also specified, and when pyridine/quinoline is linked to the quinoline/pyridine substituted at the meta position as described above, the amine of the pyridine/quinoline can additionally bond to the metal, resulting in a stronger bond. As a result, excellent effects can be obtained in terms of drive voltage and efficiency.

A heterocyclic compound including the structural characteristics has appropriate electron transport capabilities, is structurally stable, and can efficiently transfer electrons. Therefore, when the heterocyclic compound of the present invention is used as a material for an organic material layer (particularly, an electron transport layer or a charge generation layer) of an organic light emitting device, the performance of the organic light emitting device can be improved.

### BRIEF DESCRIPTION OF THE DRAWINGS

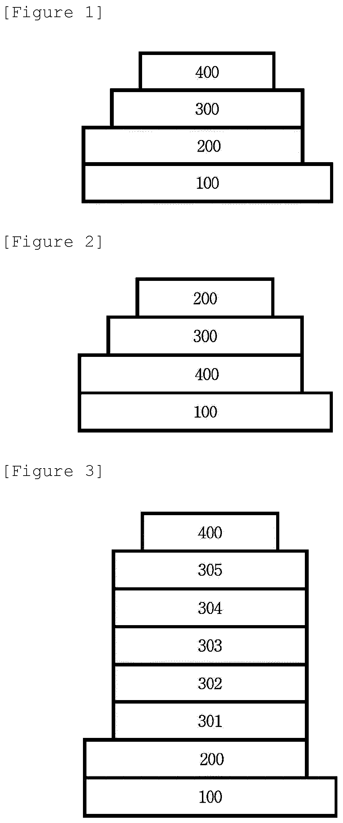

FIGS. 1 to 4 are views each exemplarily illustrating a stacking structure of an organic light emitting device according to an exemplary embodiment of the present specification.

### DETAILED DESCRIPTION

Hereinafter, the present specification will be described in more detail.

When one part "includes" one constituent element in the present specification, unless otherwise specifically described, this does not mean that another constituent element is excluded, but means that another constituent element may be further included.

In the present specification, "N to N'" means N or more and N' or less.

In the present specification, Cn (n: an integer of 1 or higher) means the number of carbon atoms. For example, C1 to C60 means 1 to 60 carbon atoms.

In the present specification, 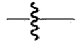 or *- of a chemical formula means a position to which a constituent element is bonded.

The term "substitution" means that a hydrogen atom bonded to a carbon atom or nitrogen atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, "substituted or unsubstituted" means being unsubstituted or substituted with one or more substituents selected from the group consisting of deuterium; a halogen group; a cyano group (-CN); a C1 to C60 alkyl group; a C2 to C60 alkenyl group; a C2 to C60 alkynyl group; a C1 to C60 haloalkyl group; a C1 to C60 alkoxy group; a C6 to C60 aryloxy group; a C1 to C60 alkylthioxy group; a C6 to C60 arylthioxy group; a C1 to C60 alkylsulfoxy group; a C6 to C60 arylsulfoxy group; a C3 to C60 cycloalkyl group; a C2 to C60 heterocycloalkyl group; a C6 to C60 aryl group; a C2 to C60 heteroaryl group; a silyl group; a phosphine oxide group; and an amine group, or a substituent to which two or more substituents selected among the exemplified substituents are linked.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the deuterium content may be 0% to 100%, and the deuterium content may be expressed as a deuterium substitution rate.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or ²H.

In an exemplary embodiment of the present application, the isotope means an atom with the same atomic number (Z), but different mass numbers (A), and may also be interpreted as an element which has the same number of protons, but different number of neutrons.

In an exemplary embodiment of the present application, when the total number of substituents of a basic compound is defined as T1 and the number of specific substituents among the substituents is defined as T2, the substitution rate T% of the specific substituent may be defined as T2/T1×100 = T%.

That is, in an example, a deuterium substitution rate of 20% in a phenyl group represented by 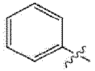 may be represented by 20% when the total number of substituents that the phenyl group can have is 5 (T1 in the formula) and the number of deuteriums among the substituents is 1 (T2 in the formula). That is, a deuterium substitution rate of 20% in the phenyl group may be represented by the following structural formula.

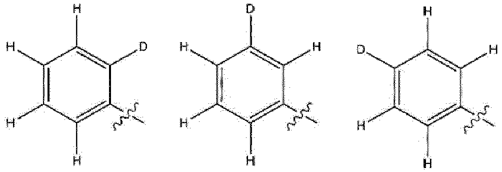

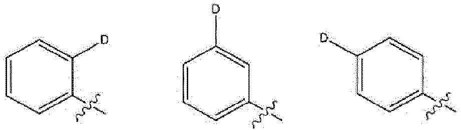

Further, in an exemplary embodiment of the present application, "a phenyl group having a deuterium substitution rate of 0%" may mean a phenyl group that does not include a deuterium atom as a substituent, that is, has five hydrogen atoms.

In the present specification, the halogen may be fluorine, chlorine, bromine or iodine.

In the present specification, an alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, an alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, an alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, a haloalkyl group means an alkyl group substituted with a halogen group, and specific examples thereof include -CF₃, -CF₂CF₃, and the like, but are not limited thereto.

In the present specification, an alkoxy group is represented by -O(R101), and the above-described examples of the alkyl group may be applied to R101.

In the present specification, an aryloxy group is represented by -O(R102), and the above-described examples of the aryl group may be applied to R102.

In the present specification, an alkylthioxy group is represented by -S(R103), and the above-described examples of the alkyl group may be applied to R103.

In the present specification, an arylthioxy group is represented by -S(R104), and the above-described examples of the aryl group may be applied to R104.

In the present specification, an alkylsulfoxy group is represented by -S(=0)₂(R105), and the above-described examples of the alkyl group may be applied to R105.

In the present specification, an arylsulfoxy group is represented by -S(=0)₂(R106), and the above-described examples of the aryl group may be applied to R106.

In the present specification, a cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, a heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, an aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, the terphenyl group may be selected from the following structures.

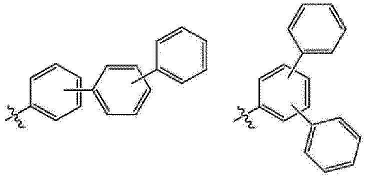

In the present specification, the fluorenyl group may be substituted, and adjacent substituents may be bonded to each other to form a ring. That is, a substituted or unsubstituted fluorenyl group may also include a spirobifluorenyl group.

When the fluorenyl group is substituted, the fluorenyl group may be, for example, the following structures, and any carbon in the following structure may be bonded to another structure, but is not limited thereto.

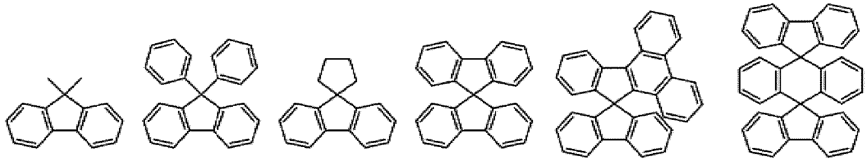

In the present specification, a heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridine group, a pyrrole group, a pyrimidine group, a pyridazine group, a furan group, a thiophene group, an imidazole group, a pyrazole group, an oxazole group, an isoxazole group, a thiazole group, an isothiazole group, a triazole group, a furazan group, an oxadiazole group, a thiadiazole group, a dithiazole group, a tetrazolyl group, a pyran group, a thiopyran group, a diazine group, an oxazine group, a thiazine group, a dioxin group, a triazine group, a tetrazine group, a quinoline group, an isoquinoline group, a quinazoline group, an isoquinazoline group, a quinozoline group, a naphthyridine group, an acridine group, a phenanthridine group, an imidazopyridine group, a diazanaphthalene group, a triazaindene group, an indole group, an indolizine group, a benzothiazole group, a benzoxazole group, a benzimidazole group, a benzothiophene group, a benzofuran group, a dibenzothiophene group, a dibenzofuran group, a carbazole group, a benzocarbazole group, a dibenzocarbazole group, a phenazine group, a dibenzosilole group, spirobi(dibenzosilole), a dihydrophenazine group, a phenoxazine group, a phenanthridine group, a thienyl group, an indolo[2,3-a]carbazole group, an indolo[2,3-b]carbazole group, an indoline group, a 10,11-dihydrodibenzo[b,f]azepine group, a 9,10-dihydroacridine group, a phenanthrazine group, a phenothiazine group, a phthalazine group, a phenanthroline group, a naphthobenzofuran group, a naphthobenzothiophene group, a benzo[c][1,2,5]thiadiazole group, a 2,3-dihydrobenzo[b]thiophene group, a 2,3-dihydrobenzofuran group, a 5,10-dihydrodibenzo[b,e][1,4]azasiline group, a pyrazolo[1,5-c]quinazoline group, a pyrido[1,2-b]indazole group, a pyrido[1,2-a]imidazo[1,2-e]indoline group, a 5,11-dihydroindeno[1,2-b]carbazole group, and the like, but are not limited thereto.

In the present specification, a benzocarbazole group may be any one of the following structures, and a nitrogen or any carbon in the following structures is bonded to another structure. When any carbon is bonded to another structure, the nitrogen may be bonded to a hydrogen or may have an additional substituent.

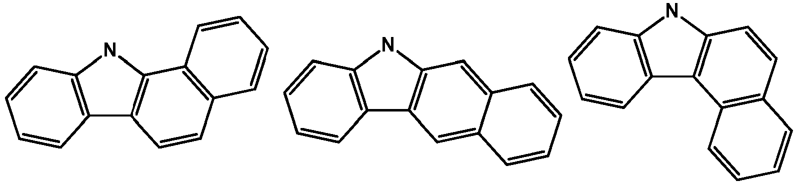

In the present specification, a dibenzocarbazole group may be any one of the following structures, and a nitrogen or any carbon in the following structures is bonded to another structure. When any carbon is bonded to another structure, the nitrogen may be bonded to a hydrogen or may have an additional substituent.

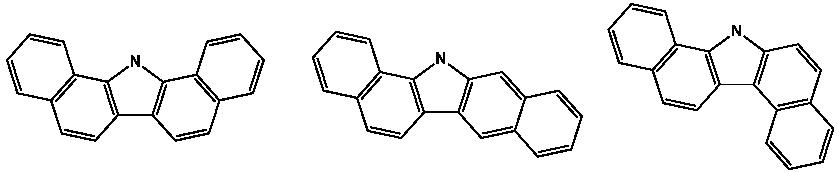

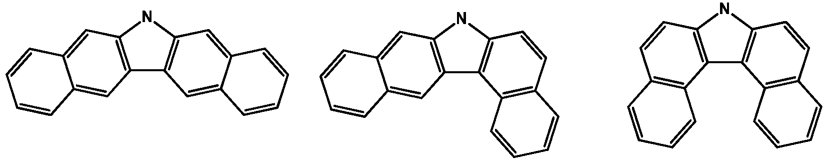

In the present specification, when the substituent is a carbazole group, a benzocarbazole group, or a dibenzocarbazole group, it means that the nitrogen or carbon of the carbazole group, benzocarbazole group, or dibenzocarbazole group is bonded to another structure.

In the present specification, when a carbazole group, a benzocarbazole group, or a dibenzocarbazole group is substituted, it means that an additional substituent is substituted on a nitrogen or carbon of the carbazole group, the benzocarbazole group, or the dibenzocarbazole group.

In the present specification, a naphthobenzofuran group may be any one of the following structures, and any carbon in the following structures is bonded to another structure.

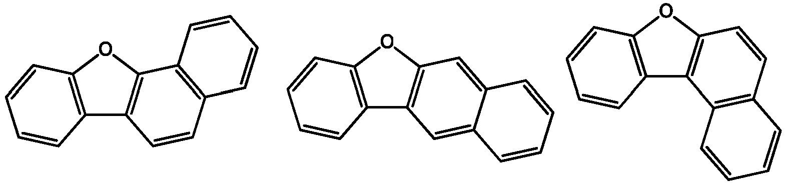

In the present specification, a naphthobenzothiophene group may be any one of the following structures, and any carbon in the following structures is bonded to another structure.

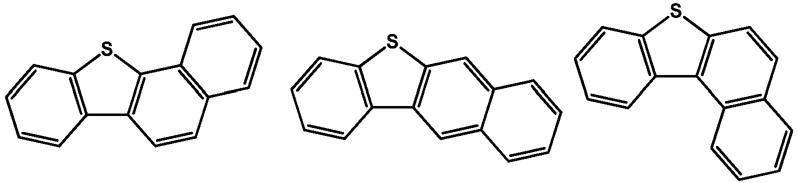

In the present specification, a silyl group includes Si and is a substituent to which the Si atom is directly linked as a radical, and is represented by - Si(R107) (R108) (R109), and R107 to R109 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group.

The silyl group may include an alkylsilyl group, an arylsilyl group, a heteroarylsilyl group, an alkylarylsilyl group, an arylheteroarylsilyl group, and the like, according to the substituent bonded to the Si element. An alkylsilyl group, an arylsilyl group, or a heteroarylsilyl group means that an alkyl group, an aryl group, or a heteroaryl group is substituted with the Si element of a silyl group, respectively, an alkylarylsilyl group means that an alkyl group and an aryl group are substituted with the Si element of a silyl group, and an arylheteroarylsilyl group means that an aryl group and a heteroaryl group are substituted with the Si element of a silyl group.

In the present , a triarylsilyl group means a silyl group substituted with three aryl groups. The number of carbon atoms of the aryl group may be 6 to 60, 6 to 30, or 6 to 20, and the number of carbon atoms of the triarylsilyl group may be 18 to 180, 18 to 90, or 18 to 60.

Specific examples of the silyl group include the following structures, but are not limited thereto. 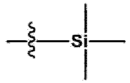 (A trimethylsilyl group), 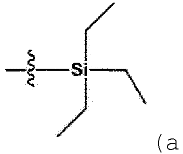 triethylsilyl group), 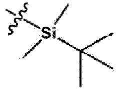 (a t-butyldimethylsilyl group), 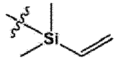 (a vinyldimethylsilyl group), 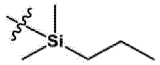 (a propyldimethylsilyl group), 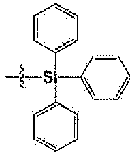 (a triphehylsilyl group), 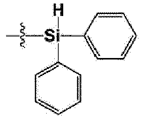 (a diphenylsilyl group), and 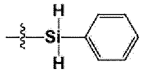 (a phenylsilyl group)

In the present specification, a phosphine oxide group is represented by -P(=O)(R110) (R111), and R110 and R111 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. Specifically, the phosphine oxide group may be substituted with an alkyl group or an aryl group, and the above-described example may be applied to the alkyl group and the aryl group. Examples of the phosphine oxide group include a dimethylphosphine oxide group, a diphenylphosphine oxide group, dinaphthylphosphine oxide, and the like, but are not limited thereto.

In the present specification, an amine group is represented by -N(R112) (R113), and R112 and R113 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, the above-described description of the aryl group may be applied to an arylene group except for a divalent arylene group.

In the present specification, the above-described description of the heteroaryl group may be applied to a heteroarylene group except for a divalent heteroarylene group.

An exemplary embodiment of the present specification provides a heterocyclic compound of the following Chemical Formula 1.

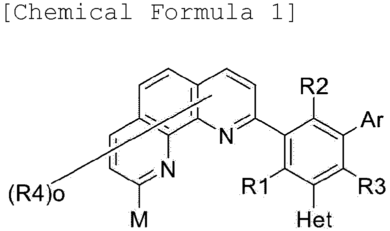

In Chemical Formula 1,
the definition of each substituent is as described above.

The heterocyclic compound according to an exemplary embodiment of the present specification is characterized by including a phenanthroline having a substituent at a specific position as a first substituent and a linking group of pyridine and quinoline as a second substituent, around a benzene ring, with the first and second substituents being substituted at the meta position of the benzene ring.

In the heterocyclic compound having the structure, the amine of quinoline and pyridine form a strong coordinate bond with a metal, increasing the bond energy with a metal to be doped (Li or Yb), and as a result, it is possible to obtain excellent effects in terms of driving voltage and efficiency. The heterocyclic compound also has appropriate electron transport capabilities, is structurally stable, and may efficiently transfer electrons.

In an exemplary embodiment of the present specification, Chemical Formula 1 may be represented by any one of the following Chemical Formulae 1-1 to 1-3.

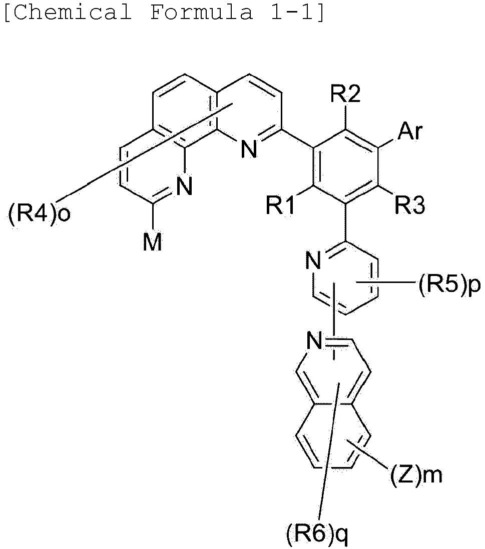

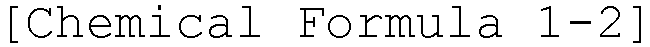

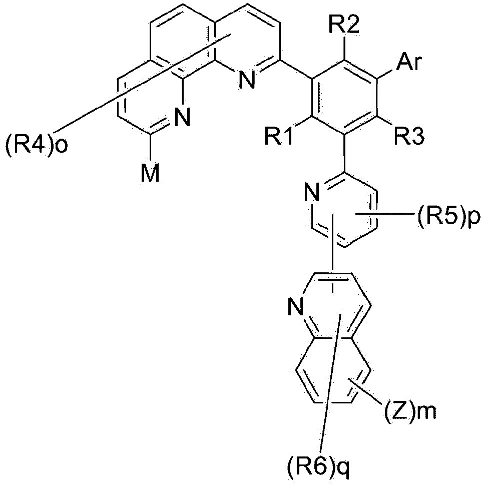

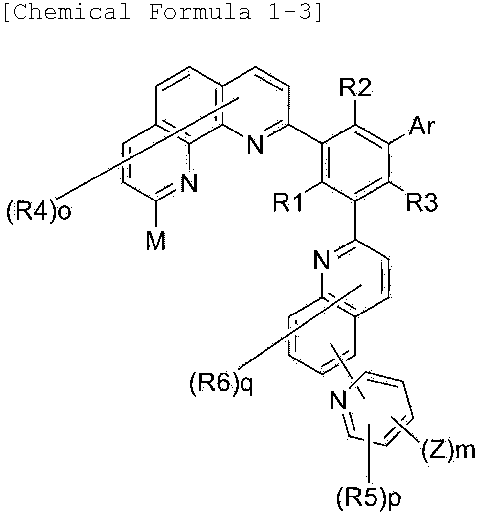

In Chemical Formulae 1-1 to 1-3,
the definition of each substituent is the same as the definition in Chemical Formula 1.

In an exemplary embodiment of the present specification, Chemical Formula 1 may be represented by the following Chemical Formula 1-1-1, 1-1-2, 1-2-1, or 1-3-1.

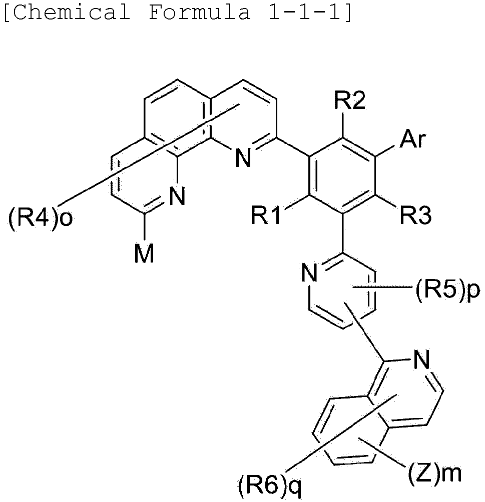

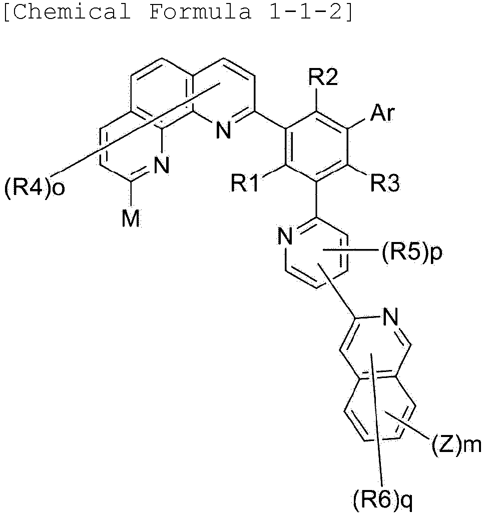

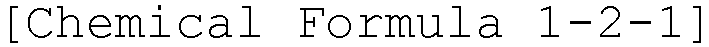

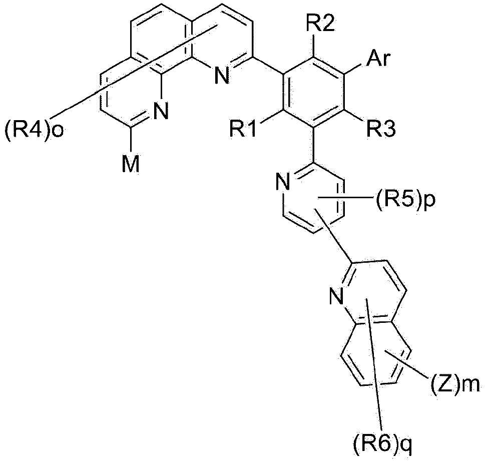

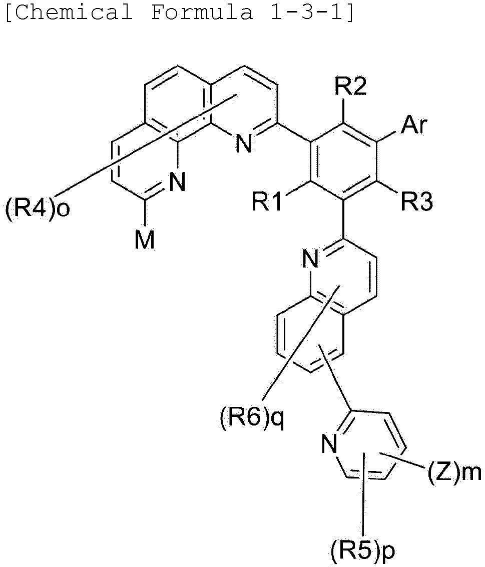

In Chemical Formulae 1-1-1, 1-1-2, 1-2-1, and 1-3-1, the definition of each substituent is the same as the definition in Chemical Formula 1.

In an exemplary embodiment of the present specification, Chemical Formula 1-3-1 may be represented by the following Chemical Formula 1-3-A or 1-3-B.

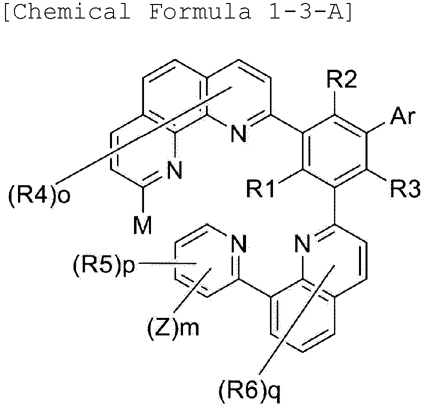

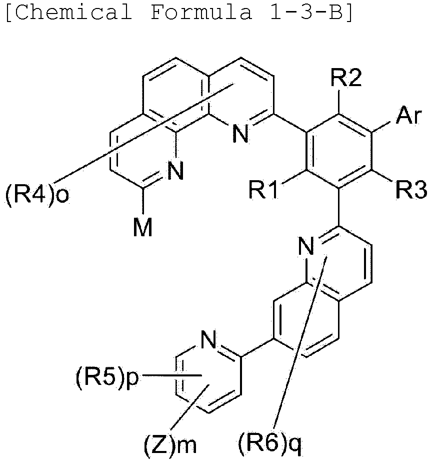

In Chemical Formulae 1-3-A and 1-3-B,
the definition of each substituent is the same as the definition in Chemical Formula 1.

In an exemplary embodiment of the present specification, M is a substituted or unsubstituted C1 to C60 alkyl group; a substituted or unsubstituted C6 to C60 aryl group; or a substituted or unsubstituted C2 to C60 heteroaryl group.

In an exemplary embodiment of the present specification, M may be a substituted or unsubstituted C1 to C40 alkyl group; a substituted or unsubstituted C6 to C40 aryl group; or a substituted or unsubstituted C2 to C40 heteroaryl group.

In an exemplary embodiment of the present specification, M may be a substituted or unsubstituted C1 to C20 alkyl group; a substituted or unsubstituted C6 to C20 aryl group; or a substituted or unsubstituted C2 to C20 heteroaryl group.

In an exemplary embodiment of the present specification, M may be a substituted or unsubstituted C1 to C10 alkyl group; a substituted or unsubstituted C6 to C20 aryl group; or a substituted or unsubstituted C2 to C20 heteroaryl group.

In an exemplary embodiment of the present specification, M may be a substituted or unsubstituted C1 to C10 alkyl group; a substituted or unsubstituted C6 to C20 aryl group; or a substituted or unsubstituted C2 to C20 heteroaryl group including N.

In an exemplary embodiment of the present specification, M may be a substituted or unsubstituted C1 to C10 alkyl group; a substituted or unsubstituted C6 to C20 aryl group; or a substituted or unsubstituted C2 to C20 heteroaryl group including a C=N bond.

In an exemplary embodiment of the present specification, M may be a substituted or unsubstituted tert-butyl group; a substituted or unsubstituted phenyl group; or a substituted or unsubstituted pyridine group.

In an exemplary embodiment of the present specification, M may be a C1 to C40 alkyl group unsubstituted or substituted with deuterium; a C6 to C40 aryl group unsubstituted or substituted with deuterium, an alkyl group, or an alkyl group substituted with deuterium; or a C2 to C40 heteroaryl group unsubstituted or substituted with deuterium.

In an exemplary embodiment of the present specification, M may be a C1 to C20 alkyl group unsubstituted or substituted with deuterium; a C6 to C20 aryl group unsubstituted or substituted with deuterium, an alkyl group, or an alkyl group substituted with deuterium; or a C2 to C20 heteroaryl group unsubstituted or substituted with deuterium.

In an exemplary embodiment of the present specification, M may be a C1 to C10 alkyl group unsubstituted or substituted with deuterium; a C6 to C20 aryl group unsubstituted or substituted with deuterium, an alkyl group, or an alkyl group substituted with deuterium; or a C2 to C20 heteroaryl group unsubstituted or substituted with deuterium.

In an exemplary embodiment of the present specification, M may be a C1 to C10 alkyl group unsubstituted or substituted with deuterium; a C6 to C20 aryl group unsubstituted or substituted with deuterium, an alkyl group, or an alkyl group substituted with deuterium; or a C2 to C20 heteroaryl group unsubstituted or substituted with deuterium and including N.

In an exemplary embodiment of the present specification, M may be a C1 to C10 alkyl group unsubstituted or substituted with deuterium; a C6 to C20 aryl group unsubstituted or substituted with deuterium, an alkyl group, or an alkyl group substituted with deuterium; or a C2 to C20 heteroaryl group unsubstituted or substituted with deuterium and including a C=N bond.

In an exemplary embodiment of the present specification, M may be a tert-butyl group unsubstituted or substituted with deuterium; a phenyl group unsubstituted or substituted with deuterium, a tert-butyl group, or a tert-butyl group substituted with deuterium; or a pyridine group unsubstituted or substituted with deuterium.

In an exemplary embodiment of the present specification, Ar is hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group.

In an exemplary embodiment of the present specification, Ar may be hydrogen; deuterium; a halogen group; a cyano group; a substituted or unsubstituted C1 to C40 alkyl group; a substituted or unsubstituted C6 to C40 aryl group; or a substituted or unsubstituted C2 to C40 heteroaryl group.

In an exemplary embodiment of the present specification, Ar may be hydrogen; deuterium; a halogen group; a cyano group; a substituted or unsubstituted C1 to C20 alkyl group; a substituted or unsubstituted C6 to C20 aryl group; or a substituted or unsubstituted C2 to C20 heteroaryl group.

In an exemplary embodiment of the present specification, Ar may be hydrogen; deuterium; a substituted or unsubstituted C1 to C10 alkyl group; a substituted or unsubstituted C6 to C20 aryl group; or a substituted or unsubstituted C2 to C20 heteroaryl group.

In an exemplary embodiment of the present specification, Ar may be hydrogen; deuterium; a substituted or unsubstituted C1 to C10 alkyl group; a substituted or unsubstituted C6 to C20 aryl group; or a substituted or unsubstituted C2 to C20 heteroaryl group including O or S.

In an exemplary embodiment of the present specification, Ar may be hydrogen; deuterium; a substituted or unsubstituted tert-butyl group; a substituted or unsubstituted phenyl group; a substituted or unsubstituted benzofuran group; or a substituted or unsubstituted benzothiophene group.

In an exemplary embodiment of the present specification, Ar may be hydrogen; deuterium; a halogen group; a cyano group; a C1 to C40 alkyl group unsubstituted or substituted with deuterium; a C6 to C40 aryl group unsubstituted or substituted with deuterium, an alkyl group, or an alkyl group substituted with deuterium; or a C2 to C40 heteroaryl group unsubstituted or substituted with deuterium.

In an exemplary embodiment of the present specification, Ar may be hydrogen; deuterium; a halogen group; a cyano group; a C1 to C20 alkyl group unsubstituted or substituted with deuterium; a C6 to C20 aryl group unsubstituted or substituted with deuterium, an alkyl group, or an alkyl group substituted with deuterium; or a C2 to C20 heteroaryl group unsubstituted or substituted with deuterium.

In an exemplary embodiment of the present specification, Ar may be hydrogen; deuterium; a C1 to C10 alkyl group unsubstituted or substituted with deuterium; a C6 to C20 aryl group unsubstituted or substituted with deuterium, an alkyl group, or an alkyl group substituted with deuterium; or a C2 to C20 heteroaryl group.

In an exemplary embodiment of the present specification, Ar may be hydrogen; deuterium; a C1 to C10 alkyl group unsubstituted or substituted with deuterium; a C6 to C20 aryl group unsubstituted or substituted with deuterium, an alkyl group, or an alkyl group substituted with deuterium; or a C2 to C20 heteroaryl group including O or S.

In an exemplary embodiment of the present specification, Ar may be hydrogen; deuterium; a tert-butyl group unsubstituted or substituted with deuterium; a phenyl group unsubstituted or substituted with deuterium, an alkyl group, or an alkyl group substituted with deuterium; a benzofuran group; or a benzothiophene group.

In an exemplary embodiment of the present specification, Ar may be hydrogen; deuterium; a tert-butyl group unsubstituted or substituted with deuterium; a phenyl group unsubstituted or substituted with deuterium, a tert-butyl group, or a tert-butyl group substituted with deuterium; a benzofuran group; or a benzothiophene group.

In an exemplary embodiment of the present specification, R1 to R3 are each independently hydrogen; deuterium; a halogen group; a cyano group; a substituted or unsubstituted C1 to C60 alkyl group; a substituted or unsubstituted C3 to C60 cycloalkyl group; or a substituted or unsubstituted C2 to C60 heterocycloalkyl group.

In an exemplary embodiment of the present specification, R1 to R3 may be each independently hydrogen; deuterium; a halogen group; a cyano group; a substituted or unsubstituted C1 to C40 alkyl group; a substituted or unsubstituted C3 to C40 cycloalkyl group; or a substituted or unsubstituted C2 to C40 heterocycloalkyl group.

In an exemplary embodiment of the present specification, R1 to R3 may be each independently hydrogen; deuterium; a halogen group; a cyano group; a substituted or unsubstituted C1 to C20 alkyl group; a substituted or unsubstituted C3 to C20 cycloalkyl group; or a substituted or unsubstituted C2 to C20 heterocycloalkyl group.

In an exemplary embodiment of the present specification, R1 to R3 may be each independently hydrogen; deuterium; or a substituted or unsubstituted C1 to C20 alkyl group.

In an exemplary embodiment of the present specification, R1 to R3 may be each independently hydrogen; or deuterium.

In an exemplary embodiment of the present specification, R1 to R3 may be hydrogen.

In an exemplary embodiment of the present specification, R4 may be hydrogen; deuterium; a halogen group; a cyano group; a substituted or unsubstituted C1 to C60 alkyl group; a substituted or unsubstituted C3 to C60 cycloalkyl group; or a substituted or unsubstituted C2 to C60 heterocycloalkyl group.

In an exemplary embodiment of the present specification, R4 may be hydrogen; deuterium; a halogen group; a cyano group; or a substituted or unsubstituted C1 to C60 alkyl group.

In an exemplary embodiment of the present specification, R4 may be hydrogen; or deuterium.

In an exemplary embodiment of the present specification, R4 may be hydrogen.

In an exemplary embodiment of the present specification, o is 5, and R4 may be all hydrogen.

In an exemplary embodiment of the present specification, R4 may be deuterium.

In an exemplary embodiment of the present specification, o is 5, and R4 may be all deuterium.

In an exemplary embodiment of the present specification, o may be an integer from 1 to 6.

In an exemplary embodiment of the present specification, Het is a group represented by any one of the following Chemical Formulae H-1 to H-3. 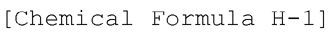 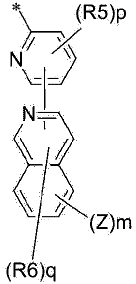 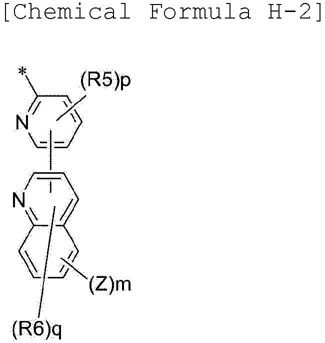 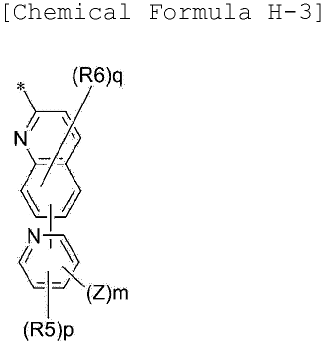 in Chemical Formulae H-1 to H-3,
Z is hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
m is an integer from 0 to 4, and when m is 2 or higher, Z's are the same as or different from each other,
R5 and R6 are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, and
p is an integer from 1 to 3, q is an integer from 1 to 5, and when p and q are each 2 or higher, R5 and R6 are the same as or different from each other.

In an exemplary embodiment of the present specification, Z may be hydrogen; deuterium; a halogen group; a cyano group; a substituted or unsubstituted C1 to C40 alkyl group; a substituted or unsubstituted C6 to C40 aryl group; or a substituted or unsubstituted C2 to C40 heteroaryl group.

In an exemplary embodiment of the present specification, Z may be hydrogen; deuterium; a halogen group; a cyano group; a substituted or unsubstituted C1 to C20 alkyl group; a substituted or unsubstituted C6 to C20 aryl group; or a substituted or unsubstituted C2 to C20 heteroaryl group.

In an exemplary embodiment of the present specification, Z may be hydrogen; deuterium; a halogen group; a cyano group; a substituted or unsubstituted C1 to C10 alkyl group; a substituted or unsubstituted C6 to C20 aryl group; or a substituted or unsubstituted C2 to C20 heteroaryl group.

In an exemplary embodiment of the present specification, Z may be hydrogen; deuterium; a halogen group; a cyano group; a substituted or unsubstituted C1 to C10 alkyl group; a substituted or unsubstituted C6 to C20 aryl group; or a substituted or unsubstituted C2 to C20 heteroaryl group including N.

In an exemplary embodiment of the present specification, Z may be hydrogen; deuterium; a halogen group; a cyano group; a substituted or unsubstituted C1 to C10 alkyl group; a substituted or unsubstituted C6 to C20 aryl group; or a substituted or unsubstituted C2 to C20 heteroaryl group including a C=N bond.

In an exemplary embodiment of the present specification, Z may be hydrogen; deuterium; a halogen group; a cyano group; a substituted or unsubstituted tert-butyl group; a substituted or unsubstituted phenyl group; a substituted or unsubstituted naphthyl group; a substituted or unsubstituted pyridine group; or a substituted or unsubstituted pyrimidine group.

In an exemplary embodiment of the present specification, Z may be hydrogen; deuterium; a halogen group; a cyano group; a C1 to C40 alkyl group unsubstituted or substituted with deuterium or a halogen group; a C6 to C40 aryl group unsubstituted or substituted with deuterium, a halogen group, a cyano group, or an alkyl group; or a C2 to C40 heteroaryl group.

In an exemplary embodiment of the present specification, Z may be hydrogen; deuterium; a halogen group; a cyano group; a C1 to C20 alkyl group unsubstituted or substituted with deuterium or a halogen group; a C6 to C20 aryl group unsubstituted or substituted with deuterium, a halogen group, a cyano group, or an alkyl group; or a C2 to C20 heteroaryl group.

In an exemplary embodiment of the present specification, Z may be hyydrogen; deuterium; a halogen group; a cyano group; a C1 to C10 alkyl group unsubstituted or substituted with deuterium or a halogen group; a C6 to C20 aryl group unsubstituted or substituted with deuterium, a halogen group, a cyano group, or an alkyl group; or a C2 to C20 heteroaryl group.

In an exemplary embodiment of the present specification, Z may be hydrogen; deuterium; a halogen group; a cyano group; a C1 to C10 alkyl group unsubstituted or substituted with deuterium or a halogen group; a C6 to C20 aryl group unsubstituted or substituted with deuterium, a halogen group, a cyano group, or an alkyl group; or a C2 to C20 heteroaryl group including C=N bond.

In an exemplary embodiment of the present specification, Z may be hydrogen; deuterium; a halogen group; a cyano group; a methyl group unsubstituted or substituted with a halogen group; a tert-butyl group unsubstituted or substituted with deuterium; a phenyl group unsubstituted or substituted with deuterium, a halogen group, a cyano group, or an alkyl group; a naphthyl group; a pyridine group; or a pyrimidine group.

In an exemplary embodiment of the present specification, Z may be hydrogen; deuterium; a halogen group; a cyano group; -CF₃; a tert-butyl group unsubstituted or substituted with deuterium; a phenyl group unsubstituted or substituted with deuterium, a halogen group, a cyano group, or a tert-butyl group; a naphthyl group; a pyridine group; or a pyrimidine group.

In an exemplary embodiment of the present specification, m may be an integer from 1 to 4.

In an exemplary embodiment of the present specification, m may be 1.

In an exemplary embodiment of the present specification, when M, Ar, or Z is an alkyl group, M, Ar, or Z may be a C2 to C10 branched alkyl group.

In an exemplary embodiment of the present specification, R5 and R6 may be each independently hydrogen; deuterium; a halogen group; a cyano group; a substituted or unsubstituted C1 to C60 alkyl group; a substituted or unsubstituted C3 to C60 cycloalkyl group; or a substituted or unsubstituted C2 to C60 heterocycloalkyl group.

In an exemplary embodiment of the present specification, R5 and R6 may be each independently hydrogen; deuterium; a halogen group; a cyano group; or a substituted or unsubstituted C1 to C60 alkyl group.

In an exemplary embodiment of the present specification, R5 and R6 may be each independently hydrogen; or deuterium.

In an exemplary embodiment of the present specification, R5 may be hydrogen.

In an exemplary embodiment of the present specification, p is 3, and R5 may be all hydrogen.

In an exemplary embodiment of the present specification, R6 may be hydrogen.

In an exemplary embodiment of the present specification, q is 5, and R6 may be all hydrogen.

In an exemplary embodiment of the present specification, R5 may be deuterium.

In an exemplary embodiment of the present specification, p is 3, and R5 may be all deuterium.

In an exemplary embodiment of the present specification, R6 may be deuterium.

In an exemplary embodiment of the present specification, q is 5, and R6 may be all deuterium.

In an exemplary embodiment of the present specification, the sum of q and m in Chemical Formula H-1 is an integer from 1 to 6.

In an exemplary embodiment of the present specification, the sum of q and m in Chemical Formula H-1 is 6.

In an exemplary embodiment of the present specification, the sum of q and m in Chemical Formula H-2 is an integer from 1 to 6.

In an exemplary embodiment of the present specification, the sum of q and m in Chemical Formula H-2 is 6.

In an exemplary embodiment of the present specification, the sum of p and m in Chemical Formula H-3 is an integer from 1 to 4.

In an exemplary embodiment of the present specification, the sum of p and m in Chemical Formula H-3 is 4.

In an exemplary embodiment of the present specification, R4 to R6 may be each independently hydrogen; or deuterium.

In an exemplary embodiment of the present specification, the deuterium substitution rate based on the total hydrogen and deuterium atoms in Chemical Formula 1 may be 0% or 5% to 100%.

In an exemplary embodiment of the present specification, the deuterium substitution rate of Chemical Formula 1 may be 0% or 10% to 100%.

In an exemplary embodiment of the present specification, the deuterium substitution rate of Chemical Formula 1 may be 0% or 15% to 100%.

In an exemplary embodiment of the present specification, the deuterium substitution rate of Chemical Formula 1 may be 0% or 20% to 100%.

In an exemplary embodiment of the present specification, the deuterium substitution rate of Chemical Formula 1 may be 0%.

In an exemplary embodiment of the present specification, the deuterium substitution rate of Chemical Formula 1 may be 100%.

In the present specification, the deuterium substitution rate refers to the ratio of the number of deuterium atoms to the total number of hydrogen and deuterium atoms included in a specific structure (for example, Chemical Formula 1). For example, when a particular structure includes 20 hydrogen atoms and 20 deuterium atoms, the deuterium substitution rate is 50% because the ratio of the 20 deuterium atoms to the total of 40 hydrogen and deuterium atoms is 50%.

In an exemplary embodiment of the present specification, the deuterium proportion of the heterocyclic compound of Chemical Formula 1 satisfies the above range, the photochemical characteristics of a compound which includes deuterium and a compound which does not include deuterium among the heterocyclic compounds of Chemical Formula 1 are almost similar, but when deposited on a thin film, the deuterium-containing material tends to be packed with a narrower intermolecular distance.

Accordingly, when an electron only device (EOD) and a hole only device (HOD) are manufactured and the current density thereof according to voltage is confirmed, it can be confirmed that among the heterocyclic compounds of Chemical Formula 1 of the present invention, a compound including deuterium exhibits much more balanced charge transport characteristics than a compound which does not include deuterium, in the same structure.

Further, when the surface of a thin film is observed using an atomic force microscope (AFM), it can be confirmed that the thin film made of a compound including deuterium is deposited with a more uniform surface without any aggregated portion.

Additionally, since the single bond dissociation energy of carbon and deuterium is higher than the single bond dissociation energy of carbon and hydrogen, when the heterocyclic compound of Chemical Formula 1 of the present invention includes deuterium, the stability of the total molecules is enhanced, so that there is an effect that the service life of the device is improved.

In an exemplary embodiment of the present specification, the heterocyclic compound of Chemical Formula 1 may be represented by any one of the following compounds.

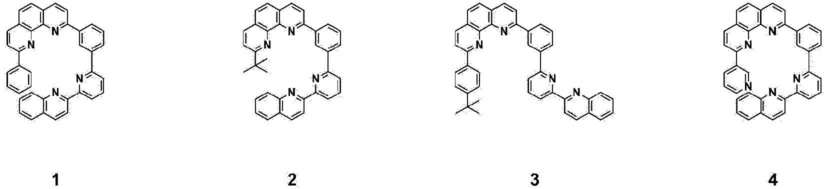

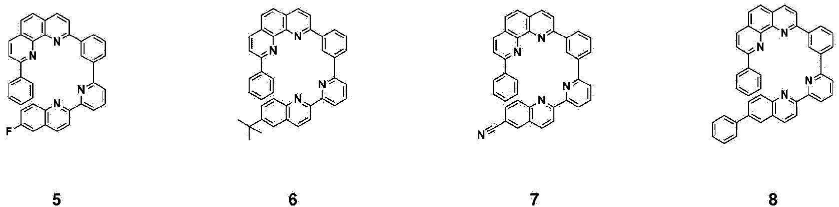

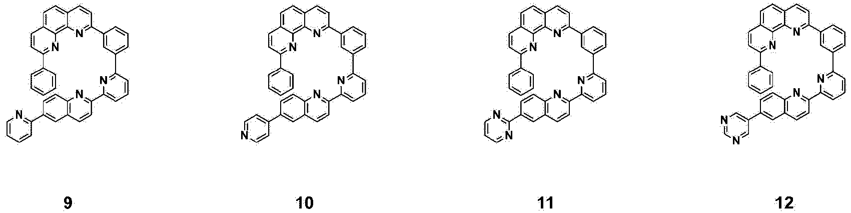

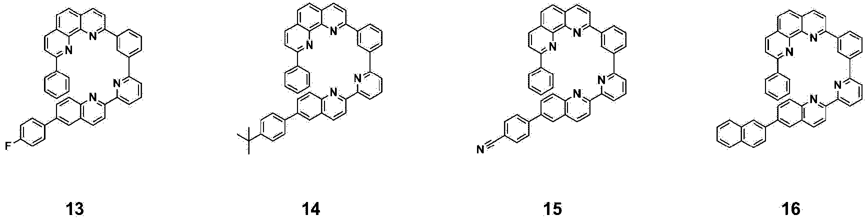

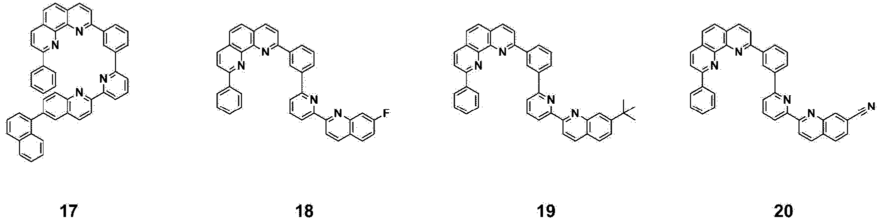

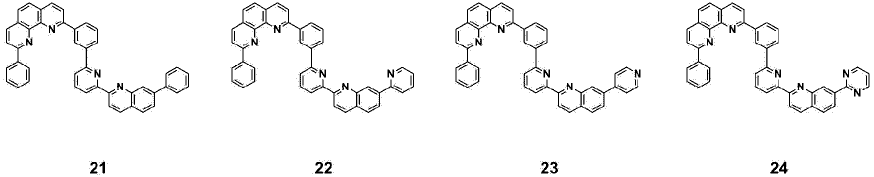

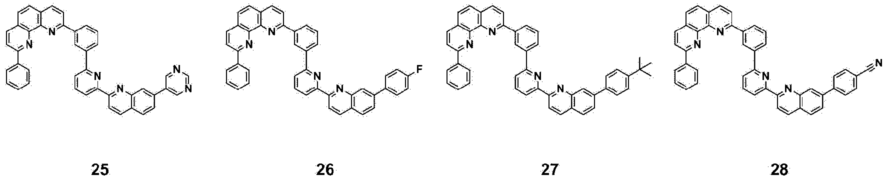

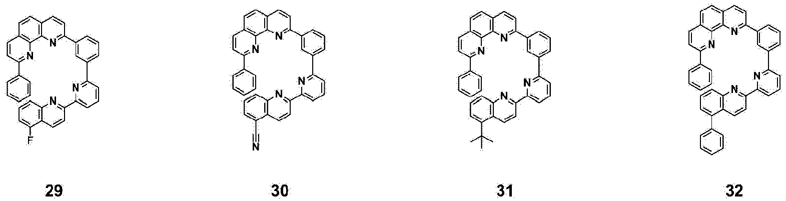

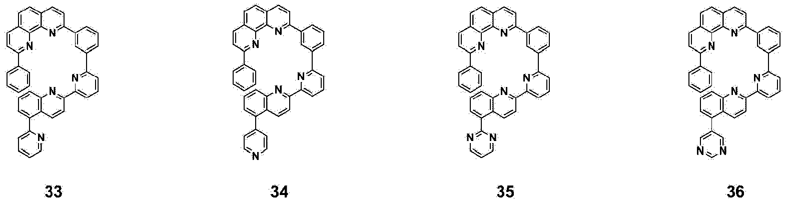

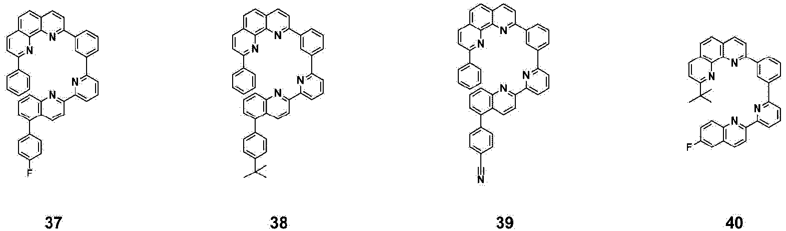

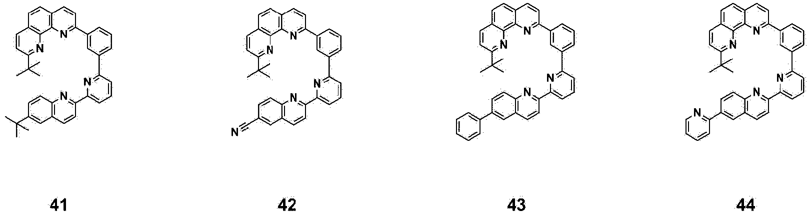

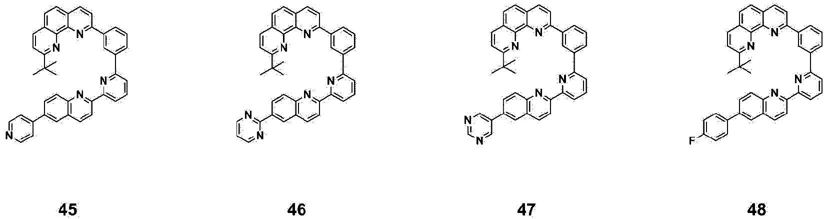

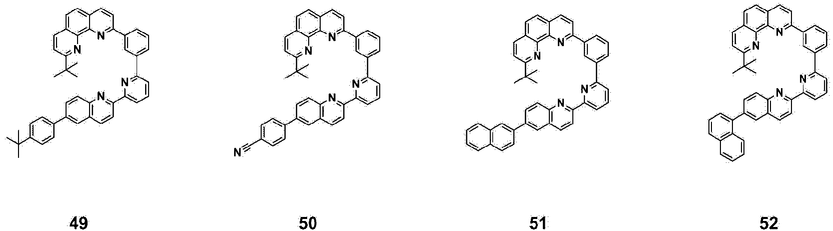

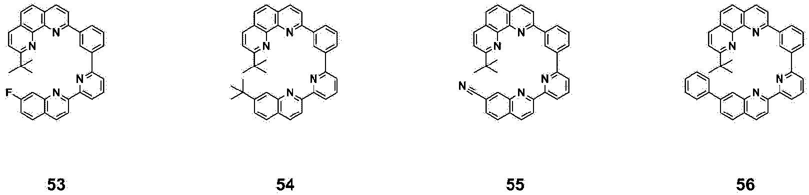

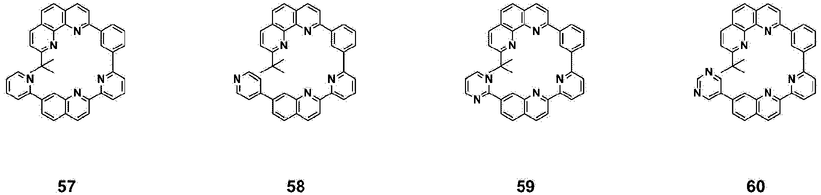

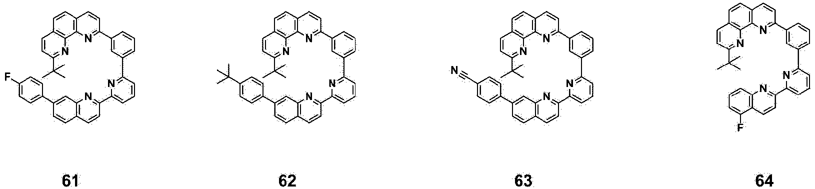

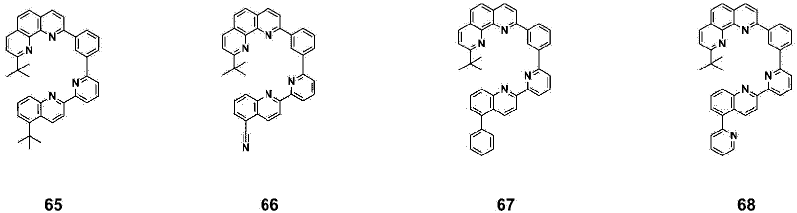

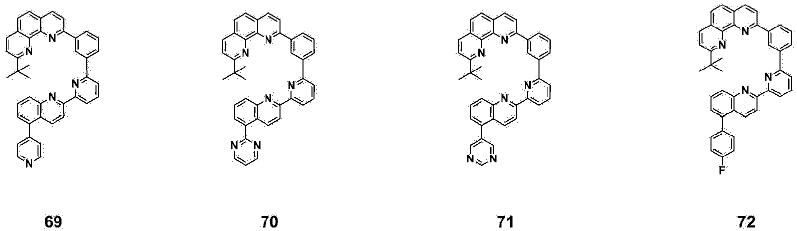

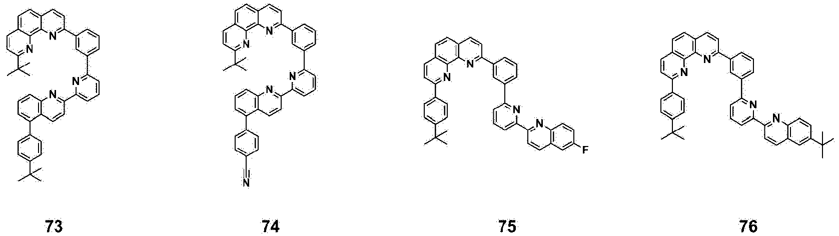

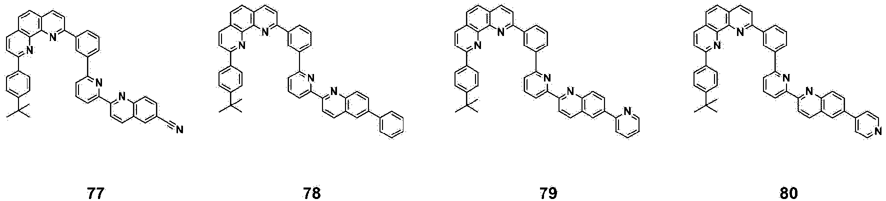

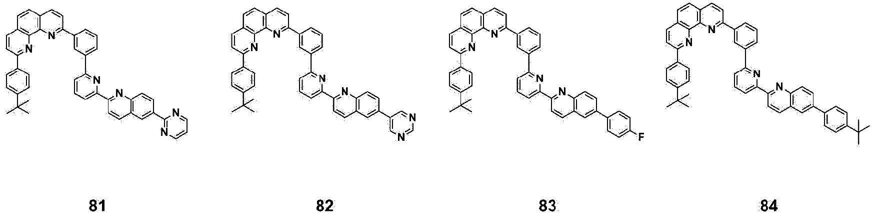

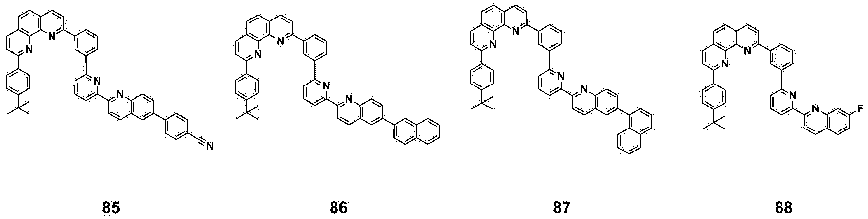

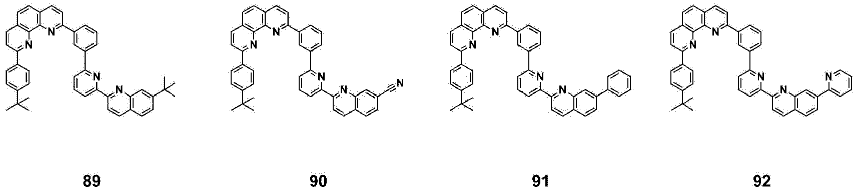

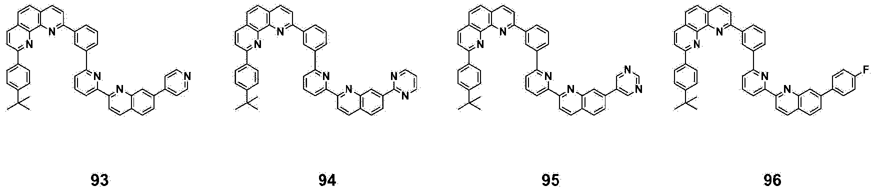

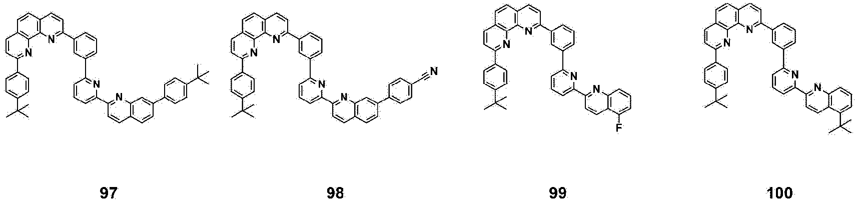

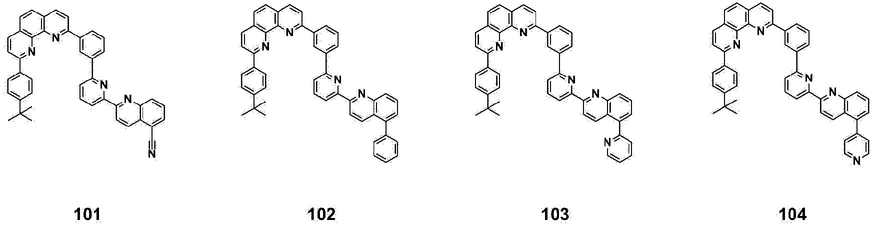

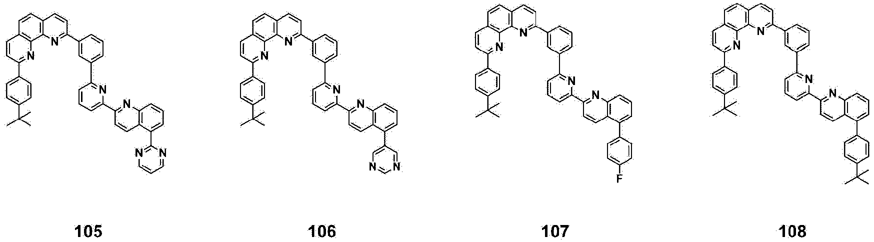

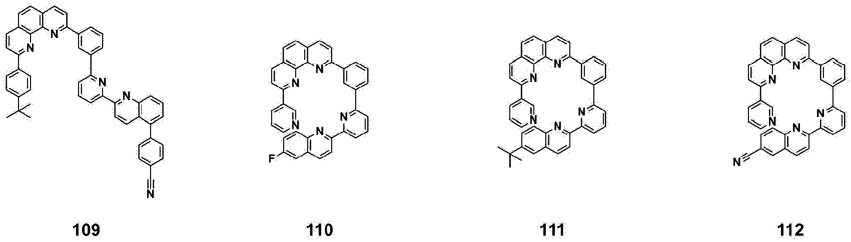

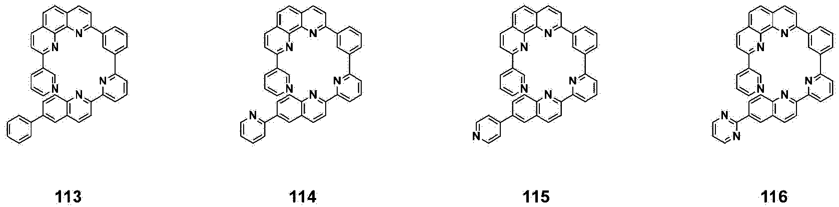

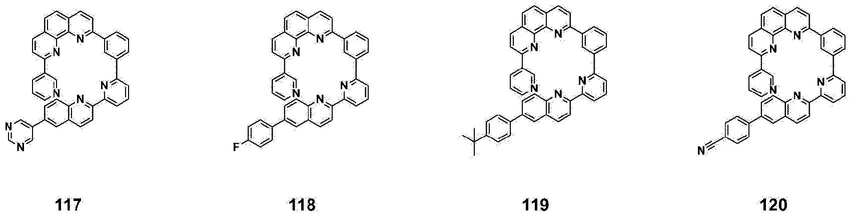

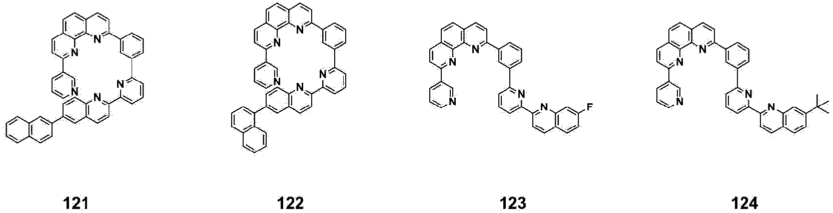

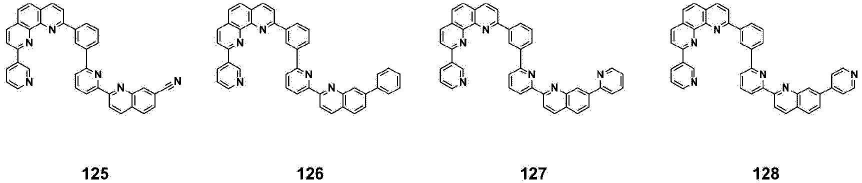

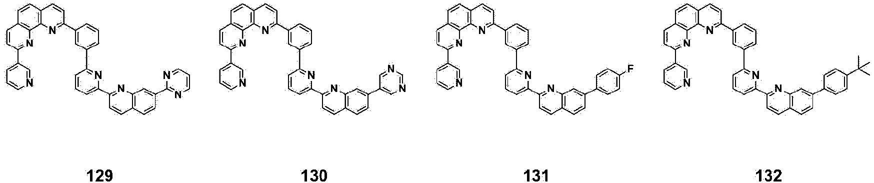

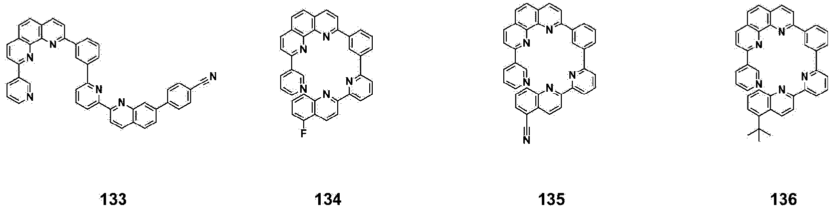

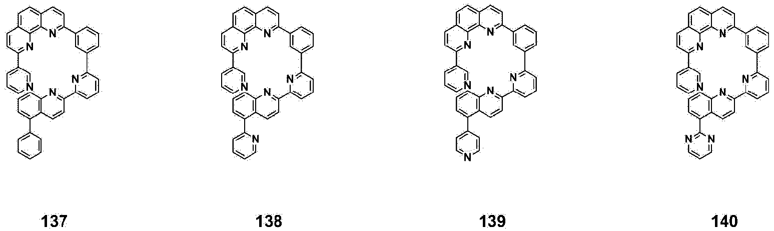

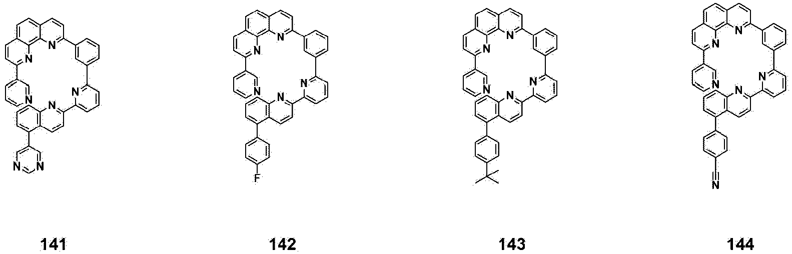

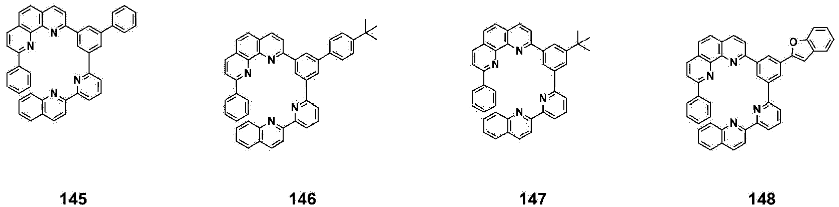

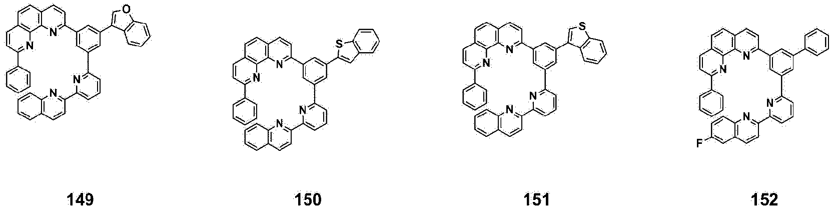

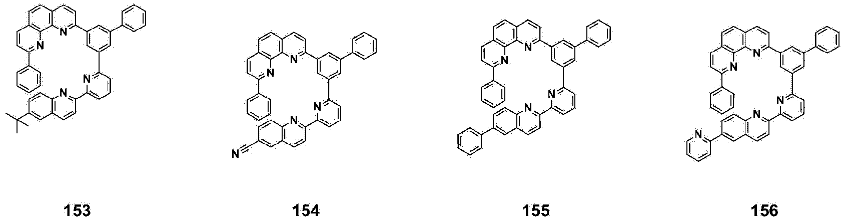

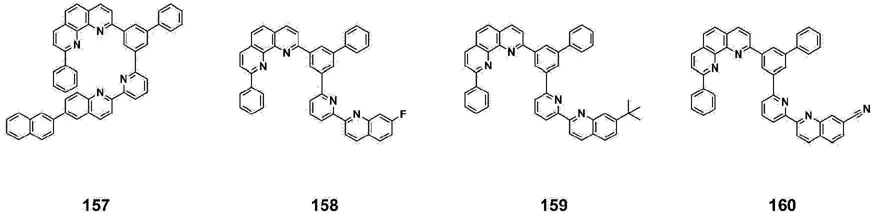

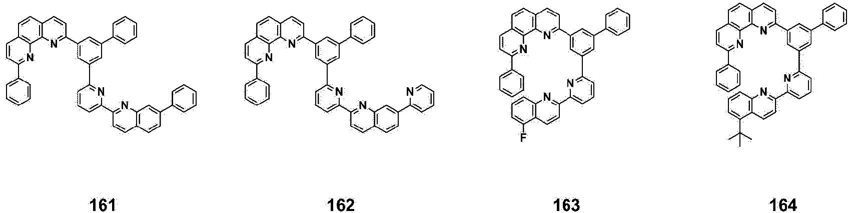

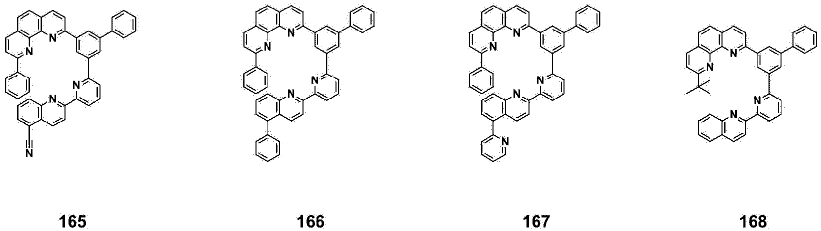

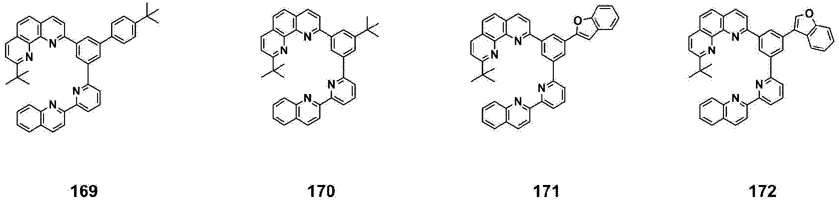

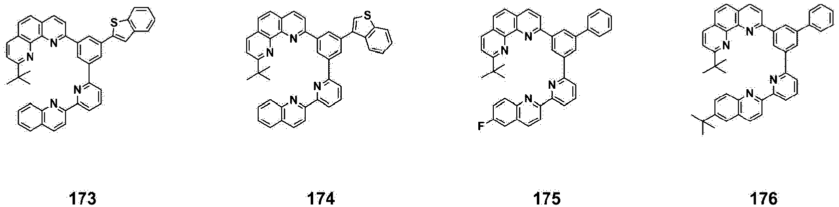

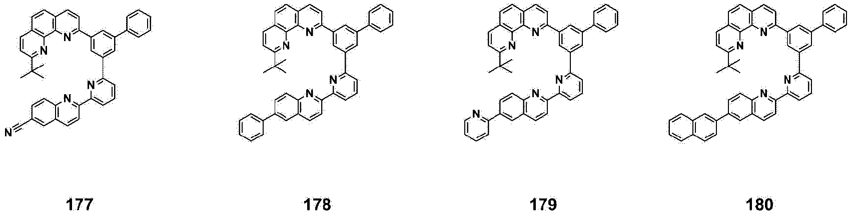

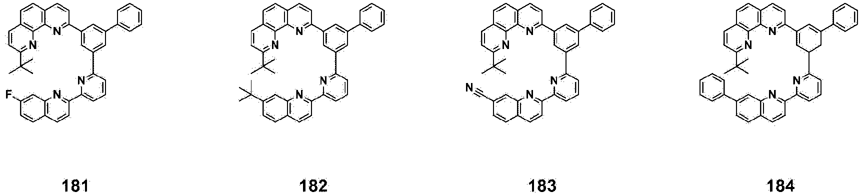

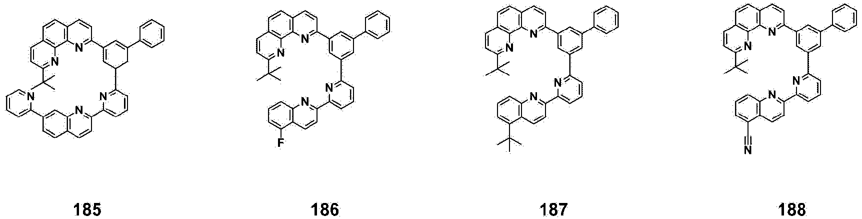

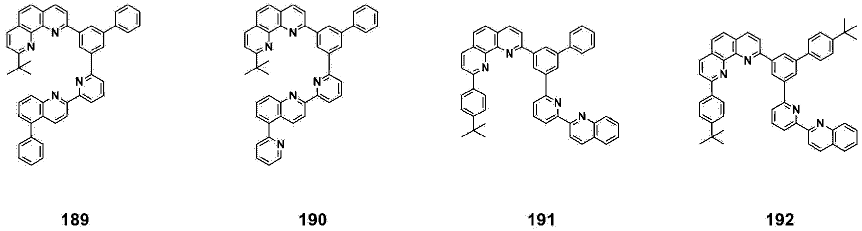

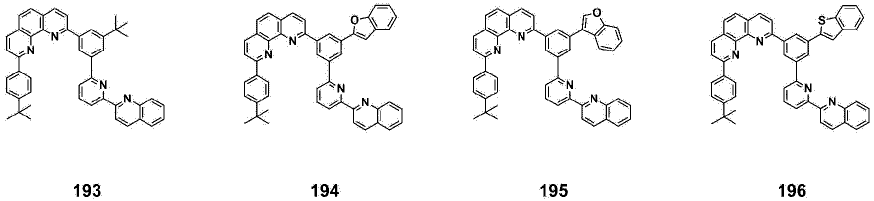

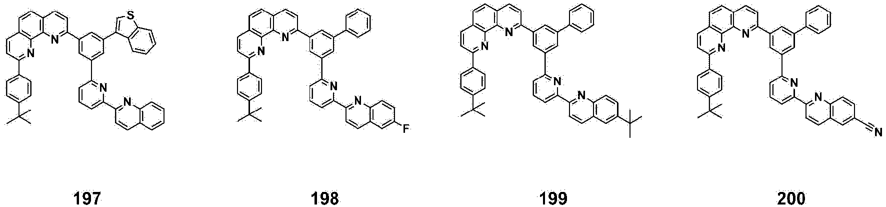

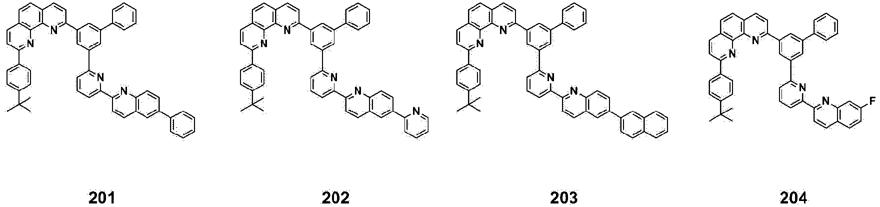

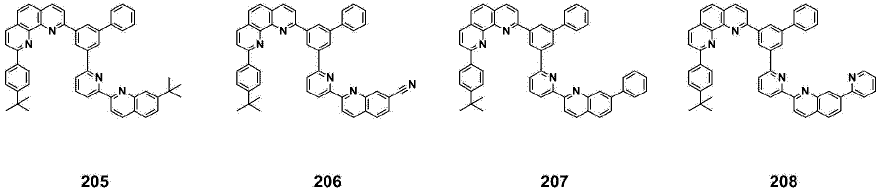

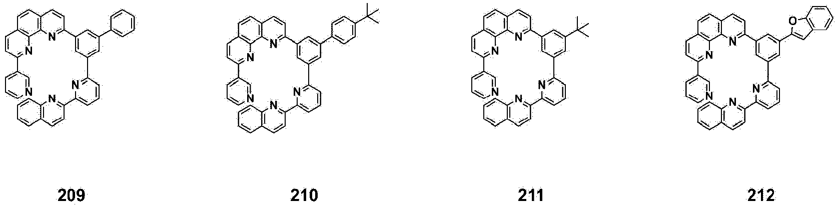

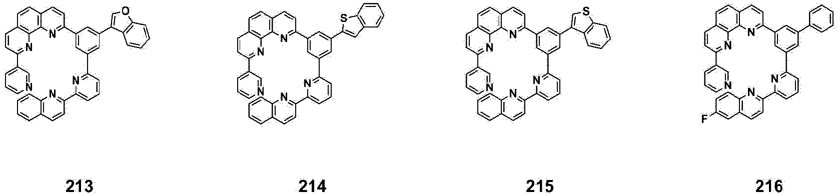

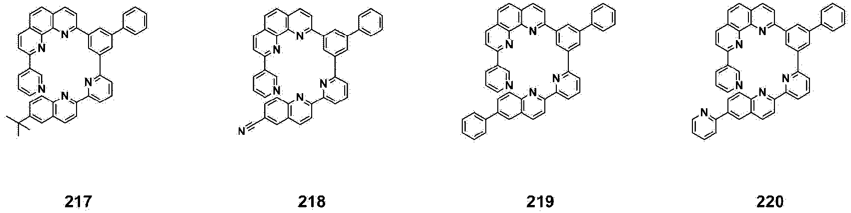

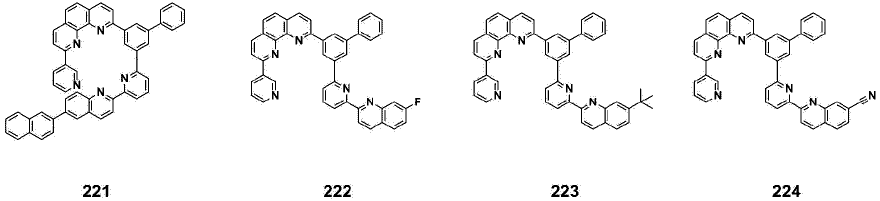

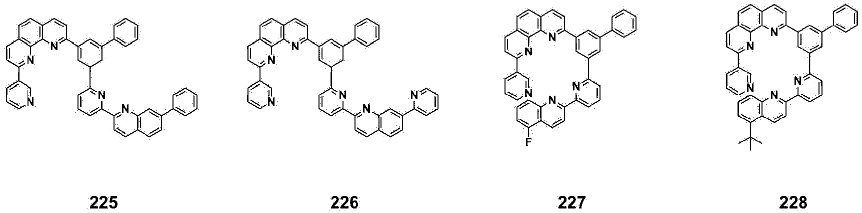

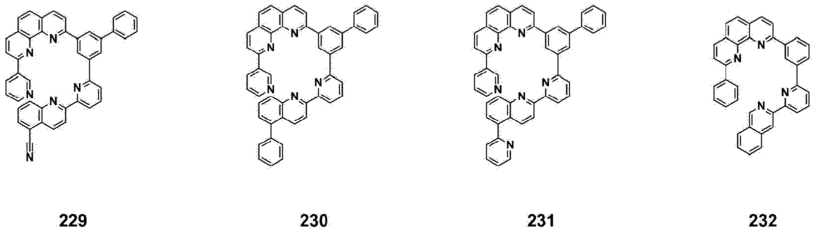

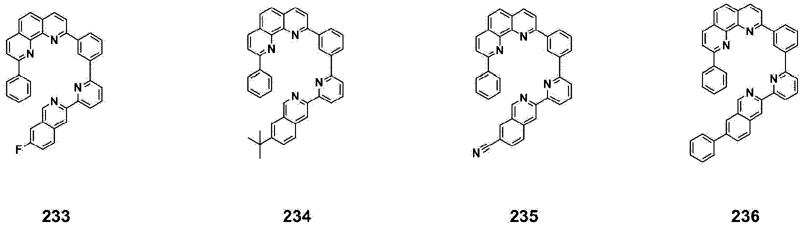

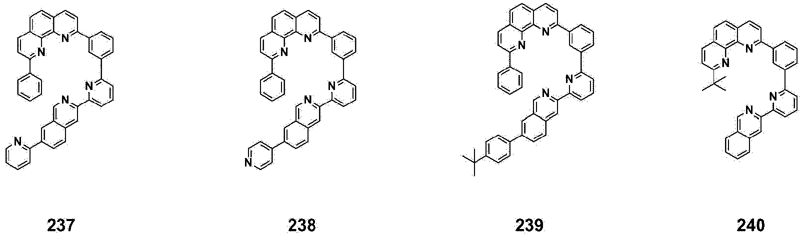

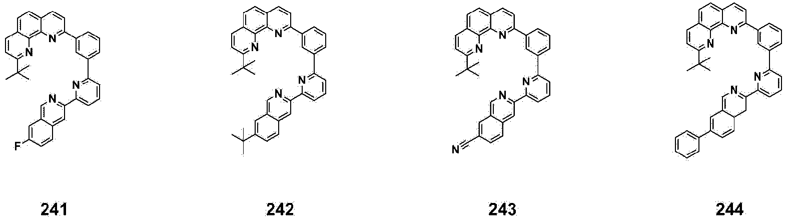

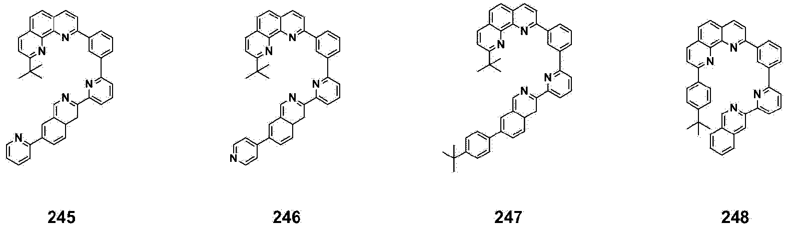

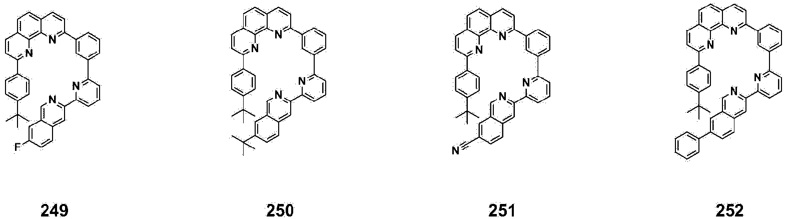

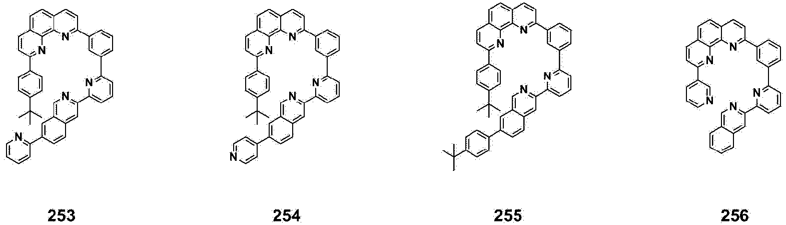

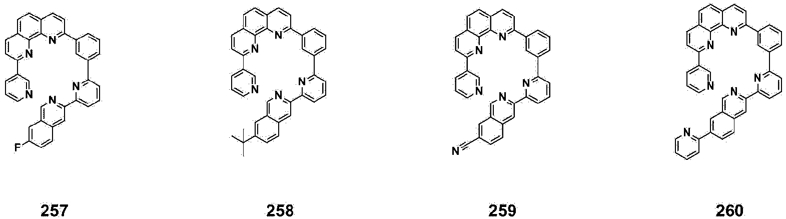

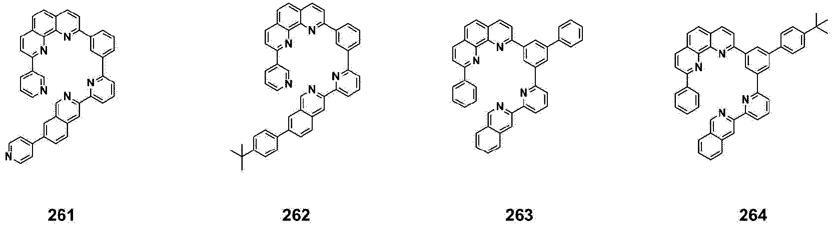

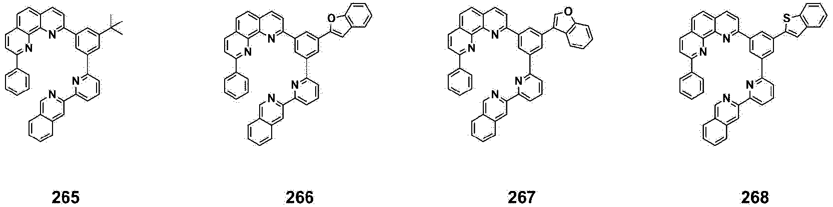

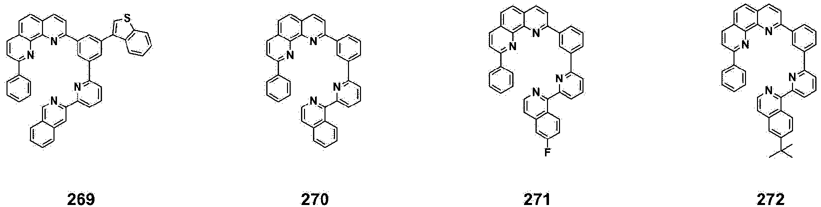

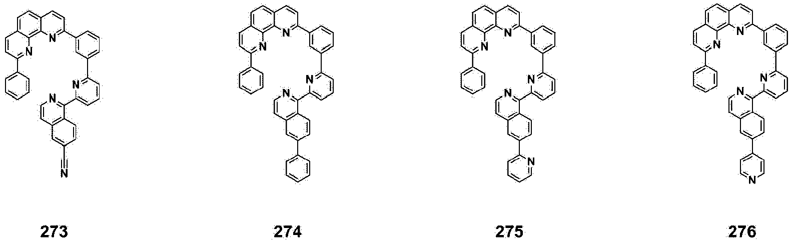

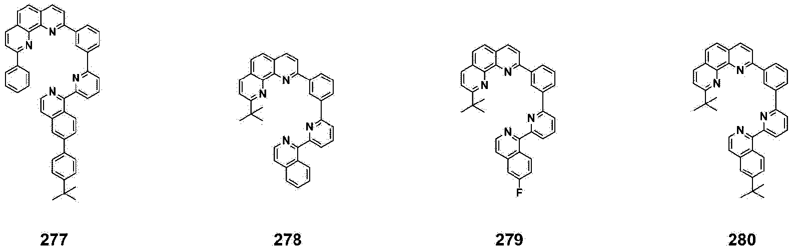

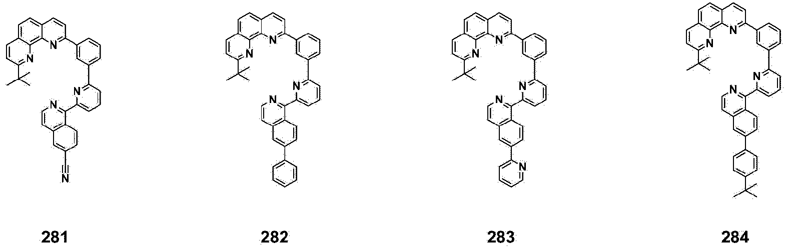

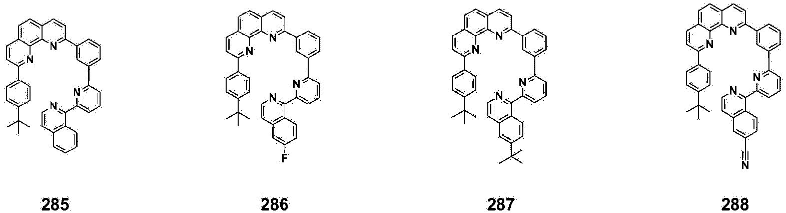

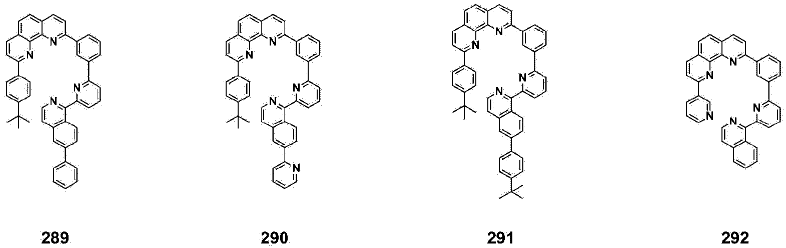

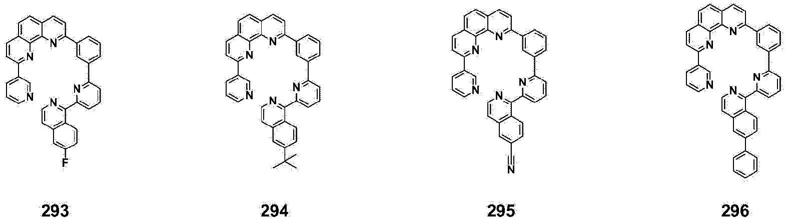

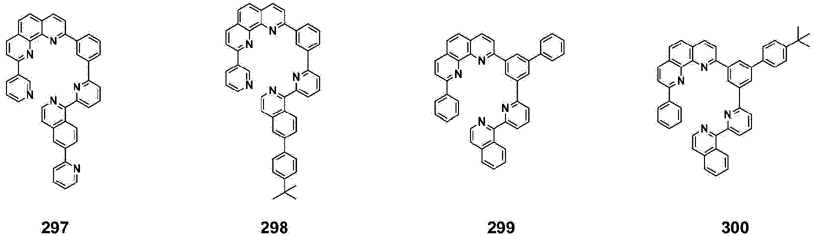

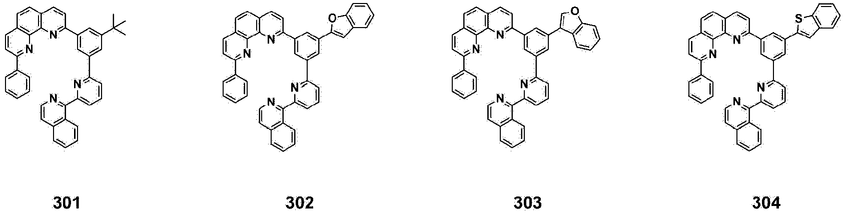

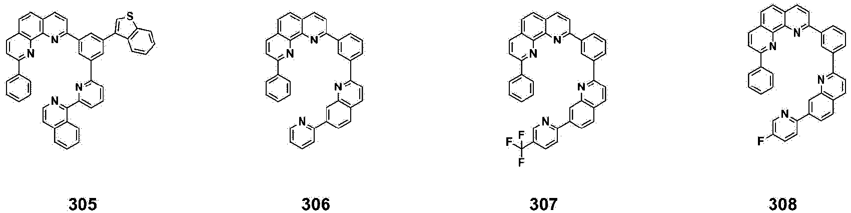

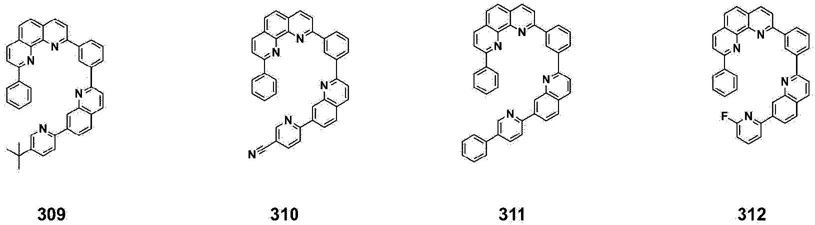

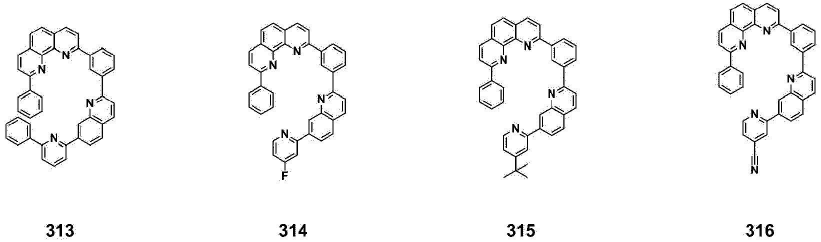

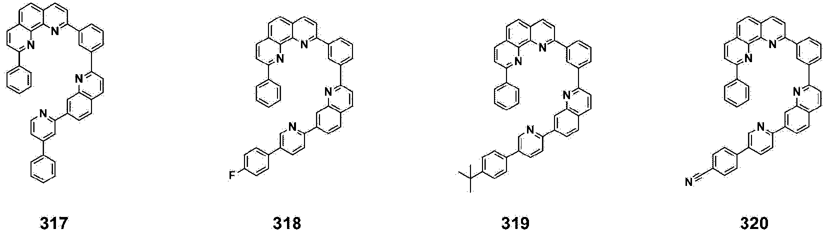

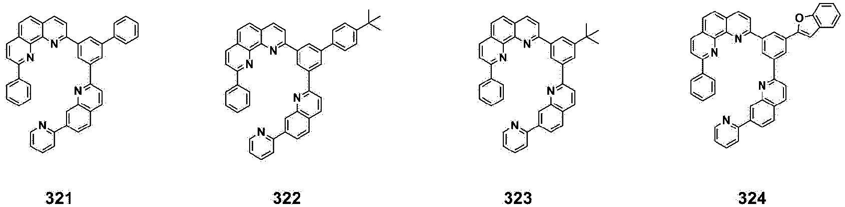

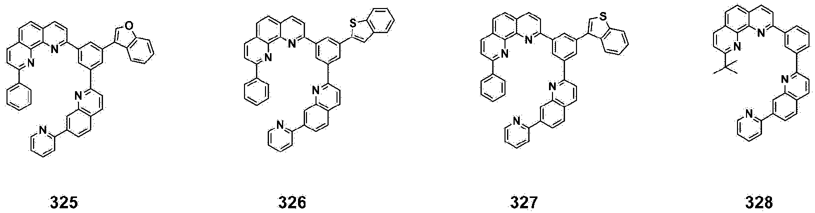

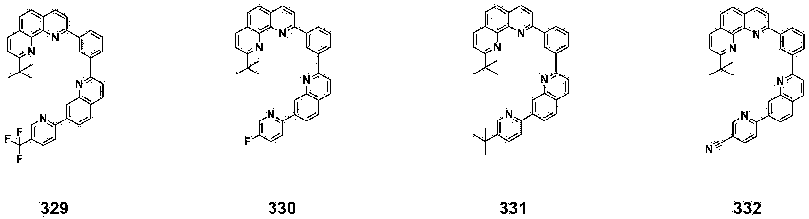

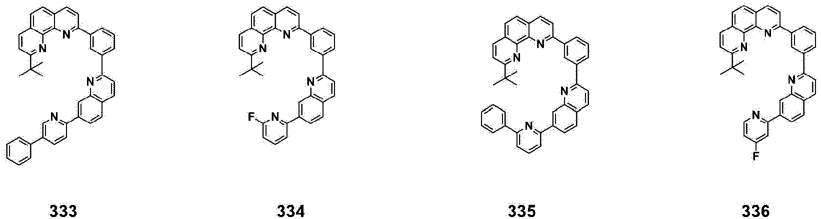

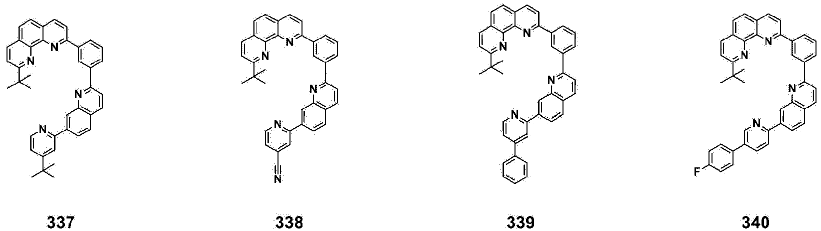

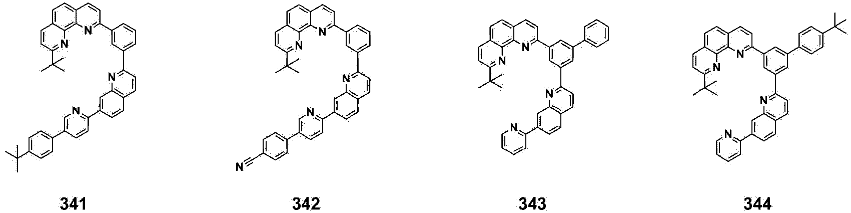

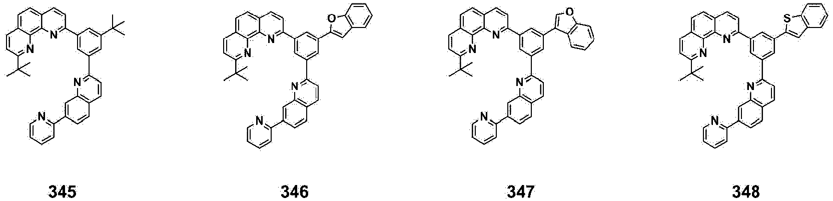

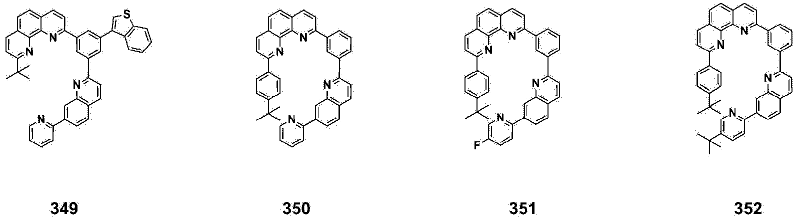

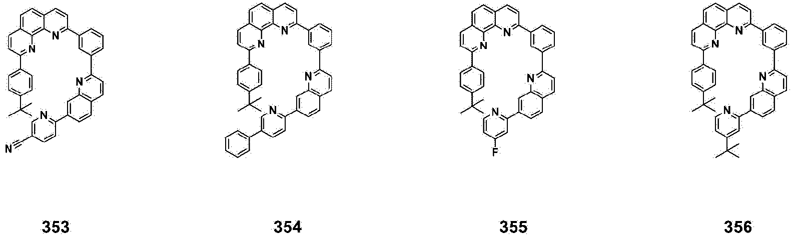

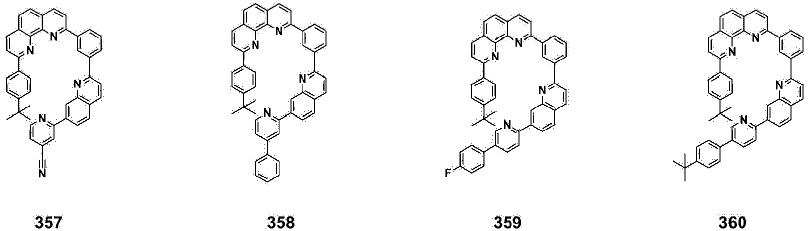

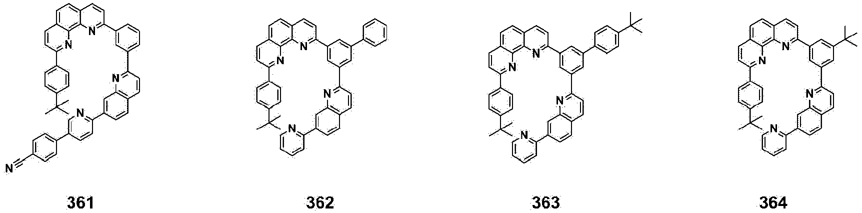

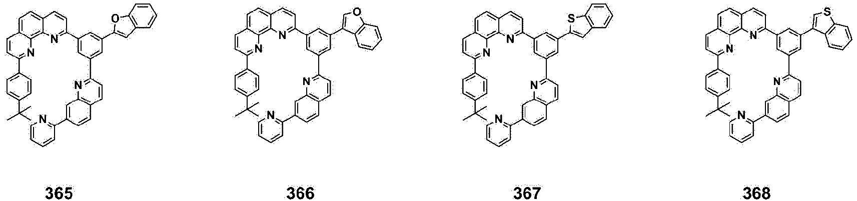

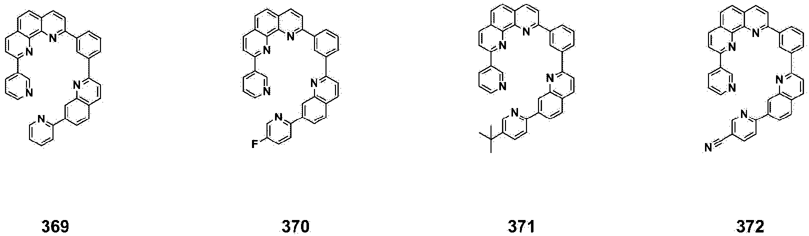

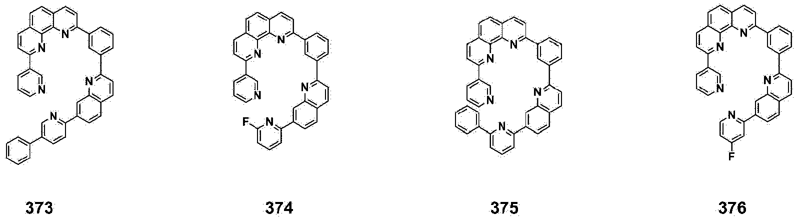

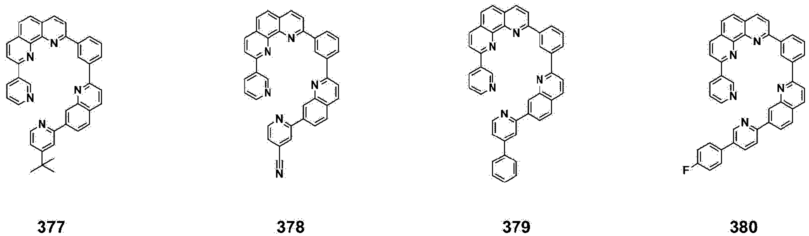

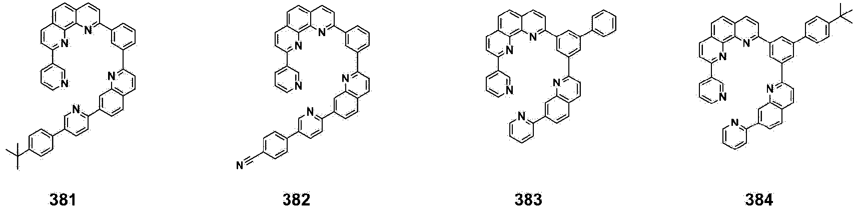

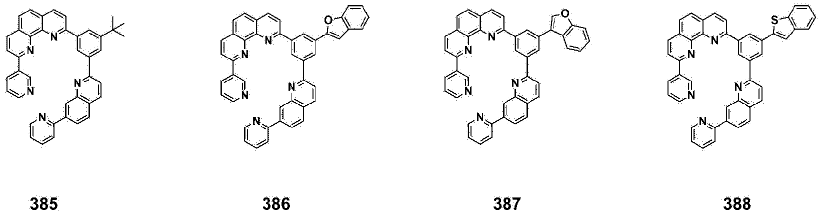

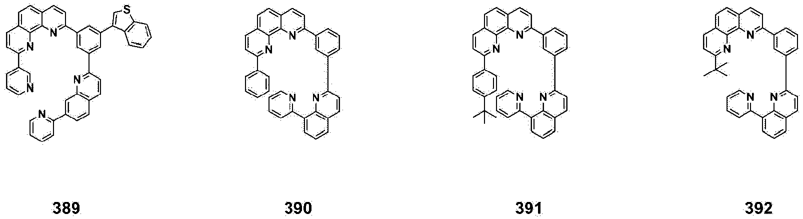

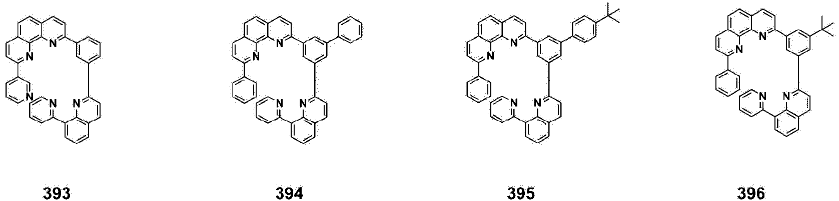

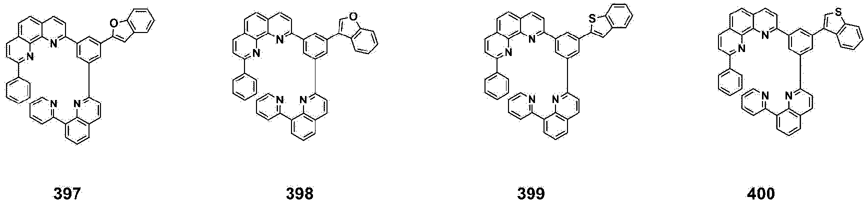

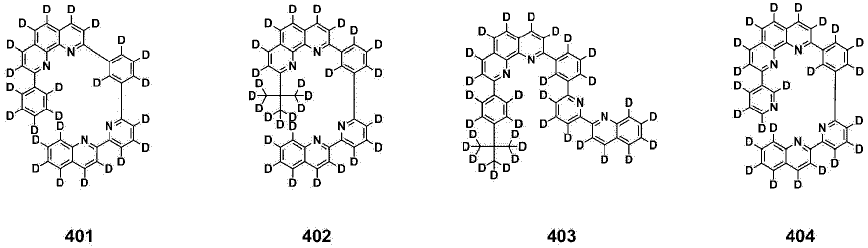

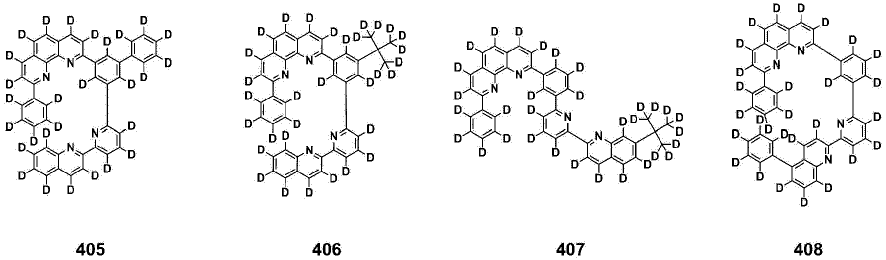

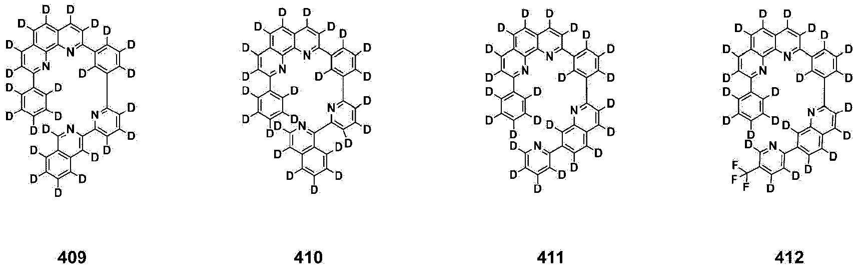

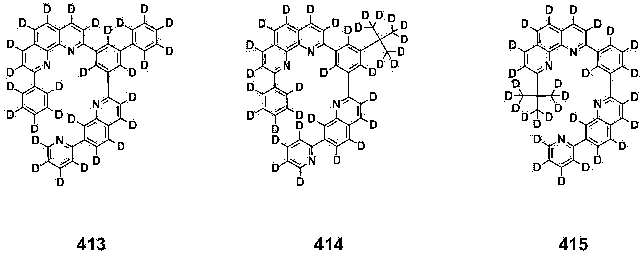

Further, various substituents may be introduced into the structure of Chemical Formula 1 to synthesize a compound having inherent characteristics of a substituent introduced. For example, it is possible to synthesize a material which satisfies conditions required for each organic material layer by introducing a substituent usually used for a hole injection layer material, a hole transport layer material, a light emitting layer material, an electron transport layer material, and a charge generation layer material, which are used for preparing an organic light emitting device, into the core structure.

In addition, it is possible to finely adjust an energy band-gap by introducing various substituents into the structure of Chemical Formula 1, and meanwhile, it is possible to improve characteristics at the interface between organic materials and diversify the use of the material.

In another exemplary embodiment of the present specification, provided is an organic light emitting device including: a first electrode; a second electrode; and an organic material layer having one or more layers provided between the first electrode and the second electrode, in which one or more layers of the organic material layer include the one or more heterocyclic compounds of Chemical Formula 1.

In an exemplary embodiment of the present specification, the organic material layer includes an electron transport layer, and the electron transport layer includes one or more of the heterocyclic compounds of Chemical Formula 1.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In an exemplary embodiment of the present specification, the organic light emitting device may include the first electrode, a first stack provided on the first electrode and including a first light emitting layer, a charge generation layer provided on the first stack, a second stack provided on the charge generation layer and including a second light emitting layer, and a second electrode provided on the second stack.

In this case, the charge generation layer may include the heterocyclic compound represented by Chemical Formula 1. When the heterocyclic compound is used in a charge generation layer, the driving, efficiency and service life of the organic light emitting device may become excellent. Specifically, the heterocyclic compound of the present invention may bind to a metal, and thus has an effect of helping to facilitate the injection of electrons generated from a P-type charge generation layer due to a high binding effect with an alkali metal or an alkaline earth metal.

Furthermore, the first stack and the second stack may each independently further include one or more of the above-described hole injection layer, hole transport layer, hole blocking layer, electron transport layer, electron injection layer, and the like.

In an exemplary embodiment of the present specification, the organic light emitting device includes the first electrode, a first stack provided on the first electrode and including a first light emitting layer, a charge generation layer provided on the first stack, a second stack provided on the charge generation layer and including a second light emitting layer, and a second electrode provided on the second stack, and the charge generation layer may include one or more of the heterocyclic compounds.

In an exemplary embodiment of the present specification, the charge generation layer is an N-type charge generation layer, and the N-type charge generation layer may include one or more of the heterocyclic compounds.

In an exemplary embodiment of the present specification, the charge generation layer may additionally include a dopant known in the art in addition to the heterocyclic compound represented by Chemical Formula 1.

In an exemplary embodiment of the present specification, the organic light emitting device may further include a P-type charge generation layer.

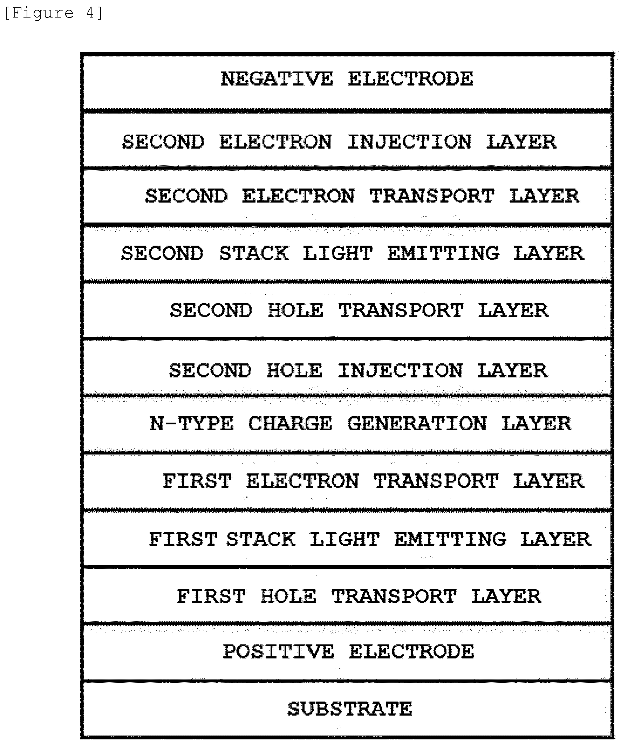

As the organic light emitting device according to an exemplary embodiment of the present application, an organic light emitting device having a 2-stack tandem structure is exemplarily illustrated in the following FIG. 4, but is not limited thereto, and an additional organic material layer may be further provided.

In this case, the first electron blocking layer, the first hole blocking layer, the second hole blocking layer, and the like described in the following FIG. 4 may be omitted depending on the case.

In an exemplary embodiment of the present specification, the first electrode may be a positive electrode, and the second electrode may be a negative electrode.

In another exemplary embodiment of the present specification, the first electrode may be a negative electrode, and the second electrode may be a positive electrode.

The organic light emitting device according to an exemplary embodiment of the present specification may be manufactured by typical methods and materials for manufacturing an organic light emitting device, except that an organic material layer having one or more layers is formed by using the heterocyclic compound of the above-described Chemical Formula 1.

The heterocyclic compound of Chemical Formula 1 may be formed as an organic material layer by not only a vacuum deposition method, but also a solution application method when an organic light emitting device is manufactured. Herein, the solution coating method means spin coating, dip coating, inkjet printing, screen printing, a spray method, roll coating, and the like, but is not limited thereto.

In an exemplary embodiment of the present specification, the organic light emitting device may be a blue organic light emitting device, and the heterocyclic compound of Chemical Formula 1 may be used as a material for the blue organic light emitting device. For example, the heterocyclic compound of Chemical Formula 1 may be included in an electron transport layer or a charge generation layer of the blue organic light emitting device.

In another exemplary embodiment of the present specification, the organic light emitting device may be a green organic light emitting device, and the heterocyclic compound of Chemical Formula 1 may be used as a material for the green organic light emitting device. For example, the heterocyclic compound of Chemical Formula 1 may be included in an electron transport layer or a charge generation layer of the green organic light emitting device.

In still another exemplary embodiment of the present specification, the organic light emitting device may be a red organic light emitting device, and the heterocyclic compound of Chemical Formula 1 may be used as a material for the red organic light emitting device. For example, the heterocyclic compound of Chemical Formula 1 may be included in an electron transport layer or a charge generation layer of the red organic light emitting device.

The organic light emitting device of the present invention may further include one or more layers selected from the group consisting of a light emitting layer, a hole injection layer, a hole transport layer, an electron injection layer, an electron transport layer, an electron blocking layer, and a hole blocking layer.

FIGS. 1 to 4 exemplify the stacking sequence of the electrodes and the organic material layer of the organic light emitting device according to an exemplary embodiment of the present specification. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as illustrated in FIG. 2, an organic light emitting device in which a negative electrode, an organic material layer, and a positive electrode are sequentially stacked on a substrate may also be implemented.

FIG. 3 exemplifies a case where an organic material layer is a multilayer. An organic light emitting device according to FIG. 3 includes a hole injection layer 301, a hole transport layer 302, a light emitting layer 303, an electron transport layer 304, and an electron injection layer 305. However, the scope of the present application is not limited by the stacking structure as described above, and if necessary, the other layers except for the light emitting layer may be omitted, and another necessary functional layer may be further added.

An organic material layer including the heterocyclic compound of Chemical Formula 1 may additionally include other materials, if necessary.

In the organic light emitting device according to an exemplary embodiment of the present specification, materials other than the heterocyclic compound of Chemical Formula 1 will be exemplified below, but these materials are illustrative only and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrenesulfonate), and the like.

As a hole transport material, a pyrazoline derivative, an arylamine-based derivative, a stilbene derivative, a triphenyldiamine derivative, and the like may be used, and a low-molecular weight or polymer material may also be used.

As an electron transport material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials are deposited and used as an individual supply source, or premixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, any two or more materials from N-type host materials or P-type host materials may be selected and used as a host material for a light emitting layer.

The organic light emitting device according to an exemplary embodiment of the present specification may be a top emission type, a bottom emission type, or a dual emission type according to the material to be used.

The compound according to an exemplary embodiment of the present specification may act even in organic electronic devices including organic solar cells, organic photoconductors, organic transistors, and the like, based on the principle similar to those applied to organic light emitting devices.

In addition, it is possible to finely adjust an energy band-gap by introducing various substituents into the structure of Chemical Formula 1, and meanwhile, it is possible to improve characteristics at the interface between organic materials and diversify the use of the material.

Another exemplary embodiment of the present specification provides a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming an organic material layer having one or more layers using a composition for an organic material layer, and the composition for an organic material layer includes the above-described heterocyclic compound of Chemical Formula 1.

Hereinafter, the present specification will be described in more detail through Examples, but these Examples are provided only for exemplifying the present application, and are not intended to limit the scope of the present application.

### <Preparation Examples>

### <Preparation Example 1> Preparation of Compound 1

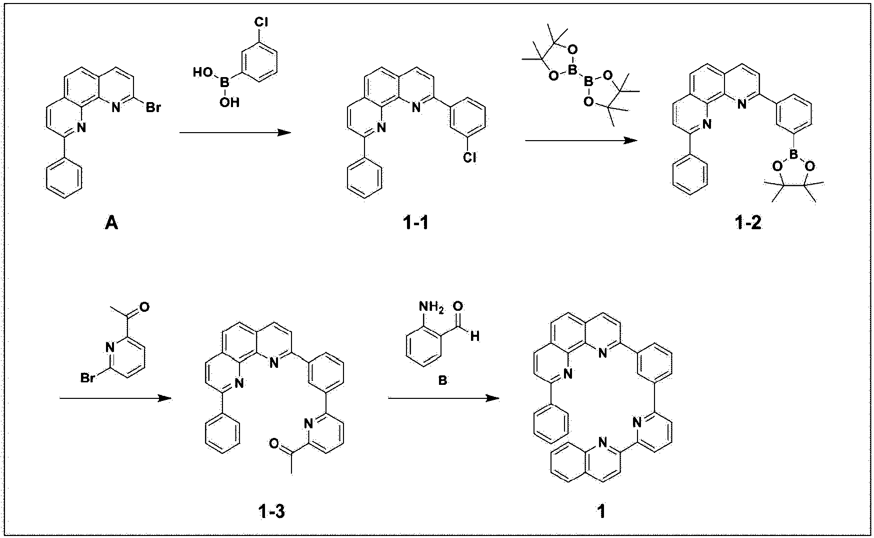

### 1) Preparation of Compound 1-1

Compound A (2-bromo-9-phenyl-1,10-phenanthroline) (50 g, 0.149 mol, 1 eq), (3-cholrophenyl)boronic acid (26.2 g, 0.164 mol, 1.1 eq), Pd(PPh₃)₄ (8.61g 0.007 mol, 0.05 eq), K₂CO₃ (41.2 g, 0.298 mol, 2.0 eq), toluene (500 ml), EtOH (ethanol) (100 ml), and water (100 ml) were put into a container, and the resulting mixture was stirred at 100°C for 6 hours. After the reaction was terminated by adding distilled water, extraction was performed using dichloromethane and distilled water. Thereafter, moisture was removed with MgSO₄. The residue was separated by a silicagel column to obtain 49.2 g of Compound 1-1 with a yield of 90%.

### 2) Preparation of Compound 1-2

Compound 1-1 (49.1 g, 0.134 mol, 1 eq), 4,4,5,5-tetramethyl-2-(4,4,5,5-tetramethyl-1,3,2-dioxaborolan-2-yl)-1,3,2-dioxaborolane (37.8 g, 0.147 mol, 1.1 eq), Pd₂(dba)₃ (4.23 g, 0.007 mol, 0.05 eq), Xphos (6.38g, 0.013mmol, 0.1eq), KOAc (potassium acetate) (32.8 g, 0.335 mol, 2.5 eq), and 1,4-dioxane (375 ml) were put into a container, and the resulting mixture was stirred at 100°C for 6 hours. After the reaction was terminated by adding distilled water, extraction was performed using dichloromethane and distilled water. Thereafter, moisture was removed with MgSO₄. The residue was separated by a silicagel column to obtain 48.0 g of Compound 1-2 with a yield of 78%.

### 3) Preparation of Compound 1-3

Compound 1-2 (26.0 g, 0.057 mol, 1 eq), 1-(6-bromo-2-pyridyl)ethanone (12.5 g, 0.062 mol, 1.1 eq), Pd(PPh₃)₄ (3.29 g, 0.003 mol, 0.05 eq), K₂CO₃ (15.8 g, 0.114 mol, 2.0 eq), 1,4-dioxane (250 ml), and water (50 ml) were put into a container, and the resulting mixture was stirred at 100°C for 10 hours. After the reaction was terminated by adding distilled water, extraction was performed using dichloromethane and distilled water. Thereafter, moisture was removed with MgSO₄. The residue was separated by a silicagel column to obtain 20.2 g of Compound 1-3 with a yield of 79%.

### 4) Preparation of Compound 1

Compound 1-3 (20.0 g, 0.045 mol, 1 eq), Compound B (2-aminobenzaldehyde) (10.7 g, 0.089 mol, 2.0 eq), NaOH (5.32 g, 0.133 mol, 3.0 eq), and isopropyl alcohol (IPA) (200 ml) were put into a container, and the resulting mixture was stirred at 90°C for 24 hours. After the reaction was terminated by adding distilled water, extraction was performed using dichloromethane and distilled water. Thereafter, moisture was removed with MgSO₄. The residue was separated by a silicagel column to obtain 10.5 g of Compound 1 with a yield of 44%.

The compounds in the following Table 1 were obtained by performing synthesis in the same manner as in Preparation Example 1, except that Intermediates A and B in the following Table 1 were used instead of Compounds A and B.

**[Table 1]**

| Compound No. | Intermediate A | Intermediate B | Yield |
|---|---|---|---|
| 2 | 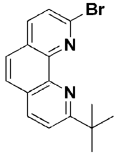 | 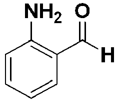 | 81% |
| 3 | 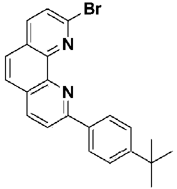 | 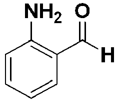 | 78% |
| 4 | 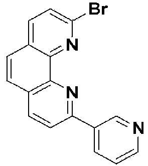 | 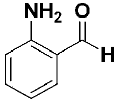 | 80% |
| 6 | 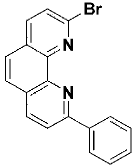 | 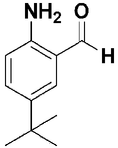 | 82% |
| 19 | 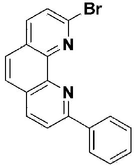 | 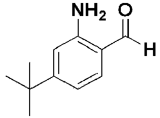 | 71% |
| 30 | 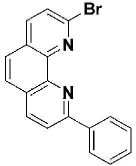 | 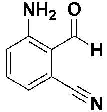 | 52% |
| 124 | 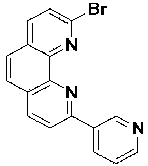 | 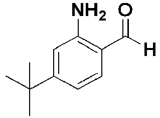 | 65% |

### <Preparation Example 2> Preparation of Intermediate C

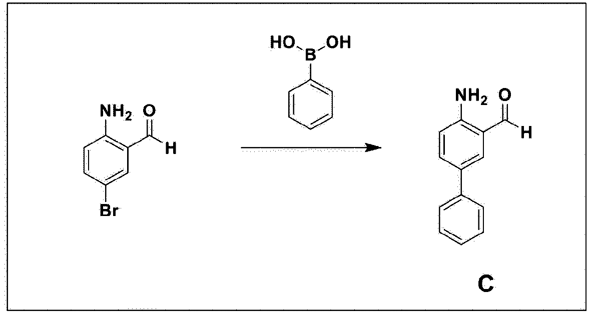

2-Amino-5-bromobenzaldehyde (30.0 g, 0.150 mol, 1 eq), phenylboronic acid (21.9 g, 0.180 mol, 1.2 eq), Pd(PPh₃)₄ (8.67 g, 0.008 mol, 0.05 eq), K₂CO₃ (41.5 g, 0.300 mol, 2.0 eq), 1,4-dioxane (300 ml), and water (60 ml) were put into a container, and the resulting mixture was stirred at 100°C for 5 hours. After the reaction was terminated by adding distilled water, extraction was performed using dichloromethane and distilled water. Thereafter, moisture was removed with MgSO₄. The residue was separated by a silicagel column to obtain 26.1 g of Intermediate C with a yield of 88%.

According to a structure to be synthesized, 2-amino-4-bromobenzaldehyde or 2-amino-6-bromobenzaldehyde was used instead of 2-amino-5-bromobenzaldehyde, and pyridin-2-ylboronic acid or pyridin-4-ylboronic acid was used instead of phenylboronic acid in Preparation Example 2 to obtain Intermediate C in the following Table 2 by performing synthesis in the same manner.

### <Preparation Example 3> Preparation of Compound 8

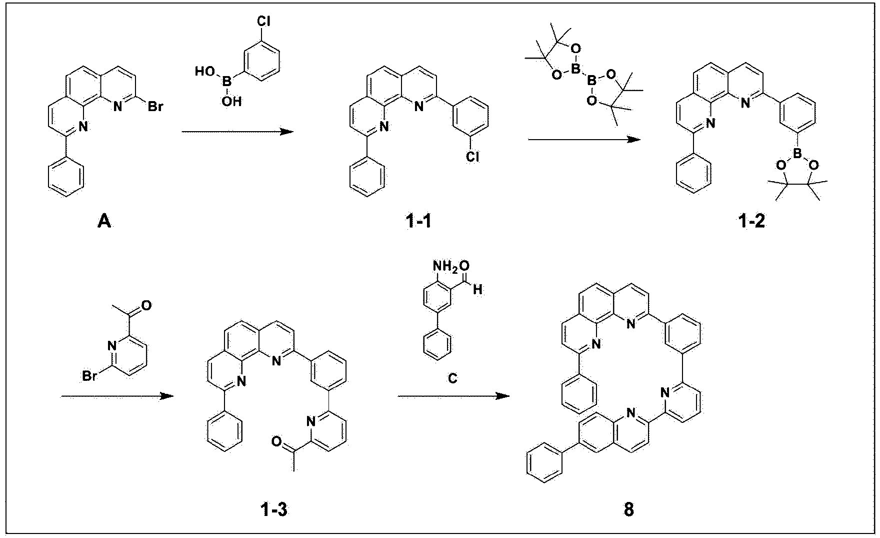

Compound 1-3 (20.0 g, 0.045 mol, 1 eq) prepared in Preparation Example 1, Compound C (17.8 g, 0.089 mmol) prepared in Preparation Example 2, NaOH (5.32 g, 0.133 mol, 3.0 eq), and IPA (200 ml) were put into a container, and the resulting mixture was stirred at 90°C for 24 hours. After the reaction was terminated by adding distilled water, extraction was performed using dichloromethane and distilled water. Thereafter, moisture was removed with MgSO₄. The residue was separated by a silicagel column to obtain 14.7 g of Compound 8 with a yield of 53%.

The compound in the following Table 2 was obtained by using a compound obtained by performing synthesis in the same manner as in Preparation Example 1 using Intermediate A in the following Table 2 instead of Compound A in Preparation Example 1, instead of Compound 1-3 in Preparation Example 3, and performing synthesis in the same manner as in Preparation Example 1 by using Intermediate C in the following Table 2 instead of Compound C in Preparation Example 3.

**[Table 2]**

| Compound No. | Intermediate A | Intermediate C | Yield |
|---|---|---|---|
| 9 | 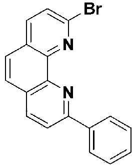 | 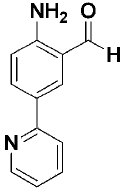 | 43% |
| 21 | 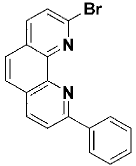 | 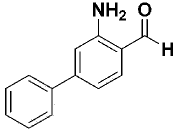 | 61% |
| 22 | 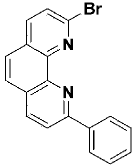 | 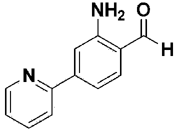 | 71% |
| 32 | 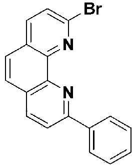 | 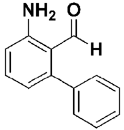 | 33% |
| 43 | 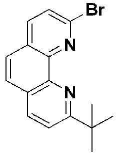 | 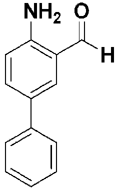 | 67% |
| 57 | 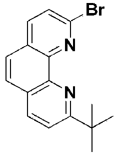 | 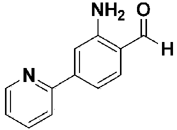 | 49% |
| 58 | 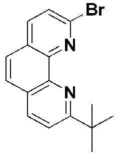 | 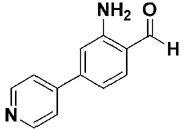 | 46% |
| 79 | 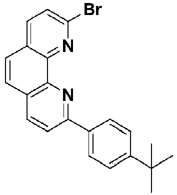 | 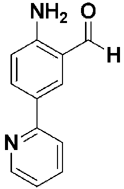 | 41% |
| 92 | 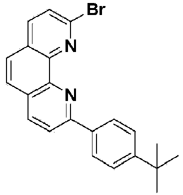 | 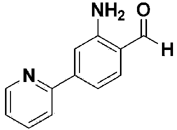 | 47% |
| 113 | 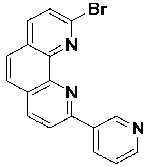 | 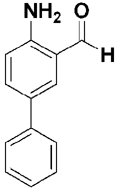 | 67% |
| 139 | 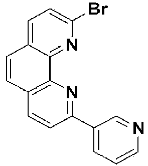 | 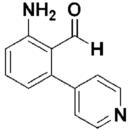 | 39% |

### <Preparation Example 4> Preparation of Intermediate C

### 1) Intermediate of Compounds 145, 146, 150, 156, 161, 162, 168, 171, 173, 191, 209, 212, and 214

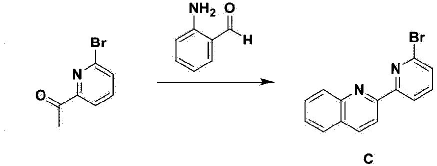

1-(6-bromo-2-pyridyl)ethanone (20.0 g, 0.100 mol, 1 eq), 2-aminobenzaldehyde (12.1 g, 0.100 mol, 1.0 eq), NaOH (6.00 g, 0.150 mol, 1.5 eq), and EtOH (200 ml) were put into a container, and the resulting mixture was stirred at 60°C for 2 hours. After the reaction was terminated by adding distilled water, extraction was performed using dichloromethane and distilled water. Thereafter, moisture was removed with MgSO₄. The residue was separated by a silicagel column to obtain 17.3 g of Intermediate C with a yield of 61%.

According to a structure to be synthesized, synthesis was performed in the same manner as in Preparation Example 4 using 3-amino-[1,1'-biphenyl]-4-carbaldehyde, 2-amino-5-(pyridin-2-yl)benzaldehyde, or 2-amino-4-(pyridin-2-yl)benzaldehyde instead of 2-aminobenzaldehyde to obtain Intermediate C in the following Table 3.

### 2) Intermediate C of Compound 321

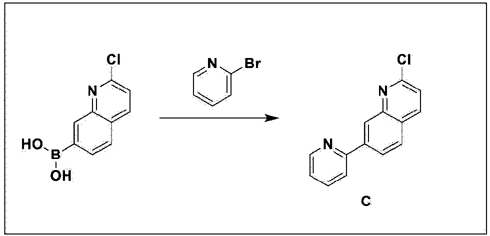

(2-chloroquinolin-7-yl)boronic acid (30.0 g, 0.145 mol, 1 eq), 2-bromopyridine (25.1 g, 0.159 mol, 1.1 eq), Pd(PPh₃)₄ (8.38 g, 0.007 mol, 0.05 eq), K₂CO₃ (40.1 g, 0.290 mol, 2.0 eq), 1,4-dioxane (300ml), and water (60 ml) were put into a container, and the resulting mixture was stirred at 100°C for 4 hours. After the reaction was terminated by adding distilled water, extraction was performed using dichloromethane and distilled water. Thereafter, moisture was removed with MgSO₄. The residue was separated by a silicagel column to obtain 25.3 g of Intermediate C with a yield of 72%.

### <Preparation Example 5> Preparation of Compound 145

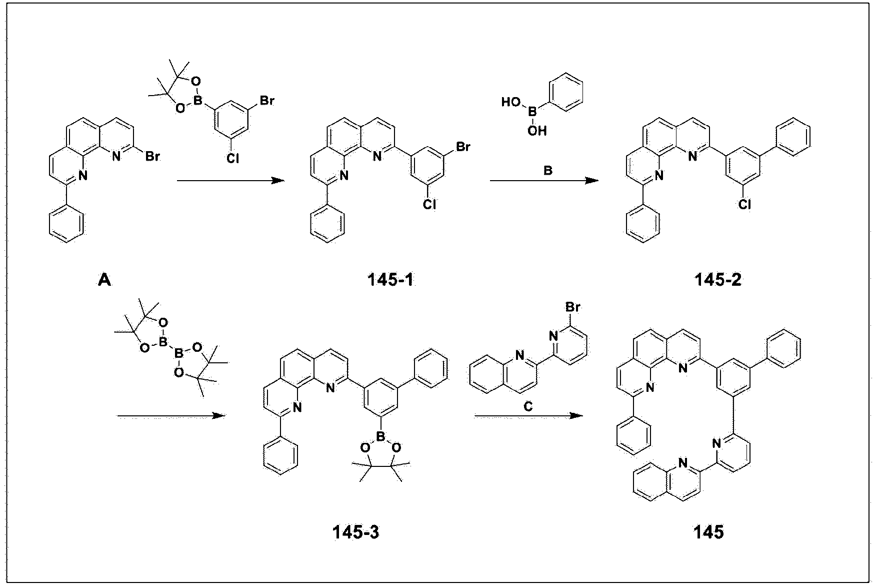

### 1) Preparation of Compound 145-1

Compound A (2-bromo-9-phenyl-1,10-phenanthroline) (30 g, 0.089 mol, 1 eq), 2-(3-bromo-5-chloro-phenyl)-4,4,5,5-tetramethyl-1,3,2-dioxaborolane (31.2 g, 0.098 mol, 1.1 eq), Pd(PPh₃)₄ (5.14 g, 0.004 mol, 0.05 eq), K₃PO₄ (38.0 g, 0.179 mol, 2.0 eq), 1,4-dioxane (300 ml), and water (60 ml) were put into a container, and the resulting mixture was stirred at 100°C for 3 hours. After the reaction was terminated by adding distilled water, extraction was performed using dichloromethane and distilled water. Thereafter, moisture was removed with MgSO₄. The residue was separated by a silicagel column to obtain 32.9 g of Compound 145-1 with a yield of 83%.

### 2) Preparation of Compound 145-2

Compound 145-1 (25.0 g, 0.056 mol, 1 eq), Compound B (phenyl boronic acid) (7.68 g, 0.062 mol, 1.1 eq), Pd(PPh+)₄ (3.24 g, 0.003 mol, 0.05 eq), Na₂CO₃ (11.9 g, 0.112 mol, 2.0 eq), 1,4-dioxane (250 ml), and water (50 ml) were put into a container, and the resulting mixture was stirred at 100°C for 10 hours. After the reaction was terminated by adding distilled water, extraction was performed using dichloromethane and distilled water. Thereafter, moisture was removed with MgSO₄. The residue was separated by a silicagel column to obtain 22.0 g of Compound 145-2 with a yield of 89%.

### 3) Preparation of Compound 145-3

Compound 145-2 (22.0 g, 0.050 mol, 1 eq), 4,4,5,5-tetramethyl-2-(4,4,5,5-tetramethyl-1,3,2-dioxaborolan-2-yl)-1,3,2-dioxaborolane (14.0 g, 0.055 mol, 1.1 eq), Pd₂(dba)₃ (1.43 g, 0.002 mol, 0.05 eq), XPhos (2.37 g, 0.005 mmol, 0.1 eq), KOAc (12.2 g, 0.124 mol, 2.5 eq), and 1,4-dioxane (250 ml) were put into a container, and the resulting mixture was stirred at 100°C for 6 hours. After the reaction was terminated by adding distilled water, extraction was performed using dichloromethane and distilled water. Thereafter, moisture was removed with MgSO₄. The residue was separated by a silicagel column to obtain 19.0 g of Compound 145-3 with a yield of 72%.

### 4) Preparation of Compound 145

Compound 145-3 (18.0 g, 0.034 mol, 1 eq), Compound C (2-(6-bromopyridin-2-yl)quinoline) (9.82 g, 0.041 mol, 1.2 eq), Pd(PPh₃)₄ (1.95 g, 0.002 mol, 0.05 eq), K₂CO₃ (9.31 g, 0.067 mol, 2.0 eq), 1,4-dioxane (200 ml), and water (40 ml) were put into a container, and the resulting mixture was stirred at 100°C for 24 hours. After the reaction was terminated by adding distilled water, extraction was performed using dichloromethane and distilled water. Thereafter, moisture was removed with MgSO₄. The residue was separated by a silicagel column to obtain 13.0 g of Compound 145 with a yield of 63%.

The compounds in the following Table 3 were obtained by performing synthesis in the same manner as in Preparation Example 5, except that Intermediates A to C in the following Table 3 were used instead of Compounds A to C in Preparation Example 5.

**[Table 3]**

| Compound No. | Intermediate A | Intermediate B | Intermediate C | Yield |
|---|---|---|---|---|
| 146 | 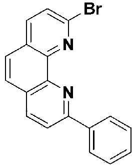 | 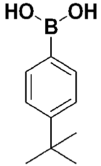 | 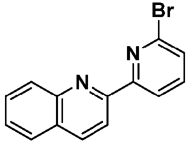 | 58% |
| 148 | 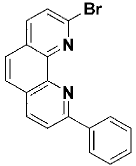 | 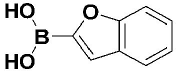 | 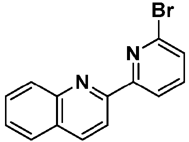 | 62% |
| 150 | 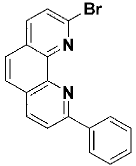 | 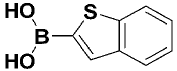 | 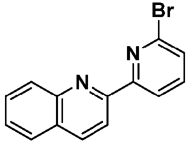 | 60% |
| 156 | 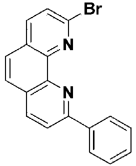 | 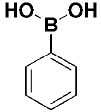 | 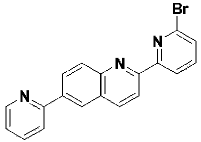 | 54% |
| 161 | 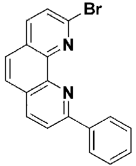 | 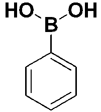 | 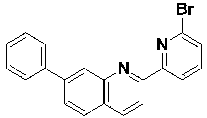 | 60% |
| 162 | 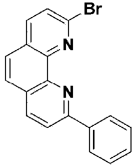 | 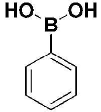 | 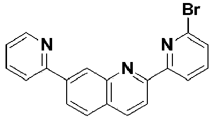 | 51% |
| 168 | 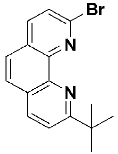 | 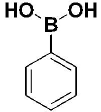 | 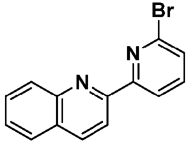 | 69% |
| 171 | 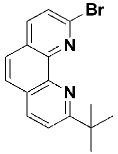 | 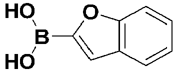 | 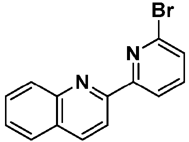 | 53% |
| 173 | 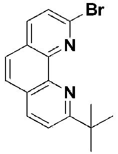 | 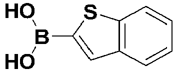 | 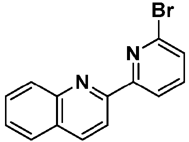 | 51% |
| 191 | 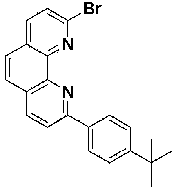 | 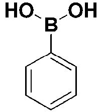 | 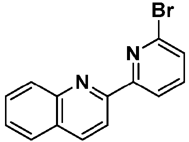 | 66% |
| 209 | 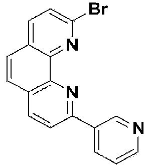 | 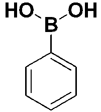 | 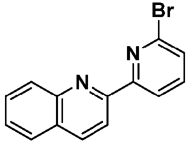 | 65% |
| 212 | 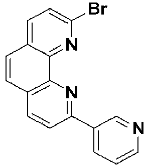 | 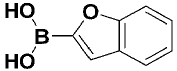 | 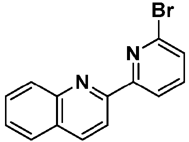 | 52% |
| 214 | 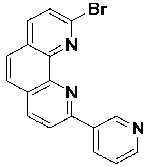 | 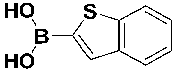 | 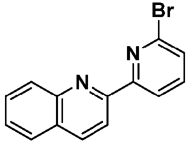 | 57% |
| 321 | 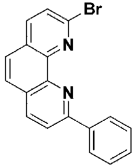 | 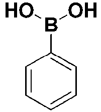 | 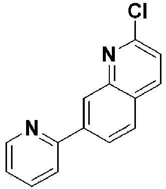 | 61% |

### <Preparation Example 6> Preparation of Compound 147

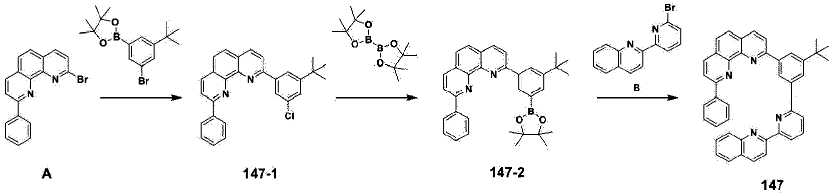

Compound 147 was obtained by performing synthesis in the same manner as in Preparation Example 5, except that in the preparation of Compound 145-1 in 1) Preparation Example 5, 2-(3-bromo-5-(tert-butyl)phenyl)-4,4,5,5-tetramethyl-1,3,2-dioxaborolane was used instead of 2-(3-bromo-5-chloro-phenyl)-4,4,5,5-tetramethyl-1,3,2-dioxaborolane. The compounds in the following Table 4 were obtained by performing synthesis in the same manner as in Preparation Example 6, except that Intermediates A and B in the following Table 4 were used instead of Compounds A and B.

**[Table 4]**

| Compound No. | Intermediate A | Intermediate B | Yield |
|---|---|---|---|
| 170 | 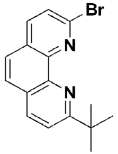 | 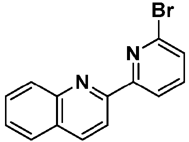 | 44% |
| 211 | 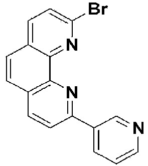 | 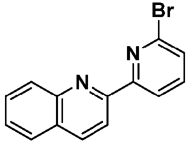 | 47% |
| 323 | 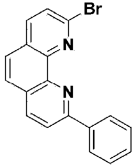 | 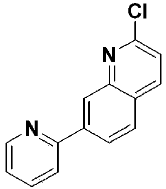 | 71% |

### <Preparation Example 7> Preparation of Intermediate B

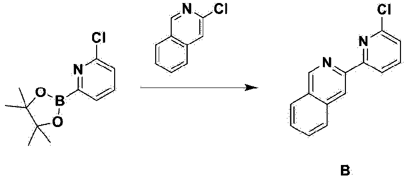

2-Chloro-6-(4,4,5,5-tetramethyl-1,3,2-dioxaborolan-2-yl)pyridine (30.0 g, 0.125 mol, 1 eq), 3-chloroisoquinoline (22.5 g, 0.138 mol, 1.1 eq), Pd(PPh₃)₄ (7.22 g, 0.006 mol, 0.05 eq), K₂CO₃ (34.6 g, 0.250 mol, 2.0 eq), 1,4-dioxane (300ml), and water (60 ml) were put into a container, and the resulting mixture was stirred at 100°C for 4 hours. After the reaction was terminated by adding distilled water, extraction was performed using dichloromethane and distilled water. Thereafter, moisture was removed with MgSO₄. The residue was separated by a silicagel column to obtain 23.3 g of Intermediate B with a yield of 77%.

According to a structure to be synthesized, Intermediate B in the following Table 5 was obtained by performing synthesis in the same manner as in Preparation Example 7, using 3-chloro-7-phenylisoquinoline or 1-chloroisoquinoline instead of 3-chloroisoquinoline.

### <Preparation Example 8> Preparation of Compound 232

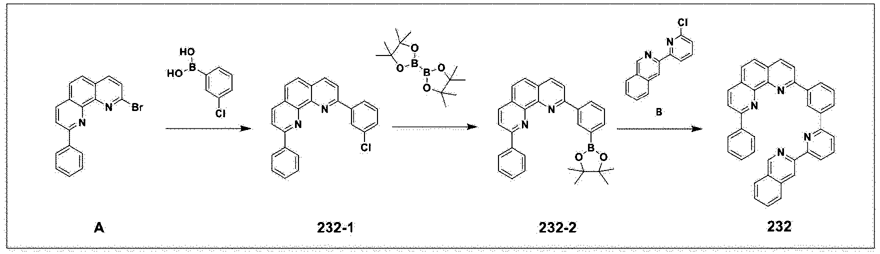

### 1) Preparation of Compound 232-1

Compound A (2-bromo-9-phenyl-1,10-phenanthroline) (50 g, 0.149 mol, 1 eq), (3-cholrophenyl)boronic acid (26.2 g, 0.164 mol, 1.1 eq), Pd(PPh₃)₄ (8.61 g, 0.007 mol, 0.05 eq), K₂CO₃ (41.2 g, 0.298 mol, 2.0 eq), toluene (500 ml), EtOH (100ml), and water (100 ml) were put into a container, and the resulting mixture was stirred at 100°C for 6 hours. After the reaction was terminated by adding distilled water, extraction was performed using dichloromethane and distilled water. Thereafter, moisture was removed with MgSO₄. The residue was separated by a silicagel column to obtain 49.2 g of Compound 232-1 with a yield of 90%.

### 2) Preparation of Compound 232-2

Compound 232-1 (49.1 g, 0.134 mol, 1 eq), 4,4,5,5-tetramethyl-2-(4,4,5,5-tetramethyl-1,3,2-dioxaborolan-2-yl)-1,3,2-dioxaborolane (37.8 g, 0.147 mol, 1.1 eq), Pd₂(dba)₃ (4.23 g, 0.007 mol, 0.05 eq), Xphos (6.38 g, 0.013 mmol, 0.1 eq), KOAc (32.8 g, 0.335 mol, 2.5 eq), and 1,4-dioxane (375 ml) were put into a container, and the resulting mixture was stirred at 100°C for 6 hours. After the reaction was terminated by adding distilled water, extraction was performed using dichloromethane and distilled water. Thereafter, moisture was removed with MgSO₄. The residue was separated by a silicagel column to obtain 48.0 g of Compound 232-2 with a yield of 78%.

### 3) Preparation of Compound 232

Compound 232-2 (15.0 g, 0.033 mol, 1 eq), Compound B (3-(6-chloropyridin-2-yl)isoquinoline) (8.66 g, 0.036 mol, 1.1 eq), Pd(PPh₃)₄ (1.91 g, 0.002 mol, 0.05 eq), K₂CO₃ (9.12 g, 0.066 mol, 2.0 eq), 1,4-dioxane (150ml), and water (30 ml) were put into a container, and the resulting mixture was stirred at 100°C for 24 hours. After the reaction was terminated by adding distilled water, extraction was performed using dichloromethane and distilled water. Thereafter, moisture was removed with MgSO₄. The residue was separated by a silicagel column to obtain 15.0 g of Compound 232 with a yield of 85%.

The compounds in the following Table 5 were obtained by performing synthesis in the same manner as in Preparation Example 8, except that Intermediate A in the following Table 5 was used instead of Compounds A and B, and Intermediate B was used instead of 3-(6-chloropyridin-2-yl)isoquinoline (B).

**[Table 5]**

| Compound No. | Intermediate A | Intermediate B | Yield |
|---|---|---|---|
| 236 | 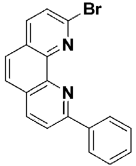 | 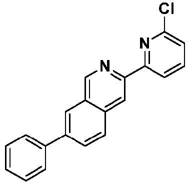 | 60% |
| 240 | 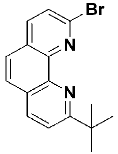 | 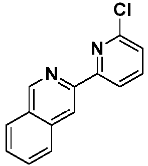 | 51% |
| 270 | 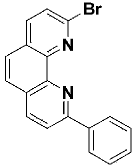 | 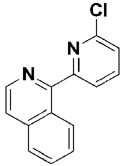 | 45% |

### <Preparation Example 9> Preparation of Compound 306

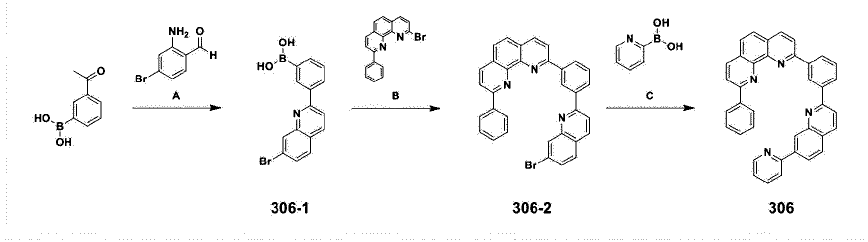

### 1) Preparation of Compound 306-1

(3-acetylphenyl)boronic acid (20 g, 0.122 mol, 1 eq), Compound A (2-amino-4-bromobenzaldehyde) (24.4 g, 0.122 mol, 1.0 eq), NaOH (7.32 g, 0.183 mol, 1.5 eq), and EtOH (ethanol) (200ml) were put into a container, and the resulting mixture was stirred at 60°C for 2 hours. After the reaction was terminated by adding distilled water, extraction was performed using dichloromethane and distilled water. Thereafter, moisture was removed with MgSO₄. The residue was separated by a silicagel column to obtain 34.0 g of Compound 306-1 with a yield of 85%.

### 2) Preparation of Compound 306-2

Compound 306-1 (28.3 g, 0.086 mol, 1.2 eq), Compound B (2-bromo-9-phenyl-1,10-phenanthroline) (25.0 g, 0.75 mol, 1.0 eq), Pd₂(dba)₃ (4.30 g, 0.004 mol, 0.05 eq), K₂CO₃ (20.6 g, 0.149 mol, 2.0 eq), 1,4-dioxane (300ml), and water (60 ml) were put into a container, and the resulting mixture was stirred at 100°C for 6 hours. After the reaction was terminated by adding distilled water, extraction was performed using dichloromethane and distilled water. Thereafter, moisture was removed with MgSO₄. The residue was separated by a silicagel column to obtain 34.0 g of Compound 306-2 with a yield of 84%.

### 3) Preparation of Compound 306

Compound 306-2 (15.0 g, 0.028 mol, 1 eq), Compound C (pyridin-2-ylboronic acid) (3.77 g, 0.031 mol, 1.1 eq), Pd(PPh₃)₄ (1.61 g, 0.001 mol, 0.05 eq), K₂CO₃ (7.70 g, 0.056 mol, 2.0 eq), 1,4-dioxane (150 ml), and water (30 ml) were put into a container, and the resulting mixture was stirred at 100°C for 6 hours. After the reaction was terminated by adding distilled water, extraction was performed using dichloromethane and distilled water. Thereafter, moisture was removed with MgSO₄. The residue was separated by a silicagel column to obtain 7.0 g of Compound 306 with a yield of 47%.

The compounds in the following Table 6 were obtained by performing synthesis in the same manner as in Preparation Example 9, except that Intermediates A to C in the following Table 6 were used instead of Compounds A to C.

**[Table 6]**

| Compound No. | Intermediate A | Intermediate B | Intermediate C | Yield |
|---|---|---|---|---|
| 307 | 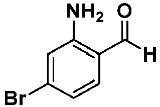 | 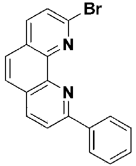 | 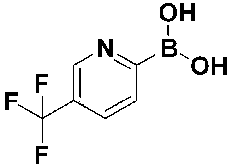 | 65% |
| 309 | 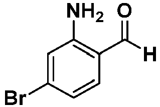 | 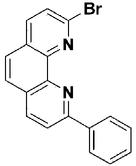 | 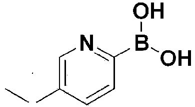 | 58 |
| 313 | 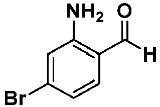 | 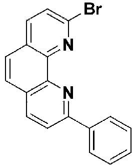 | 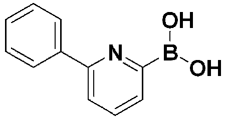 | 61 |
| 369 | 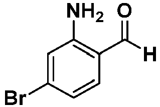 | 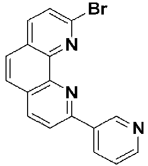 | 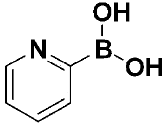 | 66 |
| 371 | 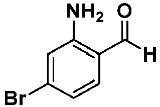 | 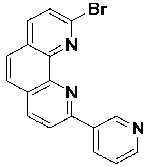 | 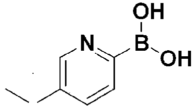 | 62 |
| 390 | 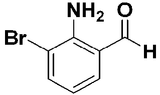 | 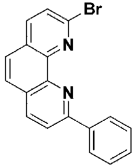 | 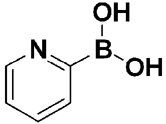 | 70 |

### <Preparation Example 10> Preparation of Compound 401

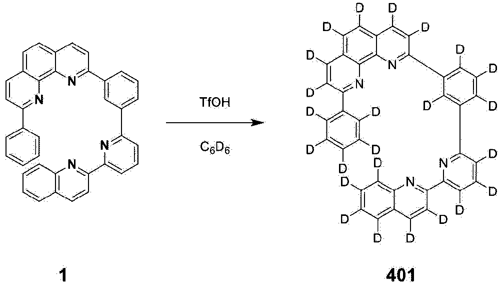

Compound 1 (5 g, 0.01 mol, 1 eq) in Preparation Example 1, triflic acid (5.76 ml 0.065 mol, 7 eq), and C6D6 (25 ml) were put into a container, and the resulting mixture was stirred at 60°C for 2 hours. D₂O was added thereto, the mixture was stirred for 30 minutes, and then the mixture was neutralized by adding triethylamine dropwise thereto. After neutralization, extraction was performed using dichloromethane. Thereafter, moisture was removed with MgSO₄. The residue was separated by a silicagel column to obtain 3.4 g of Compound 401 with a yield of 65%.

### <Preparation Example 11> Preparation of Compound 402

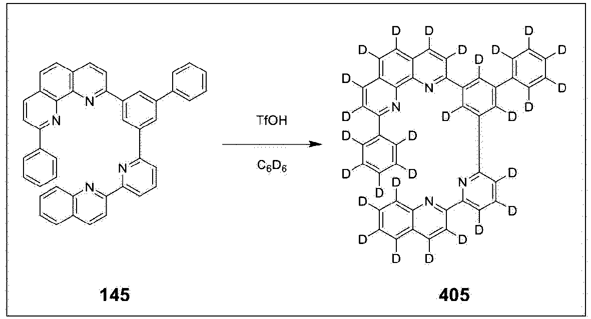

Compound 405 was obtained by performing synthesis in the same manner as in Preparation Example 10, except that Compound 145 in Preparation Example 5 was used instead of Compound 1.

### <Preparation Example 12> Preparation of Compound 411

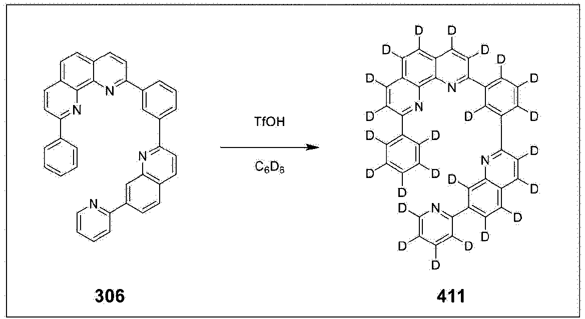

Compound 411 was obtained by performing synthesis in the same manner as in Preparation Example 10, except that Compound 306 in Preparation Example 9 was used instead of Compound 1.

It was confirmed through ¹H-NMR and FD-mass spectrometry that the compounds synthesized in the Preparation Examples were synthesized into the desired compounds. The measured values of ¹H NMR (CDCl₃, 200 MHz) are shown in the following Table 7, and the measured values of field desorption mass spectrometry (FD-Mass) are shown in the following Table 8.

**[Table 7]**

| Compound No. | ¹H NMR(CDCl₃, 200Mz) |
|---|---|
| 1 | δ= 9.40(1H, s), 8.89(1H, d), 8.72(1H, d), 8.54-8.51(3H, m), 8.44 (1H, d), 8.36-8.21(5H, m), 8.15(2H, t), 8.04 (1H, t), 7.85(1H, d), 7.80(2H, s), 7.76(2H, t), 7.58-7.51(3H, m), 7.45(1H, t) |
| 2 | δ= 8.78(1H, d), 8.72-8.71(2H, m), 8.41-8.37(2H, m), 8.33(2H, d), 8.20(1H, d), 8.09(1H, d), 8.00-7.95(2H, m), 7.90(1H, d), 7.83(1H, t), 7.73(1H, t), 7.53(1H, t), 7.37 (1H, t), 7.30-7.29(2H, m), 6.88 (1H, dd), 1.36(9H, s) |
| 3 | δ= 8.78 (1H, d), 8.72-8.71(2H, m), 8.41-8.33(4H, m), 8.20(1H, d), 8.00-7.90(5H, m), 7.83 (1H, t), 7.73(1H, t), 7.53 (1H, t), 7.38-7.36(3H, m), 7.29(2H, d), 6.88 (1H, dd), 1.33(9H, s) |
| 4 | δ= 9.63(1H, s), 8.81-8.65(6H, m), 8.41-8.33(3H, m), 8.14(1H, d), 8.00-7.96(2H, m), 7.89-7.83(2H, m), 7.73(1H, t), 7.63-7.53(3H, m), 7.37(1H, t), 7.29(1H, d), 6.88(1H, d) |
| 6 | δ= 8.78 (1H, d), 8.72-8.71(3H, m), 8.33-8.27 (6H, m), 8.20(1H, d), 7.91-7.985(3H, m), 7.73-7.70(2H, m), 7.55-7.49(3H, m), 7.37(1H, t), 7.29(2H, d), 6.88(1H, d), 1.43(9H, s) |
| 8 | δ= 8.78 (1H, d), 8.72-8.71(3H, m), 8.43(1H, d), 8.35-8.33(6H, m), 8.20(1H, d), 8.07 (1H, d), 7.90-7.88 (2H, m), 7.75-7.73(3H, m), 7.55-7.49(5H, m), 7.41-7.37(2H, m), 7.29 (2H, d), 6.88(1H, d) |
| 9 | δ= 8.80-8.78(2H, m), 8.72-8.71(3H, m), 8.41-8.33(8H, m), 8.27(1H, d), 8.20(1H, d), 7.90(1H, d), 7.73(1H, t), 7.55-7.49(3H, m), 7.38-7.37(2H, m), 7.29(2H, d), 7.14(1H, d), 6.90-6.88(2H, m) |
| 19 | δ= 8.78(1H, d), 8.72-8.71(3H, m), 8.33-8.31(5H, m), 8.25-8.20(2H, m), 7.90(1H, d), 7.84(1H, d), 7.73(1H, t), 7.55-7.46(5H, m), 7.37(1H, t), 7.29(2H, d), 6.88(1H, d), 1.38(9H, s) |
| 21 | δ= 8.78(1H, d), 8.72-8.71(3H, m), 8.43-8.39(3H, m), 8.33(4H, d), 8.25(1H, s), 8.20(1H, d) 7.90-7.89 (2H, m), 7.75-7.73(3H, m), 7.55-7.37(7H, m), 7.29(2H, d), 6.88 (1H, d) |
| 22 | δ= 8.78-8.76(2H, m), 8.72-8.71(3H, m), 8.62(1H, d), 8.41-8.33(8H, m), 8.20(1H, d), 7.90(1H, d), 7.73(1H, t), 7.55-7.49(3H, m), 7.38-7.37(2H, m), 7.29(2H, d), 7.14(1H, d), 6.90-6.88(2H, m) |
| 30 | δ= 8.78-8.71(5H, m), 8.62(1H, d), 8.33(4H, dd), 8.20-8.19(2H, m), 7.90-7.85(3H, m), 7.73(1H, t), 7.55-7.49(3H, m), 7.37(1H, t), 7.29(2H, d), 6.88(1H, d) |
| 32 | δ= 8.78 (1H, d), 8.72-8.71(3H, m), 8.35-8.33(6H, m), 8.20(1H, d), 7.90-7.85(3H, m), 7.79-7.73(4H, m), 7.55-7.37(7H, m), 7.29(2H, d), 6.88(1H, d) |
| 43 | δ= 8.78(1H, d), 8.72-8.71(2H, m), 8.43(1H, d), 8.35-8.33(4H, m), 8.20(1H, d), 8.09-8.07(2H, m), 7.90-7.88(2H, m), 7.75-7.73(3H, m), 7.49-7.32(4H, m), 7.30-7.29(2H, m), 6.88(1H, d), 1.36(9H) |
| 57 | δ= 8.79-8.77(2H, m), 8.72-8.71(2H, m), 8.62(1H, d), 8.41-8.33(6H, m), 8.20(1H, d), 8.09(1H, d), 7.90(1H, d), 7.73(1H, t), 7.38-7.37(2H, m), 7.30-7.29(2H, m), 7.14(1H, d), 6.90-6.88(2H, m), 1.36(9H) |
| 58 | δ= 8.78 (1H, d), 8.72-8.70(4H, m), 8.41-8.33(6H, m), 8.23-8.20(2H, m), 8.09(1H, d), 8.00(2H, d), 7.90(1H, d), 7.73(1H, t), 7.37 (1H, t), 7.31-7.28(2H, m), 6.88(1H, d), 1.36(9H) |
| 79 | δ= 8.80-8.78(2H, m), 8.72-8.70(3H, m), 8.41-8.33(6H, m), 8.27-8.20(2H, m), 7.95(2H, d), 7.90(1H, d), 7.73(1H, t), 7.39-7.36(4H, m), 7.29(2H, d), 7.14(1H, d), 6.91-6.85(2H, m), 1.33(9H) |
| 92 | δ= 8.79-8.77(2H, m), 8.72-8.70(3H, m), 8.41-8.33(6H, m), 8.20(1H, d), 7.95(2H, d), 7.90(1H, d), 7.73(1H, t), 7.39-7.37(4H, m), 7.29(2H, d), 7.14(1H, d), 6.91-6.88(2H, m), 1.33(9H) |
| 113 | δ= 9.63(1H, s), 8.81-8.78(2H, m), 8.72-8.70(3H, m), 8.65(1H, dd), 8.43-8.33(4H, m), 8.14(1H, d), 8.07(1H, d), 7.90-7.88(2H, m), 7.75-7.73(3H, m), 7.63-7.57(2H, m), 7.49-7.41(3H, m), 7.29(2H, d), 6.88(1H, d) |
| 124 | δ= 9.63(1H, s), 8.81-8.78(2H, m), 8.72-8.70(3H, m), 8.65(1H, d), 8.33-8.31(3H, m), 8.25(1H, d), 8.14 (1H, d), 7.89(1H, d), 7.84 (1H, d), 7.73(1H, t), 7.63 (1H, d), 7.57-7.52(2H, m), 7.46(1H, s), 7.37 (1H, t), 7.29(1H, d), 6.88 (1H, d), 1.38 (9H, s) |
| 139 | δ= 9.63(1H, s), 8.81-8.78(2H, m), 8.72-8.65(6H, m), 8.41-8.33(4H, m), 8.23 (1H, d), 8.14-8.00(5H, m), 7.89(1H, d), 7.73(1H, t), 7.63-7.57(2H, m), 7.37-7.29(2H, m), 6.88(1H, d) |
| 145 | δ= 9.32(1H, s), 9.04(1H, s), 8.81-8.78(2H, m), 8.63(1H, s), 8.58(2H, d), 8.40-8.30(5H, m), 8.22(1H, d), 8.18(1H, d), 8.01-7.97(4H, m), 7.61-7.55(4H, m), 7.50-7.45(2H, m), 7.32-7.30(1H, m) |
| 146 | δ= 8.82-8.78 (2H, m), 8.71(2H, d), 8.43(2H, s), 8.41-8.33(4H, m), 8.20(1H, d), 8.00-7.90(3H, m), 7.83(1H, t), 7.55-7.49(4H, m), 7.38-7.29(7H, m), 6.88 (1H, d), 1.33(9H, s) |
| 147 | δ= 8.78(1H, d), 8.71(2H, d), 8.52(1H, s), 8.42-8.33(6H, m), 8.20(1H, d), 8.02-7.96(2H, m), 7.90(1H, d), 7.83(1H, t), 7.55-7.49(4H, m), 7.37(1H, t), 7.29(2H, d) 6.88(1H, d), 1.32(9H, s) |
| 148 | δ= 8.82-8.78 (2H, m), 8.71(2H, d), 8.43-8.33 (6H, m), 8.20(1H, d), 8.04-7.82(4H, m), 7.59-7.49(6H, m), 7.39-7.37(2H, m), 7.29(2H, d), 7.22(1H, t), 7.14(1H, s), 6.88(1H, d) |
| 150 | δ= 8.83-8.78 (2H, m), 8.70(2H, d), 8.43-8.33 (6H, m), 8.21(1H, d), 8.05-7.90(4H, m), 7.83-7.79(2H, m), 7.59-7.49(6H, m), 7.37-7.28(4H, m), 6.88(1H, d) |
| 156 | δ= 8.84-8.79(3H, m), 8.70(2H, d), 8.45-8.27 (9H, m), 8.19(1H, d), 7.91(1H, d), 7.75(2H, d), 7.55-7.48 (5H, m), 7.42-7.37 (3H, m), 7.30(2H, d), 7.14 (1H, d), 6.92-6.87 (2H, m) |
| 161 | δ= 8.83-8.77(2H, m), 8.72(2H, d), 8.44-8.34(7H, m), 8.26(1H, s), 8.21(1H, d), 7.91-7.89(2H, m), 7.77-7.73(4H, m), 7.56-7.36(10H, m), 7.30(2H, d), 6.89(1H, d) |
| 162 | δ= 8.84-8.77 (3H, m), 8.71(2H, d), 8.62(1H, d), 8.45-8.31(8H, m), 8.19(1H, d), 7.91(1H, d), 7.74(2H, d), 7.57-7.35(8H, m), 7.30(2H, d), 7.14(1H, d), 6.89(1H, d) |
| 168 | δ= 8.82(1H, s), 8.78(1H, d), 8.71(1H, d), 8.43(2H, s), 8.41-8.37(2H, m), 8.20(1H, d), 8.09(1H, d), 8.01-7.95(2H, m), 7.91-7.83(2H, m), 7.75(2H, d), 7.54-7.48(3H, m), 7.41-7.29(4H, m), 6.88(1H, d), 1.37(9H, s) |
| 170 | δ= 8.78(1H, d), 8.71(1H, d), 8.52(1H, s), 8.44-8.36(4H, m), 8.19(1H, d), 8.10(1H, d), 8.01-7.90(3H, m), 7.83(1H, t), 7.53(1H, t), 7.38(1H, t), 7.30-7.29(2H, m), 6.88(1H, d), 1.36(9H, s), 1.32(9H, s) |
| 171 | δ= 8.82(1H, s), 8.79(1H, d), 8.71(1H, d), 8.44 (2H, s), 8.41-8.36(2H, m), 8.20(1H, d), 8.10(1H, d), 8.03-7.95(2H, m), 7.90-7.83(2H, m), 7.60-7.53(3H, m), 7.39-7.37(2H, m), 7.30-7.28(2H, m), 7.22(1H, t), 7.14(1H, s), 6.89(1H, d), 1.36(9H, s) |
| 173 | δ= 8.82(1H, s), 8.78(1H, d), 8.70(1H, d), 8.45(2H, s), 8.41-8.36(2H, m), 8.20(1H, d), 8.10(1H, d), 8.04-7.90(4H, m), 7.83-7.79(2H, m), 7.59(1H, s), 7.53-7.38(3H, m), 7.32(1H, t), 7.30-7.29(2H, m), 6.87(1H, d), 1.37(9H, s) |
| 191 | δ= 8.82-8.77(2H, m), 8.71(2H, d), 8.43(2H, s), 8.41-8.37 (2H, m), 8.21(1H, d), 8.02-7.90(5H, m), 7.83-7.74(3H, m), 7.53-7.41(4H, m), 7.39-7.37(3H, m), 7.29(2H, d) 6.89(1H, d), 1.33(9H, s) |
| 209 | δ= 9.63(1H, s), 8.82-8.78(3H, m), 8.71-8.65(3H, m), 8.43-8.37 (4H, m), 8.14(1H, d), 8.05-7.95(2H, m), 7.89-7.83(2H, m), 7.75(2H, d), 7.63-7.48(5H, m), 7.41-7.29(3H, m), 6.89(1H, d) |
| 211 | δ= 9.63 (1H, s), 8.81-8.78(2H, m), 8.72-8.70(2H, m), 8.65(1H, d), 8.52(1H, s), 8.42-8.37 (4H, m), 8.14 (1H, d), 8.03-7.95(2H, m), 7.89-7.83(2H, m), 7.6-7.53(3H, m), 7.37(1H, t), 7.29(1H, d), 6.89(1H, d), 1.32(9H, s) |
| 212 | δ= 8.82(1H, s), 8.78(1H, d), 8.70(1H, d), 8.45(2H, s), 8.41-8.36(2H, m), 8.20(1H, d), 8.10(1H, d), 8.04-7.90(4H, m), 7.83-7.79(2H, m), 7.59(1H, s), 7.53-7.38(3H, m), 7.32(1H, t), 7.30-7.29(2H, m), 6.87(1H, d), 1.37(9H, s) |
| 214 | δ= 9.63(1H, s), 8.83-8.78 (3H, m), 8.72-8.65(3H, m), 8.44-8.36(4H, m), 8.14 (1H, d), 8.02-7.79(6H, m), 7.63-7.48 (5H, m), 7.37-7.28(3H, m), 6.89 (1H, d) |
| 232 | δ= 9.32(1H, s), 8.78(1H, d), 8.72-8.70(3H, m), 8.62(1H, s), 8.35-8.32(4H, m), 8.20(1H, d), 7.98(1H, d), 7.90(1H, d), 7.73-7.64(3H, m), 7.57-7.48(4H, m), 7.37(1H, t), 7.29(2H, d), 6.89(1H, d) |
| 236 | δ= 9.30(1H, s), 8.78 (1H, d), 8.72-8.67(4H, m), 8.35-8.31(4H, m), 8.20(1H, d), 8.09-8.06(2H, m), 7.90(1H, d), 7.76-7.72(3H, m), 7.69-7.48(6H, m), 7.42-7.36(2H, m), 7.29(2H, d), 6.87(1H, d) |
| 240 | δ= 9.32(1H, s), 8.78(1H, d), 8.73-8.70(2H, m), 8.62(1H, s), 8.33(2H, d), 8.20(1H, d), 8.09(1H, d), 7.98(1H, d), 7.90(1H, d), 7.74-7.56(4H, m), 7.37(1H, t), 7.30-7.29(2H, m), 6.89(1H, d), 1.35(9H, s) |
| 270 | δ= 8.79(1H, d), 8.73-8.71(3H, m), 8.52(1H, d), 8.35-8.32(4H, m), 8.20(1H, d), 7.99(1H, d), 7.90(1H, d), 7.73-7.49(7H, m), 7.39-7.36(2H, m), 7.29(2H, d), 6.87(1H, d) |
| 306 | δ= 9.44 (1H, s), 8.79(2H, d), 8.60(1H, d), 8.54 (2H, d), 8.42-8.31(6H, m), 8.22-8.16(2H, m), 7.99(2H, t), 7.83-7.75(4H, m), 7.56(2H, t), 7.46(1H, t), 7.32-7.29(1H, m) |
| 307 | δ= 8.76-8.71 (5H, m), 8.63(1H, d), 8.40-8.29(6H, m), 8.20(1H, d), 7.90(1H, d), 7.82(1H, d), 7.73(1H, t), 7.56-7.48(3H, m), 7.36-7.28(4H, m) |
| 309 | δ= 8.76(1H, s), 8.73-8.70(4H, m), 8.63(1H, d), 8.39(1H, d), 8.34-8.30(5H, m), 8.20(1H, d), 7.90(1H, d), 7.75-7.72(2H, m), 7.56-7.48(3H, m), 7.31-7.28(4H, m), 1.35(9H, s) |
| 313 | δ= 8.76-8.70(5H, m), 8.63(1H, d), 8.39-8.31(7H, m), 8.20(1H, d), 7.90(1H, d), 7.73(1H, t), 7.57-7.48(6H, m), 7.30-7.28(3H, m), 7.18(1H, t), 6.74(2H, d) |
| 321 | δ= 8.82 (1H, s), 8.76-8.71(4H, m), 8.63(1H, d), 8.44-8.32(6H, m), 8.20(1H, d), 7.90(1H, d), 7.76-7.74 (2H, m), 7.57-7.48(5H, m), 7.42-7.37(2H, m), 7.30-7.29(3H, m), 7.14(1H, d), 6.90(1H, t) |
| 323 | δ= 8.76(1H, s), 8.72-8.70(3H, m), 8.63(1H, d), 8.52 (1H, s), 8.43-8.36(6H, m), 8.20(1H, d), 7.90(1H, d), 7.57-7.48(3H, m), 7.38 (1H, t), 7.30-7.29(3H, m), 7.14(1H, d), 6.90(1H, t), 1.32(9H, s) |
| 369 | δ= 9.63(1H, s), 8.83-8.62(8H, m), 8.39-8.32(4H, m), 8.14 (1H, d), 7.89(1H, d), 7.73 (1H, t), 7.64-7.56(2H, m), 7.38(1H, t), 7.30-7.29(2H, m), 7.14(1H, d), 6.90(1H, t) |
| 371 | δ= 9.36(1H, s), 8.81-8.63(8H, m), 8.39(1H, d), 8.34-8.30(3H, m), 8.14 (1H, d), 7.89(1H, d), 7.75-7.72(2H, m), 7.63-7.57 (2H, m), 7.30-7.29(3H, m), 1.35(9H, s) |
| 390 | δ= 9.04 (1H, d), 8.72-8.70(4H, m), 8.38-8.26(6H, m), 8.20(1H, d), 7.91-7.88(2H, m), 7.73(1H, t), 7.56-7.48(3H, m), 7.38(1H, t), 7.30-7.29(3H, m), 7.14(1H, d), 6.90(1H, t) |

**[Table 8]**

| Compound No. | FD-MS | Compound No. | FD-MS |
|---|---|---|---|
| 1 | m/z= 536.20 (C38H24N4= 536.64) | 2 | m/z= 516.24 (C36H28N4= 516.65) |
| 3 | m/z= 592.26 (C42H32N4= 592.75) | 4 | m/z= 537.20 (C37H23N5= 537.63) |
| 6 | m/z= 592.26(C42H32N4= 592.75) | 8 | m/z= 612.23 (C44H28N4= 612.74) |
| 9 | m/z= 613.23(C43H27N5= 613.72) | 19 | m/z= 592.26 (C42H32N4= 592.75) |
| 21 | m/z= 612.23 (C44H28N4= 612.74) | 22 | m/z= 613.23 (C43H27N5= 613.72) |
| 30 | m/z= 561.20(C39H23N5= 561.65) | 32 | m/z= 612.23 (C44H28N4= 612.74) |
| 43 | m/z= 592.26(C42H32N4= 592.75) | 57 | m/z= 593.26 (C41H31N5= 593.71) |
| 58 | m/z= 593.26(C41H31N5= 593.71) | 79 | m/z= 669.29 (C47H35N5= 669.83) |
| 92 | m/z= 669.29 (C47H35N5= 669.83) | 113 | m/z= 613.23 (C43H27N5= 613.72) |
| 124 | m/z= 593.26(C41H31N5= 593.73) | 139 | m/z= 614.22 (C42H26N6= 614.71) |
| 145 | m/z= 612.23 (C44H28N4= 612.74) | 146 | m/z= 668.29 (C48H36N4= 668.84) |
| 147 | m/z= 592.26(C42H32N4= 592.75) | 148 | m/z= 652.23 (C46H28N4O= 652.76) |
| 150 | m/z= 668.20(C46H28N4S= 668.82) | 156 | m/z= 689.26 (C49H31N5= 689.82) |
| 161 | m/z= 688.26(C50H32N4= 688.83) | 162 | m/z= 689.26 (C49H31N5= 689.82) |
| 168 | m/z= 592.26(C42H32N4= 592.75) | 170 | m/z= 572.29 (C40H36N4= 572.76) |
| 171 | m/z= 632.26(C44H32N4O= 632.77) | 173 | m/z= 648.23 (C44H32N4S= 648.83) |
| 191 | m/z= 668.29 (C48H36N4= 668.84) | 209 | m/z= 613.23 (C43H27N5= 613.72) |
| 211 | m/z= 593.26(C41H31N5= 593.73) | 212 | m/z= 653.22 (C45H27N5O= 653.75) |
| 214 | m/z= 669.20(C45H27N5S= 669.81) | 232 | m/z= 536.20 (C38H24N4= 536.64) |
| 236 | m/z= 612.23 (C44H28N4= 612.74) | 240 | m/z= 516.24 (C36H28N4= 516.65) |
| 270 | m/z= 536.20 (C38H24N4= 536.64) | 306 | m/z= 536.20 (C38H24N4= 536.64) |
| 307 | m/z= 604.19(C39H24F3N4= 604.64) | 309 | m/z= 592.26 (C42H32N4= 592.75) |
| 310 | m/z= 561.20(C39H23N5= 561.65) | 313 | m/z= 612.23 (C44H28N4= 612.74) |
| 321 | m/z= 612.23 (C44H28N4= 612.74) | 323 | m/z= 592.26 (C42H32N4= 592.75) |
| 369 | m/z= 537.20(C37H23N5= 537.63) | 371 | m/z= 593.26 (C41H31N5= 593.73) |
| 390 | m/z= 536.20 (C38H24N4= 536.64) | 401 | m/z= 560.35 (C38D24N4= 560.78) |
| 405 | m/z= 640.41 (C44D28N4= 640.91) | 411 | m/z= 560.35 (C38D24N4= 560.78) |

### <Experimental Examples>

### <Experimental Example 1>

### 1) Manufacture of Organic Light Emitting Device

### <Comparative Example 1>

Trichloroethylene, acetone, ethanol, and distilled water were each sequentially used to ultrasonically wash a transparent electrode indium tin oxide (ITO) thin film obtained from glass for OLED (manufactured by Samsung-Corning Co., Ltd.) for 5 minutes, and then the ITO thin film was placed in isopropanol, stored, and then used. Next, the ITO substrate was disposed in a substrate folder of a vacuum deposition apparatus, and the following 4,4',4"-tris(N,N-(2-naphthyl)-phenylamino)triphenyl amine (2-TNATA) was placed in a cell in the vacuum deposition apparatus.

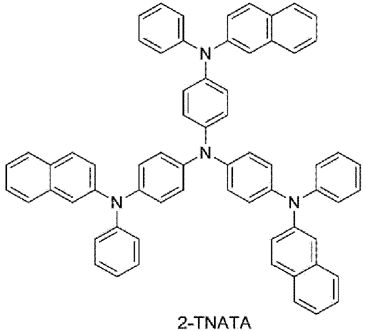

Subsequently, air in the chamber was evacuated until the degree of vacuum in the chamber reached 10⁻⁶ torr, and then a hole injection layer having a thickness of 600 Å was deposited on the ITO substrate by applying current to the cell to evaporate 2-TNATA. A hole transport layer having a thickness of 300 Å was deposited on the hole injection layer by placing the following N,N'-bis(α-naphthyl)-N,N'-diphenyl-4,4'-diamine (NPB) in another cell in the vacuum deposition apparatus and applying current to the cell to evaporate NPB.

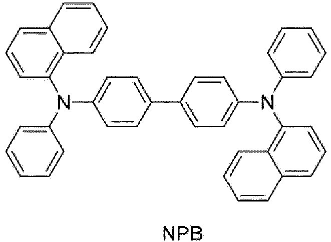

The hole injection layer and the hole transport layer were formed as described above, and then a blue light

emitting material having the following structure as a light emitting layer was deposited thereon. Specifically, a blue light emitting host material H1 was vacuum deposited to have a thickness of 200 Å on one cell in the vacuum deposition apparatus, and a blue light emitting dopant material D1 was vacuum deposited thereon in an amount of 5% based on the host material.

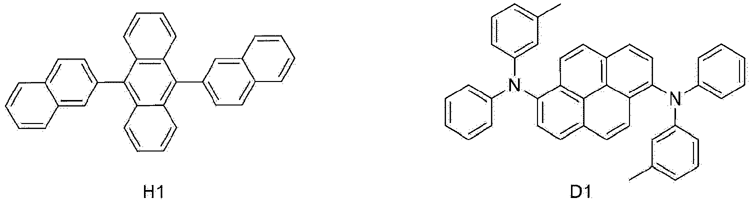

Subsequently, a compound having the following structural formula E1 as an electron transport layer was deposited to have a thickness of 300 Å.

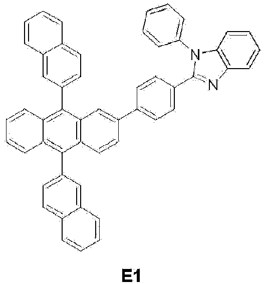

An OLED device was manufactured by depositing lithium fluoride (LiF) as an electron injection layer to have a thickness of 10 Å and allowing the Al negative electrode to have a thickness of 1,000 Å. Meanwhile, all the organic compounds required for manufacturing an OLED device were subjected to vacuum sublimed purification under 10⁻⁸ to 10⁻⁶ torr for each material, and used for the manufacture of OLED.

### <Examples 1 to 50 and Comparative Examples 2 to 11>

An organic light emitting device was manufactured in the same manner as in Comparative Example 1, except that the compound shown in the following Table 9 was used instead of E1 used when an electron transport layer was formed in Comparative Example 1.

The structures of the comparative compounds used in Comparative Examples 2 to 11 are as follows.

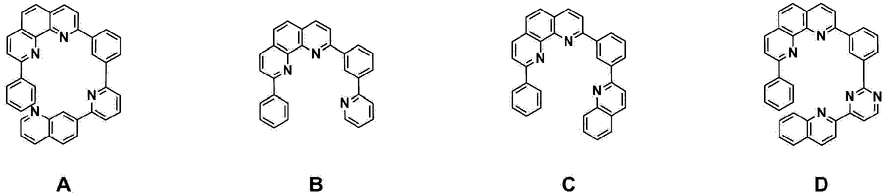

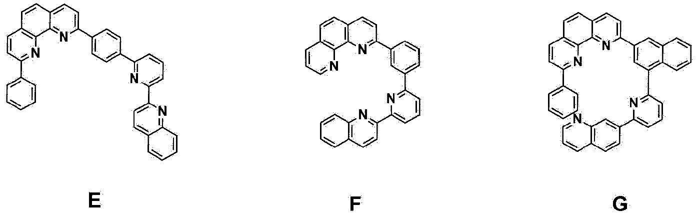

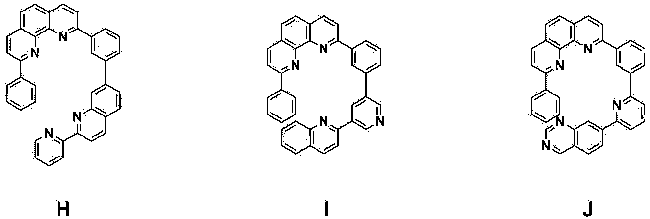

### 2) Driving Voltage and Light Emitting Efficiency of Organic Light Emitting Device

For the organic light emitting device manufactured as described above, electroluminescence (EL) characteristics were measured by M7000 manufactured by McScience Inc., and based on the measurement result thereof, T₉₅ was measured by a service life measurement device (M6000) manufactured by McScience Inc., when the reference luminance was 3,500 cd/m². The results of measuring the driving voltage, light emitting efficiency, color coordinate (CIE), and service life (T₉₅) of the blue organic electroluminescence device manufactured according to the present invention are shown as in Table 9.

**[Table 9]**

| | Compound | Driving voltage (V) | Light emitting efficiency (cd/A) | CIE (x, y) | Service life (T₉₅) |
|---|---|---|---|---|---|
| Example 1 | 1 | 5.42 | 7.84 | (0.134, 0.100) | 109 |
| Example 2 | 2 | 5.43 | 7.78 | (0.133, 0.100) | 107 |
| Example 3 | 3 | 5.44 | 7.62 | (0.133, 0.101) | 110 |
| Example 4 | 4 | 5.41 | 7.75 | (0.132, 0.101) | 101 |
| Example 5 | 6 | 5.57 | 7.67 | (0.133, 0.101) | 99 |
| Example 6 | 8 | 5.45 | 7.55 | (0.135, 0.100) | 98 |
| Example 7 | 9 | 5.55 | 7.73 | (0.130, 0.100) | 105 |
| Example 8 | 19 | 5.53 | 7.74 | (0.132, 0.102) | 111 |
| Example 9 | 21 | 5.55 | 7.82 | (0.131, 0.100) | 98 |
| Example 10 | 22 | 5.56 | 7.75 | (0.134, 0.100) | 120 |
| Example 11 | 30 | 5.44 | 7.60 | (0.133, 0.100) | 105 |
| Example 12 | 32 | 5.51 | 7. 68 | (0.132, 0.101) | 106 |
| Example 13 | 43 | 5.50 | 7.80 | (0.131, 0.103) | 109 |
| Example 14 | 57 | 5.45 | 7.75 | (0.132, 0.101) | 101 |
| Example 15 | 58 | 5.47 | 7.66 | (0.134, 0.101) | 98 |
| Example 16 | 79 | 5.53 | 7.77 | (0.134, 0.100) | 104 |
| Example 17 | 92 | 5.55 | 7.58 | (0.133, 0.099) | 101 |
| Example 18 | 113 | 5.47 | 7.80 | (0.134, 0.101) | 112 |
| Example 19 | 124 | 5.49 | 7.79 | (0.133, 0.100) | 98 |
| Example 20 | 139 | 5.51 | 7.77 | (0.132, 0.102) | 97 |
| Example 21 | 145 | 5.45 | 7.80 | (0.132, 0.101) | 113 |
| Example 22 | 146 | 5.60 | 7.63 | (0.134, 0.100) | 115 |
| Example 23 | 147 | 5.57 | 7.78 | (0.133, 0.100) | 100 |
| Example 24 | 148 | 5.50 | 7.80 | (0.134, 0.100) | 102 |
| Example 25 | 150 | 5.51 | 7.84 | (0.134, 0.101) | 99 |
| Example 26 | 156 | 5.58 | 7.70 | (0.132, 0.101) | 100 |
| Example 27 | 161 | 5.49 | 7.72 | (0.135, 0.101) | 98 |
| Example 28 | 162 | 5.50 | 7.80 | (0.130, 0.100) | 97 |
| Example 29 | 168 | 5.49 | 7.83 | (0.132, 0.101) | 96 |
| Example 30 | 170 | 5.47 | 7.81 | (0.133, 0.100) | 96 |
| Example 31 | 171 | 5.53 | 7.77 | (0.134, 0.101) | 100 |
| Example 32 | 173 | 5.55 | 7.74 | (0.134, 0.100) | 101 |
| Example 33 | 191 | 5.49 | 7.76 | (0.134, 0.101) | 99 |
| Example 34 | 209 | 5.50 | 7.80 | (0.133, 0.101) | 100 |
| Example 35 | 211 | 5.51 | 7.78 | (0.133, 0.101) | 97 |
| Example 36 | 212 | 5.65 | 7.77 | (0.132, 0.101) | 103 |
| Example 37 | 214 | 5.62 | 7.69 | (0.134, 0.100) | 105 |
| Example 38 | 232 | 5.55 | 7.73 | (0.133, 0.100) | 96 |
| Example 39 | 236 | 5.58 | 7.58 | (0.135, 0.101) | 102 |
| Example 40 | 240 | 5.54 | 7. 68 | (0.134, 0.101) | 98 |
| Example 41 | 270 | 5.68 | 7.54 | (0.134, 0.100) | 97 |
| Example 42 | 306 | 5.41 | 7.83 | (0.132, 0.101) | 95 |
| Example 43 | 307 | 5.43 | 7.80 | (0.133, 0.101) | 116 |
| Example 44 | 309 | 5.43 | 7.81 | (0.133, 0.100) | 115 |
| Example 45 | 313 | 5.47 | 7.78 | (0.132, 0.101) | 99 |
| Example 46 | 321 | 5.48 | 7.80 | (0.132, 0.101) | 121 |
| Example 47 | 323 | 5.47 | 7.77 | (0.134, 0.101) | 119 |
| Example 48 | 369 | 5.57 | 7.76 | (0.133, 0.099) | 100 |
| Example 49 | 371 | 5.63 | 7.72 | (0.134, 0.100) | 107 |
| Example 50 | 390 | 5.70 | 7.69 | (0.134, 0.100) | 96 |
| Example 51 | 401 | 5.45 | 7.82 | (0.134, 0.100) | 120 |
| Example 52 | 405 | 5.52 | 7.79 | (0.134, 0.100) | 114 |
| Example 53 | 411 | 5.42 | 7.80 | (0.134, 0.100) | 129 |
| Comparative Example 1 | E1 | 6.23 | 6.42 | (0.134, 0.100) | 50 |
| Comparative Example 2 | Comparative Compound A | 6.10 | 7.12 | (0.134, 0.101) | 67 |
| Comparative Example 3 | Comparative Compound B | 6.21 | 7.03 | (0.132, 0.101) | 64 |
| Comparative Example 4 | Comparative Compound C | 6.43 | 7.02 | (0.133, 0.100) | 69 |
| Comparative Example 5 | Comparative Compound D | 6.41 | 7.14 | (0.133, 0.100) | 68 |
| Comparative Example 6 | Comparative Compound E | 6.22 | 7.01 | (0.134, 0.101) | 71 |
| Comparative Example 7 | Comparative Compound F | 6.55 | 7.10 | (0.134, 0.102) | 62 |
| Comparative Example 8 | Comparative Compound G | 6.17 | 7.03 | (0.134, 0.102) | 72 |
| Comparative Example 9 | Comparative Compound H | 6.32 | 6. 98 | (0.134, 0.102) | 68 |
| Comparative Example 10 | Comparative Compound I | 6.44 | 7.00 | (0.134, 0.102) | 63 |
| Comparative Example 11 | Comparative Compound J | 6.36 | 6.78 | (0.134, 0.102) | 68 |

As can be seen from the results in Table 9, the organic light emitting device using an electron transport layer material of the blue organic light emitting device of the present invention has a low driving voltage and notably improved light emitting efficiency and service life compared to Comparative Examples 1 to 11.

It is determined that these results are because the skeleton of the present invention has an appropriate electron transport ability, and that the more stabilized compound can efficiently transfer electrons without decomposition or destruction of the compound due to the stable bond between the phenanthroline functional group and the metals used for the negative electrode.

In particular, Comparative Compounds A to J used in Comparative Examples 2 to 11 include a partial structure of the heterocyclic compound of the present invention, but do not satisfy Chemical Formula 1 of the present invention. Specifically, Comparative Compounds A to D and H to J have Het configurations different from those of the present invention, Comparative Compound E has an Het position different from that of the present invention, Comparative Compound F does not include the M configuration of the present invention, and Comparative Compound G includes a naphthalene ring instead of a benzene ring of the present invention.

As a result, it could be confirmed that even though a partial structure is equally adopted in the heterocyclic compound structure of the present invention, when the overall configuration of the present invention is not satisfied, excellent effects cannot be provided as a device compared to the heterocyclic compound of the present invention.

When specific values are compared, in the case of Comparative Examples 2 to 11, which are not included in the scope of the present invention, but use compounds including similar structures, the light emitting efficiency and service life were somewhat improved compared to Comparative Example 1, which used Compound E1, but the maximum light emitting efficiency was 7.12 cd/A and the maximum service time was only 72.

In contrast, in the case of Examples 1 to 53 using the compound of the present invention, the light emitting efficiency was 7.54 to 7.84 cd/A and the service life was 95 to 129, and the light emitting efficiency was increased by a minimum of 17% and the service life was increased by a minimum of 1.9-fold compared to the material in the related art (Comparative Example 1). Further, it can be seen that compared to the groups in the Comparative Examples, the light emitting efficiency was improved by a minimum of 5% and the service life was improved by a minimum of 30%.

Therefore, it is determined that the heterocyclic compound of the present invention satisfies all of the configurations of Chemical Formula 1, and thus has improved electron-transport properties and stability compared to similar structures, resulting in excellence in all aspects of driving voltage, efficiency, and service life.

### <Experimental Example 2>

### 1) Manufacture of Organic Light Emitting Device

A glass substrate, in which ITO was thinly coated to have a thickness of 1500 Å, was ultrasonically washed with distilled water. When the washing with distilled water is finished, the glass substrate was ultrasonically washed with a solvent such as acetone, methanol, and isopropyl alcohol, was dried and then was subjected to UVO treatment for 5 minutes by using UV in a UV washing machine. Thereafter, the substrate was transferred to a plasma washing machine (PT), and then was subjected to plasma treatment in order to implement an ITO work function and remove a residual film in a vacuum state, and thus, was transferred to a thermal deposition equipment for organic deposition.

An organic material having a 2-stack white organic light device (WOLED) structure was formed on the ITO transparent electrode (positive electrode). For a first stack, a hole transport layer was first formed by thermally vacuum depositing TAPC to have a thickness of 300 Å. The hole transport layer was formed, and then a light emitting layer was thermally vacuum deposited thereon as follows. The light emitting layer was deposited to have a thickness of 300 Å by doping a host TCz1 with a blue phosphorescent dopant FIrpic at a concentration of 8%. An electron transport layer was formed to have a thickness of 400 Å by using TmPyPB, and then a charge generation layer was formed to have a thickness of 100 Å by doping the compound described in the following Table 10 with Cs₂CO₃ at a concentration of 20%.

For a second stack, a hole injection layer was first formed by thermally vacuum depositing MoO₃ to have a thickness of 50 Å. A hole transport layer, which is a common layer, was formed to have a thickness of 100 Å by doping TAPC with MoO₃ at a concentration of 20%, and then formed by depositing TAPC to have a thickness of 300 Å. A light emitting layer was deposited thereon to have a thickness of 300 Å by doping a host TCz1 with a green phosphorescent dopant Ir(ppy)₃ at a concentration of 8%, and then formed to have a thickness of 600 Å using TmPyPB as an electron transport layer. Finally, lithium fluoride (LiF) was deposited to have a thickness of 10 Å on the electron transport layer to form an electron injection layer, and then an aluminum (Al) negative electrode was deposited to have a thickness of 1,200 Å on the electron injection layer to form a negative electrode, thereby manufacturing an organic electroluminescence device.

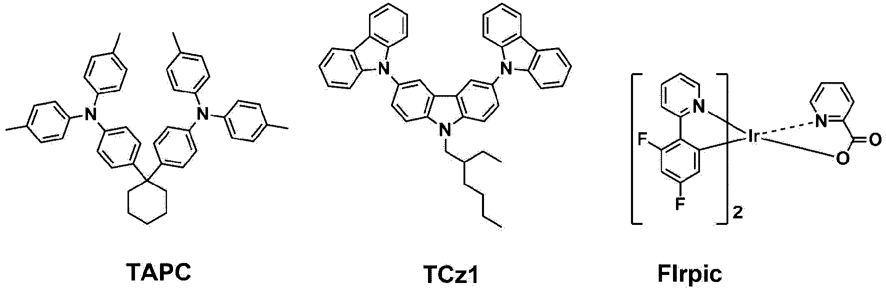

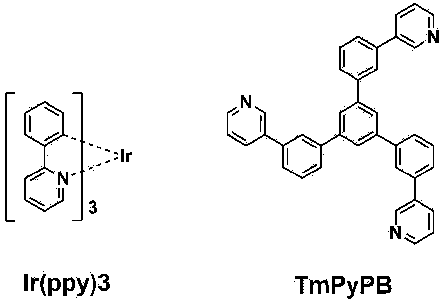

The comparative compounds in the following Table 10 have the same structures as those in Experimental Example 1 above.

Meanwhile, all the organic compounds required for manufacturing an OLED device were subjected to vacuum sublimed purification under 10⁻⁸ to 10⁻⁶ torr for each material, and used for the manufacture of the OLED.

### 2) Driving Voltage and Light Emitting Efficiency of Organic Light Emitting Device

For the organic electroluminescence device manufactured as described above, electroluminescence (EL) characteristics were measured by M7000 manufactured by McScience Inc., and based on the measurement result thereof, T₉₅ was measured by a service life measurement device (M6000) manufactured by McScience Inc., when the reference luminance was 3,500 cd/m². The results of measuring the driving voltage, light emitting efficiency, color coordinate (CIE), and service life (T₉₅) of the white organic electroluminescence device manufactured according to the present invention are shown as in Table 10.

**[Table 10]**

| | Compound | Driving voltage (V) | Light emitting efficiency (cd/A) | CIE (x, y) | Service life (T₉₅) |
|---|---|---|---|---|---|
| Example 54 | 1 | 6.93 | 73.60 | (0.212, 0.430) | 112 |
| Example 55 | 2 | 7.00 | 73.48 | (0.211, 0.428) | 110 |
| Example 56 | 3 | 7.12 | 73.21 | (0.210, 0.430) | 113 |
| Example 57 | 4 | 7.03 | 73.33 | (0.212, 0.430) | 104 |
| Example 58 | 6 | 7.04 | 73.45 | (0.210, 0.430) | 101 |
| Example 59 | 8 | 7.10 | 73.51 | (0.213, 0.428) | 100 |
| Example 60 | 9 | 7.01 | 73.61 | (0.210, 0.425) | 110 |
| Example 61 | 19 | 7.10 | 72.88 | (0.214, 0.427) | 109 |
| Example 62 | 21 | 6.95 | 73.70 | (0.208, 0,424) | 102 |
| Example 63 | 22 | 7.14 | 72.80 | (0.210, 0.430) | 121 |
| Example 64 | 30 | 7.34 | 73.13 | (0.213, 0.426) | 105 |
| Example 65 | 32 | 7.25 | 72.30 | (0.212, 0.425) | 107 |
| Example 66 | 43 | 7.00 | 73.83 | (0.210, 0.425) | 110 |
| Example 67 | 57 | 7.04 | 73.03 | (0.210, 0.425) | 100 |
| Example 68 | 58 | 7.13 | 73.11 | (0.211, 0.428) | 99 |
| Example 69 | 79 | 7.32 | 73.53 | (0.210, 0.430) | 104 |
| Example 70 | 92 | 7.11 | 72.67 | (0.212, 0.428) | 108 |
| Example 71 | 113 | 7.20 | 73.23 | (0.211, 0.429) | 116 |
| Example 72 | 124 | 7.11 | 72.80 | (0.210, 0.430) | 100 |
| Example 73 | 139 | 7.01 | 73.01 | (0.214, 0.430) | 99 |
| Example 74 | 145 | 7.02 | 73.61 | (0.215, 0.425) | 112 |
| Example 75 | 146 | 7.12 | 73.25 | (0.215, 0.435) | 115 |
| Example 76 | 147 | 7.06 | 72.96 | (0.213, 0.430) | 101 |
| Example 77 | 148 | 7.21 | 72.64 | (0.214, 0.425) | 103 |
| Example 78 | 150 | 7.24 | 72.78 | (0.212, 0.430) | 102 |
| Example 79 | 156 | 7.33 | 73.23 | (0.213, 0.428) | 101 |
| Example 80 | 161 | 7.08 | 73.52 | (0.210, 0.428) | 100 |
| Example 81 | 162 | 6.98 | 73.13 | (0.219, 0.425) | 105 |
| Example 82 | 168 | 7.02 | 72.55 | (0.210, 0.428) | 99 |
| Example 83 | 170 | 7.00 | 72.51 | (0.210, 0.430) | 98 |
| Example 84 | 171 | 7.13 | 73.13 | (0.210, 0.425) | 102 |
| Example 85 | 173 | 7.20 | 73.01 | (0.212, 0.430) | 106 |
| Example 86 | 191 | 7.21 | 72.88 | (0.212, 0.428) | 99 |
| Example 87 | 209 | 6.99 | 73.43 | (0.213, 0.430) | 107 |
| Example 88 | 211 | 7.12 | 72.73 | (0.210, 0.430) | 100 |
| Example 89 | 212 | 7.30 | 73.00 | (0.210, 0.428) | 103 |
| Example 90 | 214 | 7.33 | 72.74 | (0.210, 0.428) | 103 |
| Example 91 | 232 | 7.13 | 72.71 | (0.208, 0,424) | 98 |
| Example 92 | 236 | 7.11 | 72.95 | (0.214, 0.427) | 101 |
| Example 93 | 240 | 7.32 | 72.98 | (0.210, 0.428) | 99 |
| Example 94 | 270 | 7.01 | 72.54 | (0.210, 0.425) | 97 |
| Example 95 | 306 | 7.10 | 73.46 | (0.212, 0.430) | 96 |
| Example 96 | 307 | 7.05 | 73.51 | (0.211, 0.429) | 110 |
| Example 97 | 309 | 7.07 | 72.88 | (0.212, 0.428) | 113 |
| Example 98 | 313 | 7.01 | 72.93 | (0.212, 0.428) | 100 |
| Example 99 | 321 | 7.11 | 73.21 | (0.212, 0.430) | 123 |
| Example 100 | 323 | 7.00 | 73.59 | (0.210, 0.428) | 120 |
| Example 101 | 369 | 7.21 | 73.23 | (0.214, 0.427) | 103 |
| Example 102 | 371 | 7.10 | 73.00 | (0.210, 0.430) | 107 |
| Example 103 | 390 | 7.23 | 72.70 | (0.210, 0.425) | 103 |
| Example 104 | 401 | 6.87 | 73.70 | (0.210, 0.425) | 119 |
| Example 105 | 405 | 7.10 | 73.01 | (0.210, 0.425) | 114 |
| Example 106 | 411 | 6.99 | 73.42 | (0.210, 0.425) | 120 |
| Comparative Example 12 | TmPyPB | 7.62 | 70.17 | (0.210, 0.425) | 72 |
| Comparative Example 13 | Comparative Compound A | 7.58 | 70.42 | (0.210, 0.430) | 73 |
| Comparative Example 14 | Comparative Compound B | 7.71 | 70.89 | (0.212, 0.430) | 69 |
| Comparative Example 15 | Comparative Compound C | 7.55 | 70.05 | (0.210, 0.428) | 72 |
| Comparative Example 16 | Comparative Compound D | 7.52 | 69.74 | (0.210, 0.428) | 72 |
| Comparative Example 17 | Comparative Compound E | 7.54 | 69.58 | (0.212, 0.430) | 68 |
| Comparative Example 18 | Comparative Compound F | 7.57 | 68.32 | (0.211, 0.429) | 71 |
| Comparative Example 19 | Comparative Compound G | 7.51 | 70.11 | (0.210, 0.428) | 64 |
| Comparative Example 20 | Comparative Compound H | 7.50 | 68.76 | (0.210, 0.428) | 62 |
| Comparative Example 21 | Comparative Compound I | 7.67 | 68.96 | (0.210, 0.428) | 70 |
| Comparative Example 22 | Comparative Compound J | 7.70 | 69.23 | (0.210, 0.428) | 74 |

As can be seen from the results in Table 10, the organic electroluminescence device using a charge generation layer material of the 2-stack white organic electroluminescence device of the present invention has a low driving voltage and improved service life and light emitting efficiency compared to Comparative Examples 12 to 22.

It is determined that these results are because the skeleton has a structure with an appropriate electron transport ability, and is composed in a form that has a phenanthroline functional group that can be bonded to a metal such as Li and Yb, used in the formation of an N-type charge generation layer.

It is though that due to these structural features, when the compound used as the N-type charge generation layer is doped with a metal, a gap state is stably formed in the N-type charge generation layer, and electrons generated from the P-type charge generation layer are easily injected into the electron transport layer through the gap state generated in the N-type charge generation layer. Therefore, it is determined that the the electrons from the P-type charge generation layer were injected into the N-type charge generation layer and subsequently transferred to the electron transport layer well, and thus the driving voltage of the organic light emitting device was lowered and the efficiency and service life were improved.

Furthermore, it was confirmed that as described above in Experimental Example 1, the comparative compounds used in Comparative Examples 12 to 22 included different configurations in only a partial structure, compared to the heterocyclic compound of the present invention, but when used as materials for a charge generation layer, the comparative compounds failed to exhibit excellent effects compared to the present invention.

As a result, it could be confirmed that even though a partial structure is equally adopted in the heterocyclic compound structure of the present invention, when the overall configuration of the present invention is not satisfied, excellent effects cannot be provided as a device compared to the heterocyclic compound of the present invention.

## Claims

1. A heterocyclic compound of the following Chemical Formula 1: 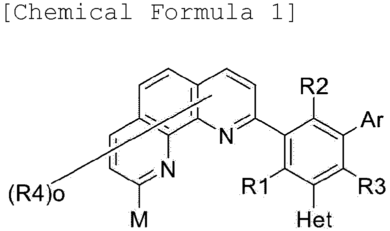 wherein, in Chemical Formula 1,
M is a substituted or unsubstituted C1 to C60 alkyl group; a substituted or unsubstituted C6 to C60 aryl group; or a substituted or unsubstituted C2 to C60 heteroaryl group,
Ar is hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
R1 to R3 are each independently hydrogen; deuterium; a halogen group; a cyano group; a substituted or unsubstituted C1 to C60 alkyl group; a substituted or unsubstituted C3 to C60 cycloalkyl group; or a substituted or unsubstituted C2 to C60 heterocycloalkyl group,
R4 is hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
o is an integer from 0 to 6, and when o is 2 or higher, R4's are the same as or different from each other,
Het is a group represented by any one of the following Chemical Formulae H-1 to H-3, 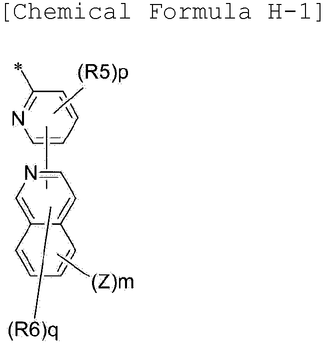 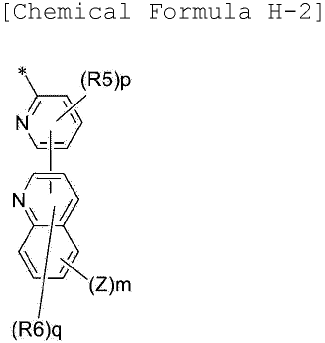 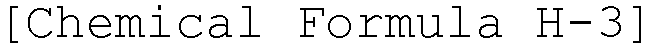 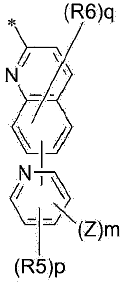
in Chemical Formulae H-1 to H-3,
Z is hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
m is an integer from 0 to 4, and when m is 2 or higher, Z's are the same as or different from each other, R5 and R6 are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, and
p is an integer from 1 to 3, q is an integer from 1 to 5, and when p and q are each 2 or higher, R5 and R6 are the same as or different from each other.

2. The heterocyclic compound of claim 1, wherein Chemical Formula 1 is represented by any one of the following Chemical Formulae 1-1 to 1-3: 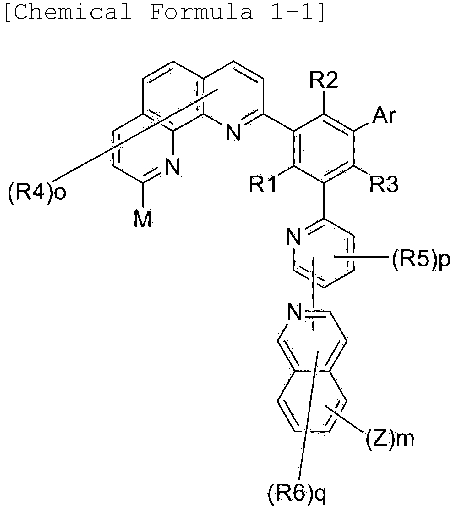 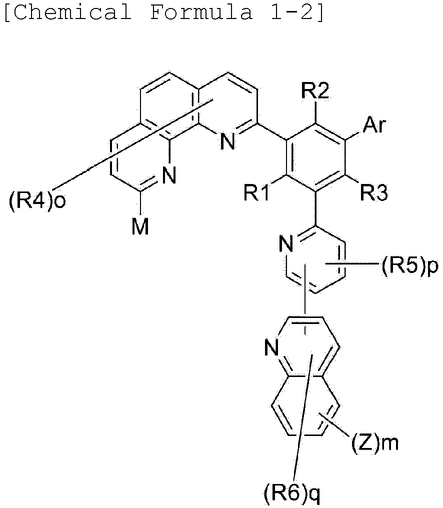 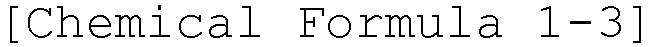 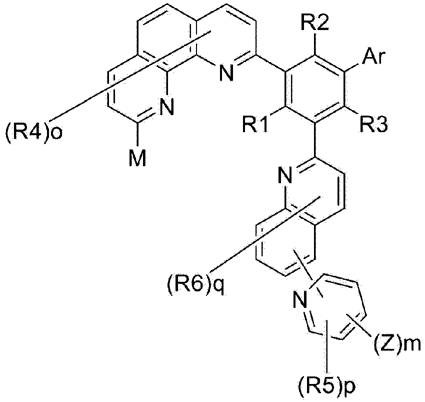 in Chemical Formulae 1-1 to 1-3,
the definition of each substituent is the same as the definition in Chemical Formula 1.

3. The heterocyclic compound of claim 1, wherein Chemical Formula 1 is represented by the following Chemical Formula 1-1-1, 1-1-2, 1-2-1, or 1-3-1: 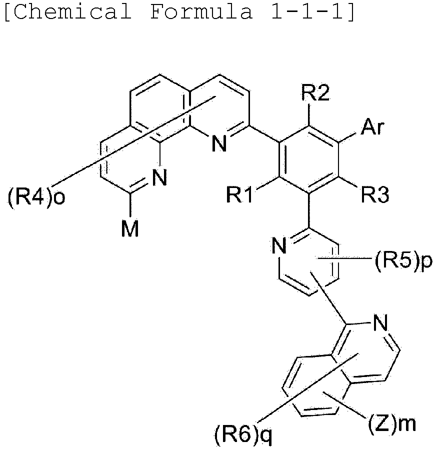 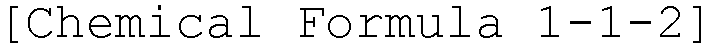 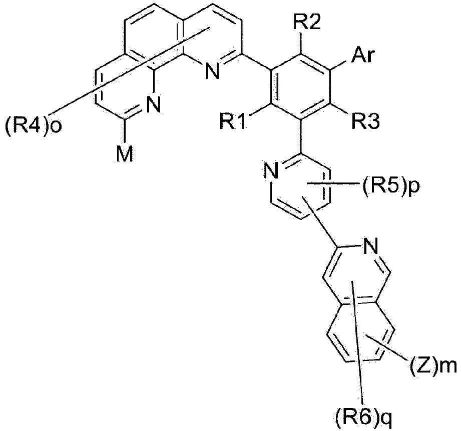 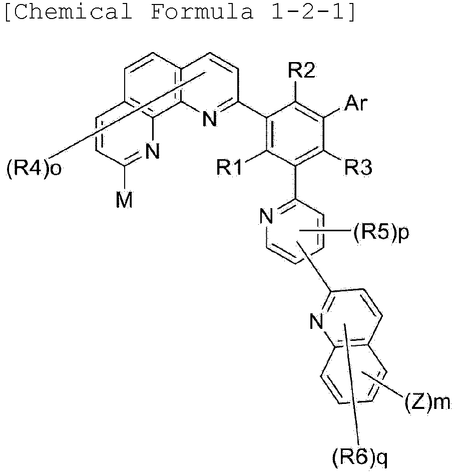 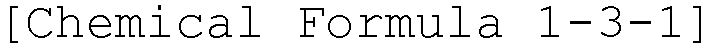 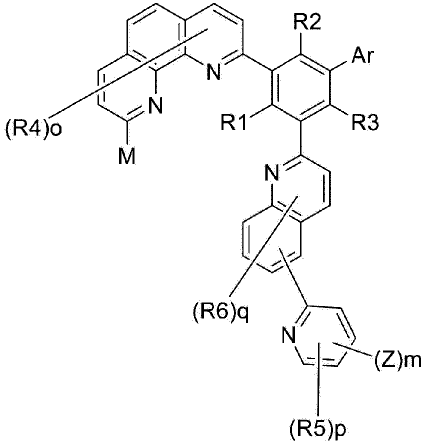 in Chemical Formulae 1-1-1, 1-1-2, 1-2-1, and 1-3-1,
the definition of each substituent is the same as the definition in Chemical Formula 1.

4. The heterocyclic compound of claim 1, wherein Ar is hydrogen; deuterium; a substituted or unsubstituted C1 to C10 alkyl group; a substituted or unsubstituted C6 to C20 aryl group; or a substituted or unsubstituted C2 to C20 heteroaryl group including O or S.

5. The heterocyclic compound of claim 1, wherein M is a substituted or unsubstituted C1 to C10 alkyl group; a substituted or unsubstituted C6 to C20 aryl group; or a substituted or unsubstituted C2 to C20 heteroaryl group.

6. The heterocyclic compound of claim 1, wherein Z is hydrogen; deuterium; a halogen group; a cyano group; a substituted or unsubstituted C1 to C10 alkyl group; a substituted or unsubstituted C6 to C20 aryl group; or a substituted or unsubstituted C2 to C20 heteroaryl group.

7. The heterocyclic compound of claim 1, wherein R1 to R3 are each independently hydrogen; or deuterium.

8. The heterocyclic compound of claim 1, wherein R4 to R6 are each independently hydrogen; or deuterium.

9. The heterocyclic compound of claim 1, wherein a deuterium substitution rate of Chemical Formula 1 is 0%, or 10% to 100%.

10. The heterocyclic compound of claim 1, wherein Chemical Formula 1 is represented by any one of the following compounds:

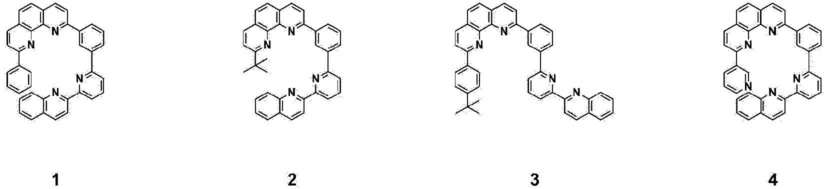

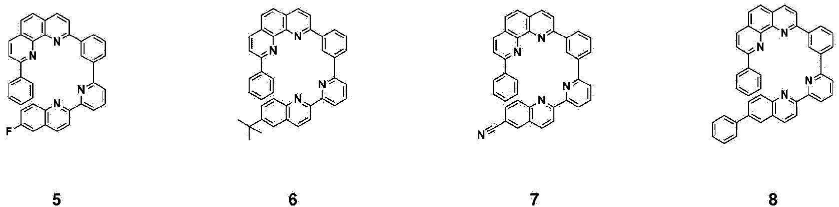

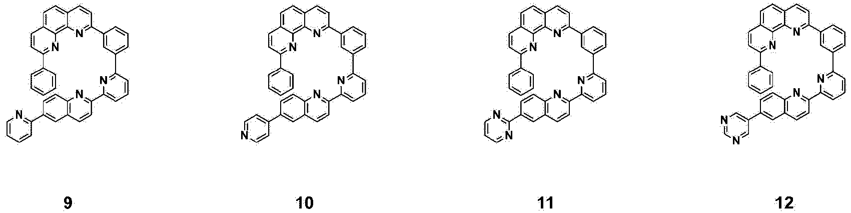

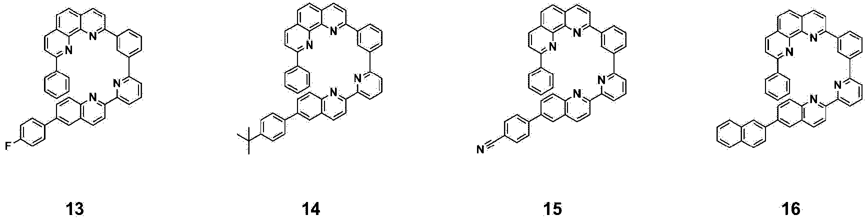

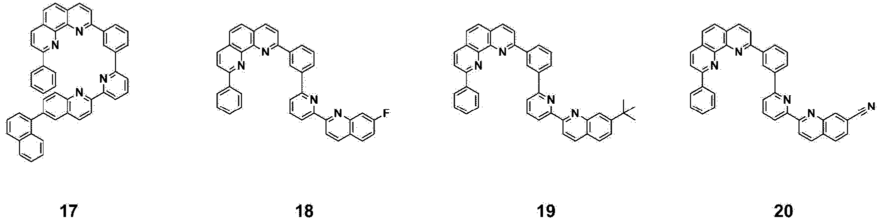

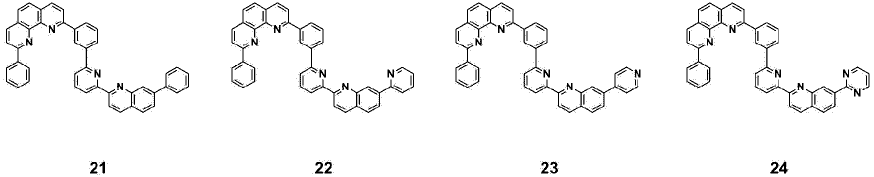

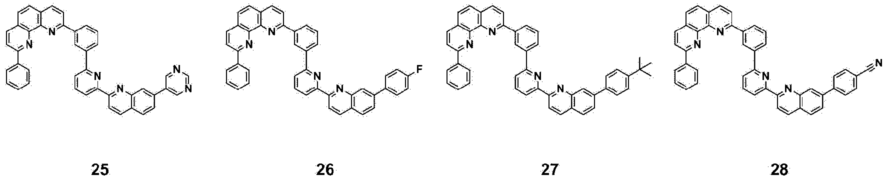

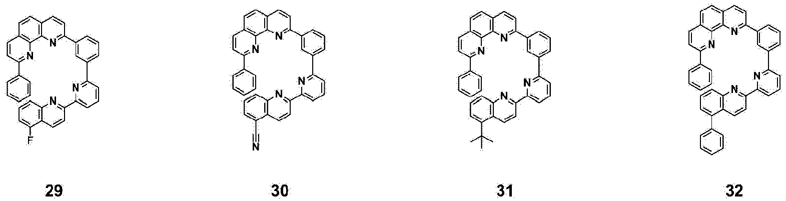

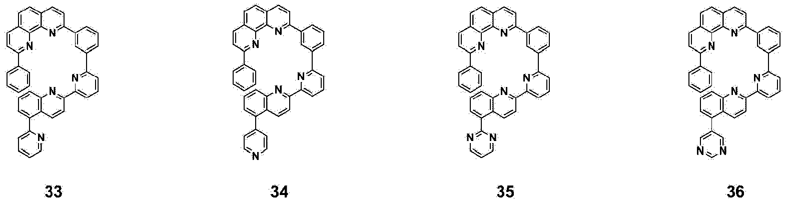

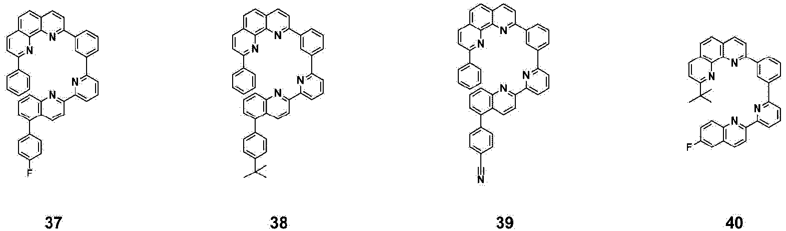

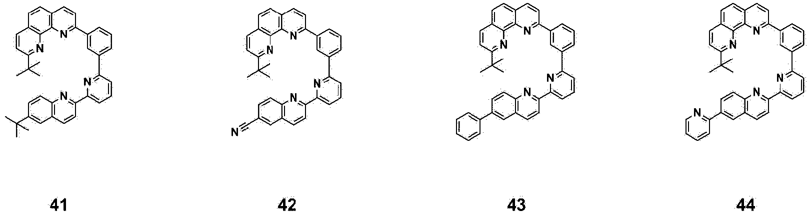

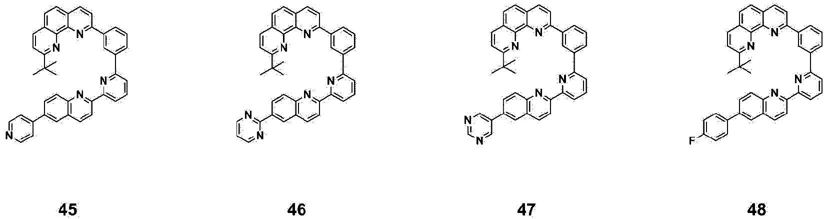

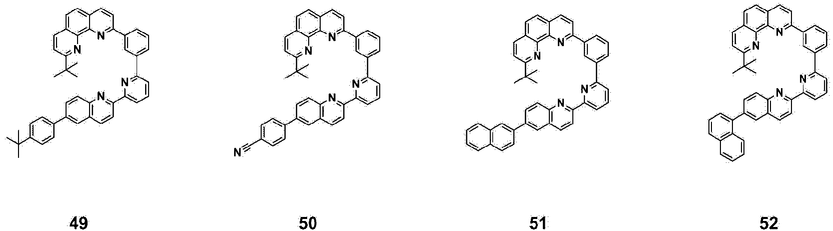

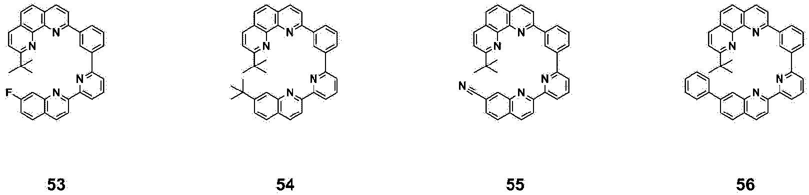

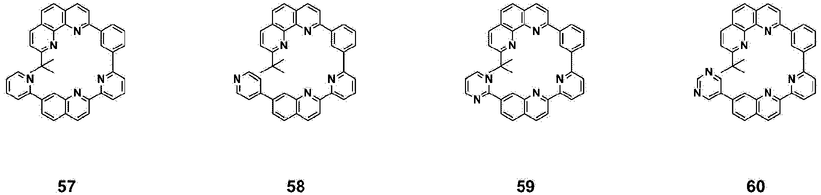

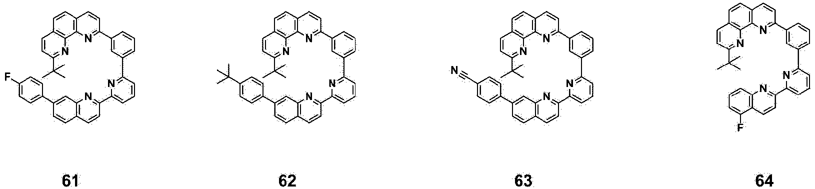

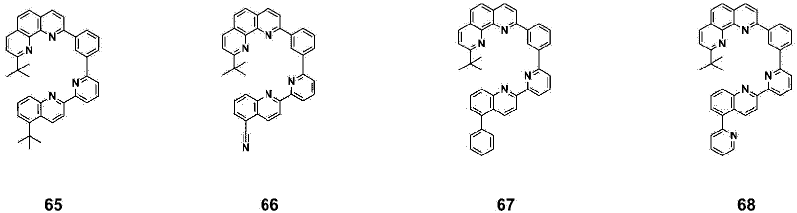

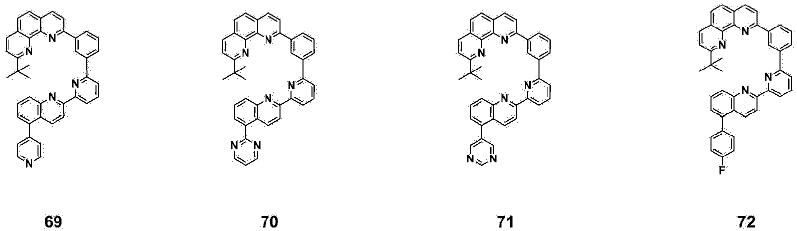

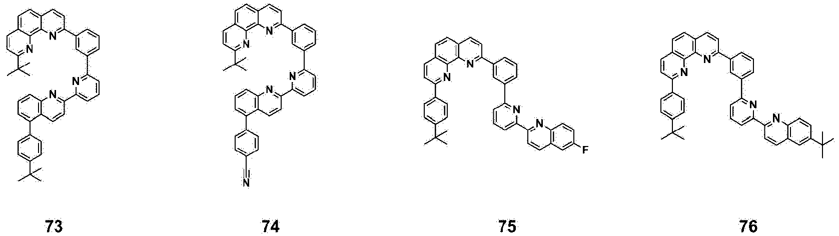

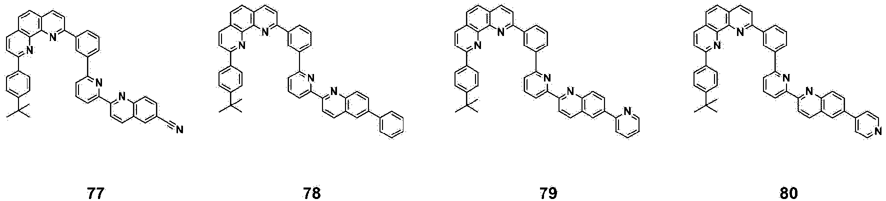

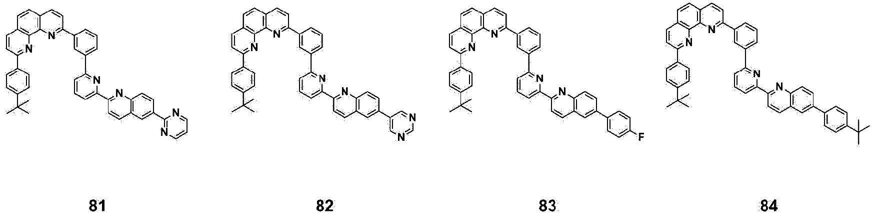

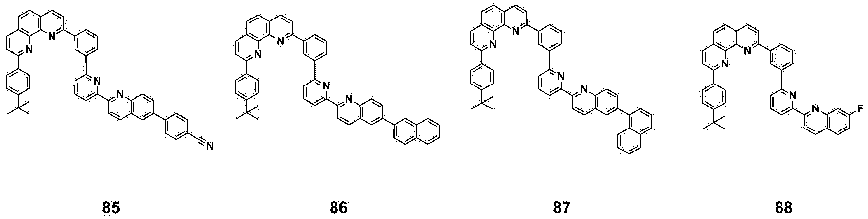

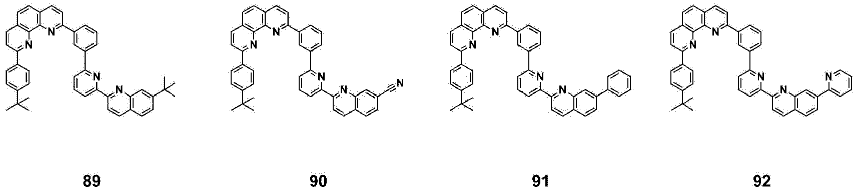

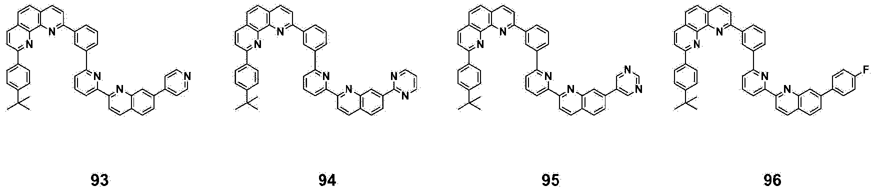

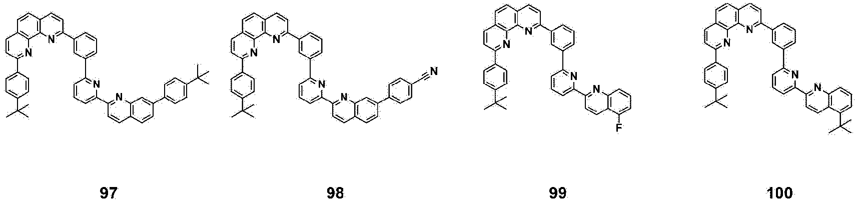

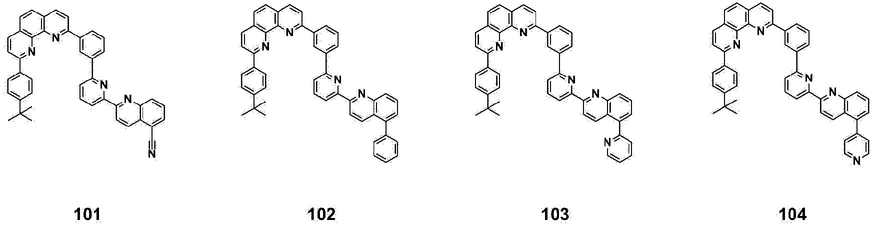

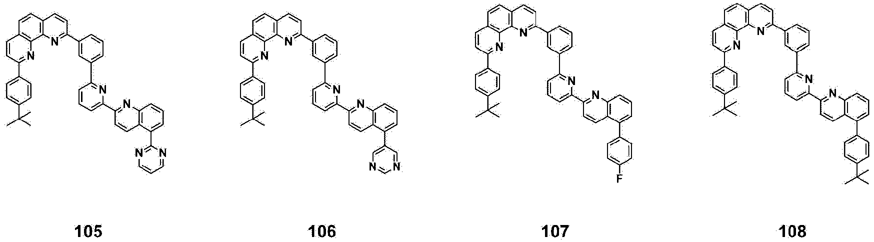

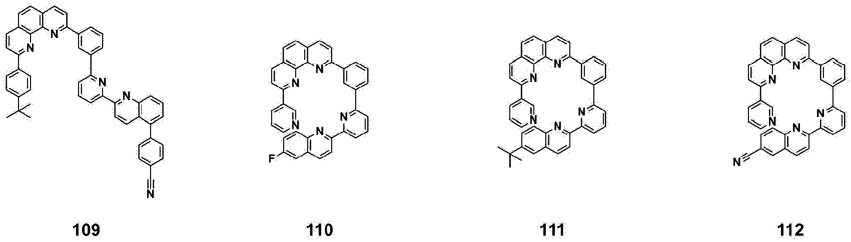

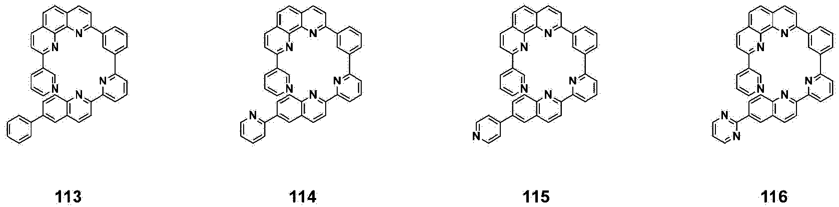

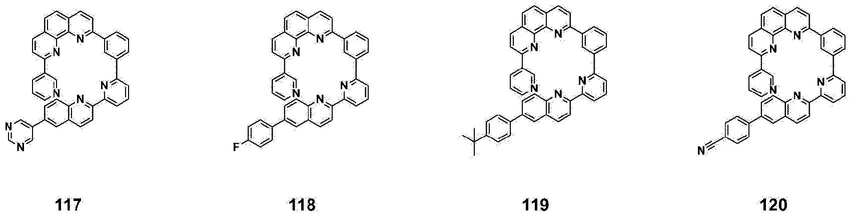

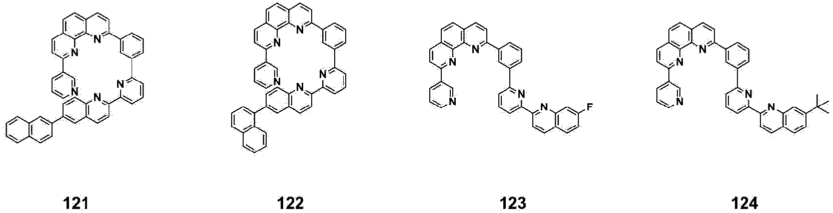

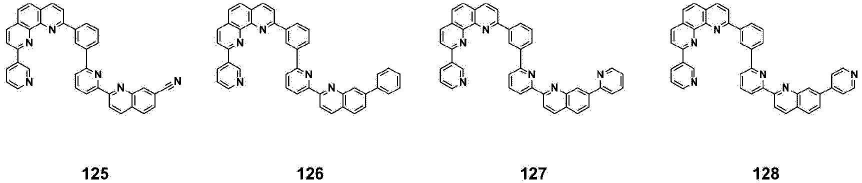

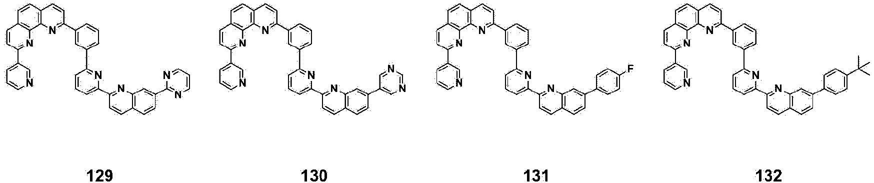

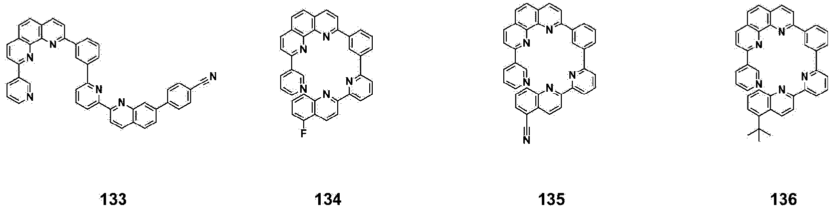

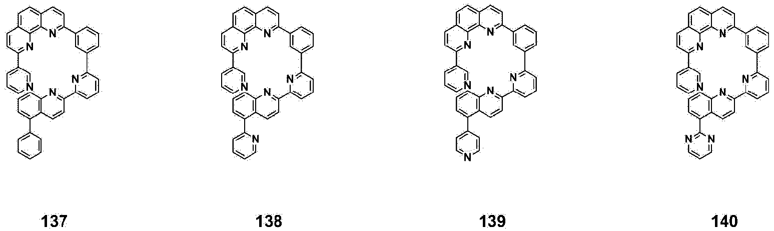

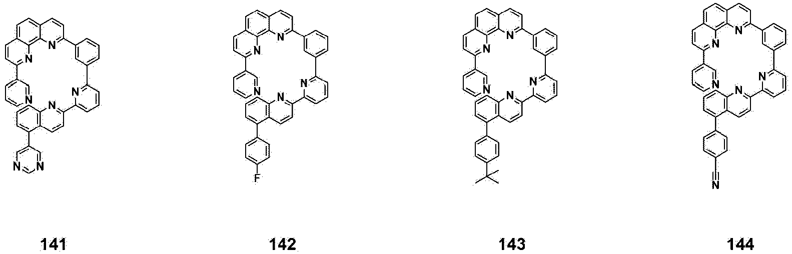

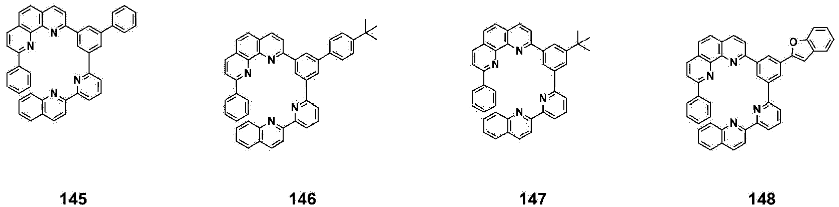

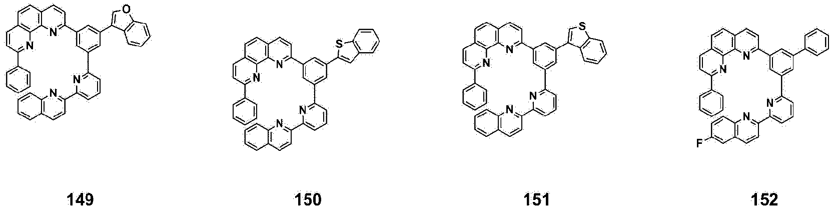

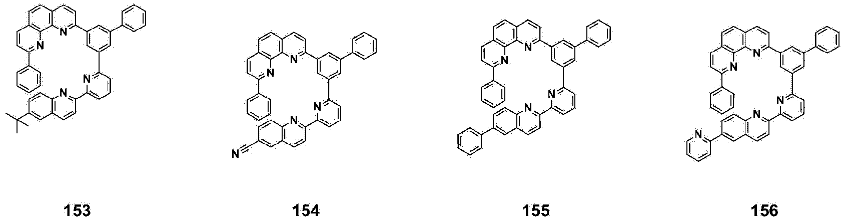

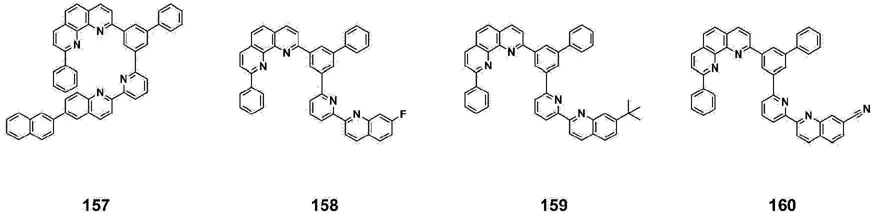

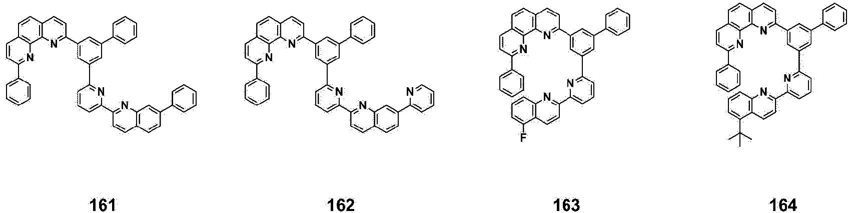

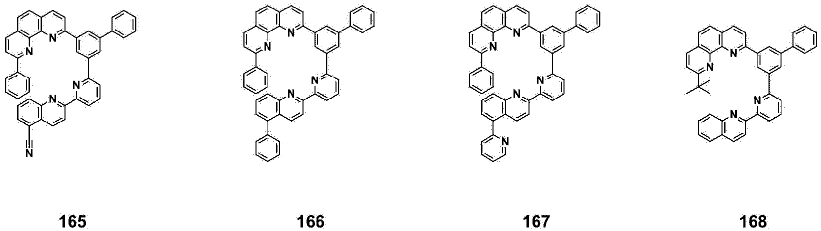

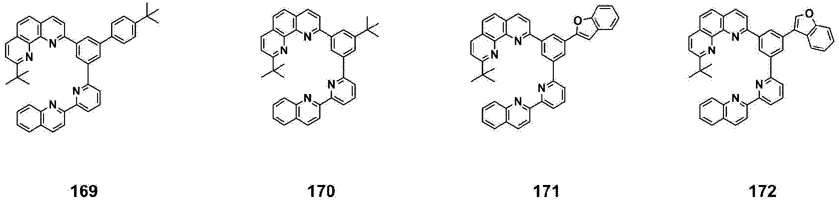

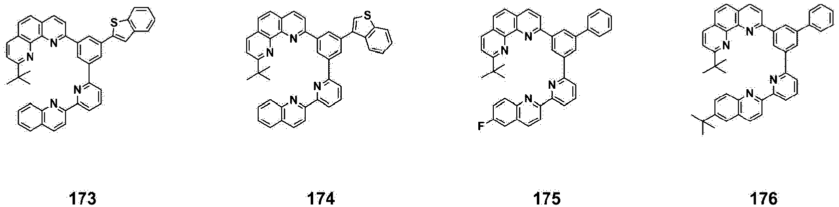

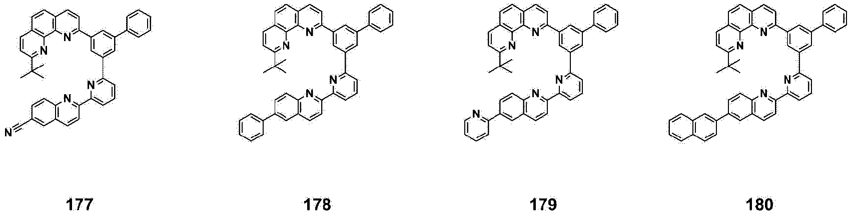

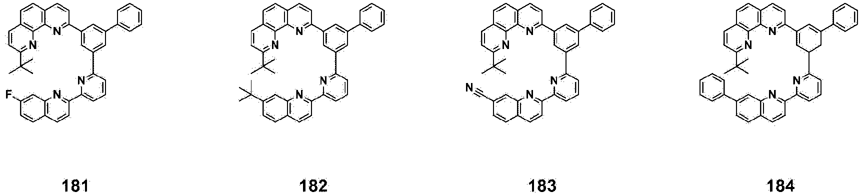

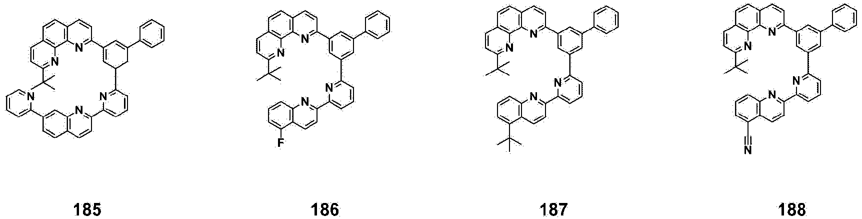

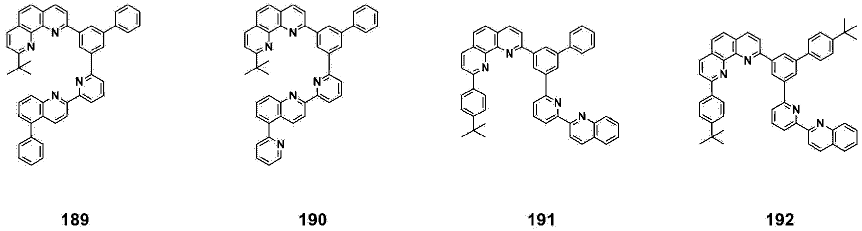

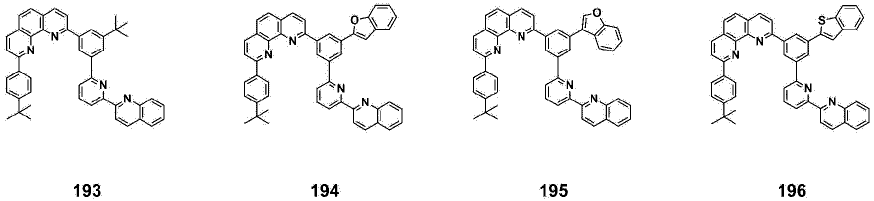

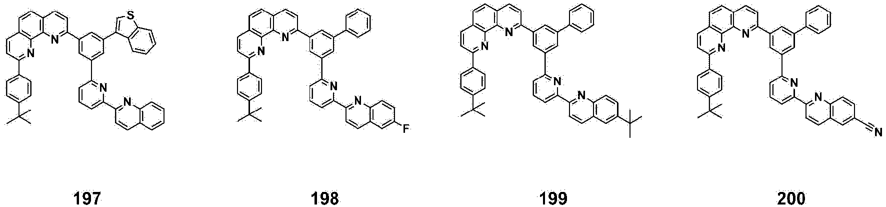

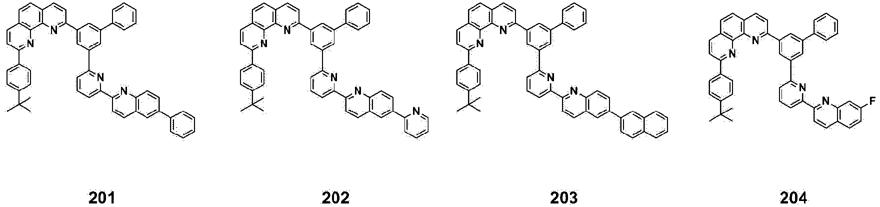

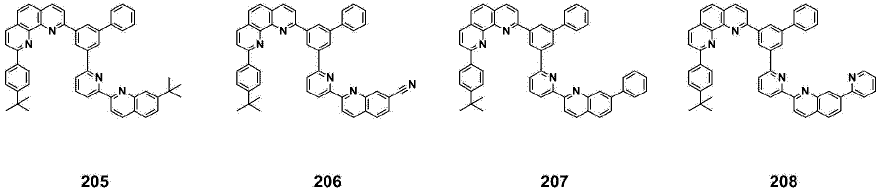

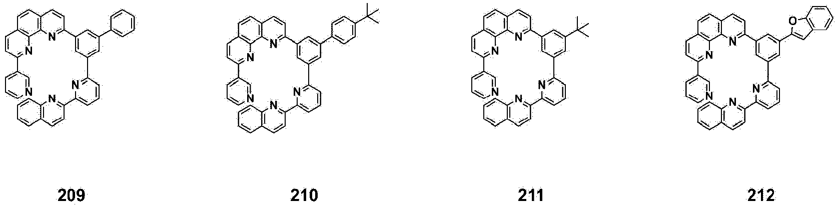

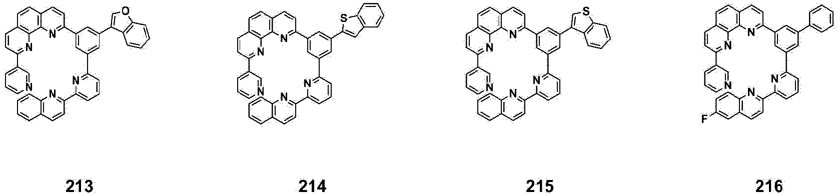

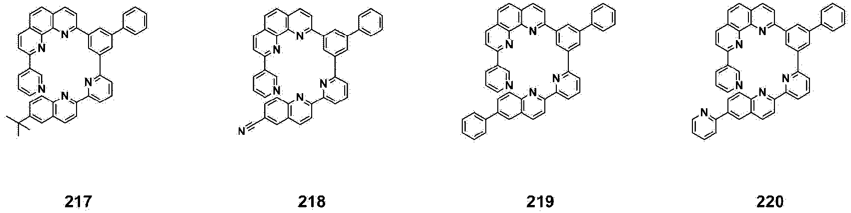

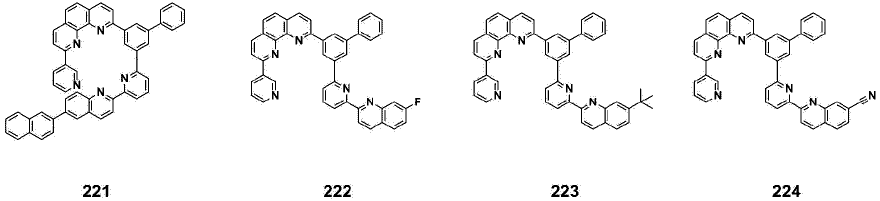

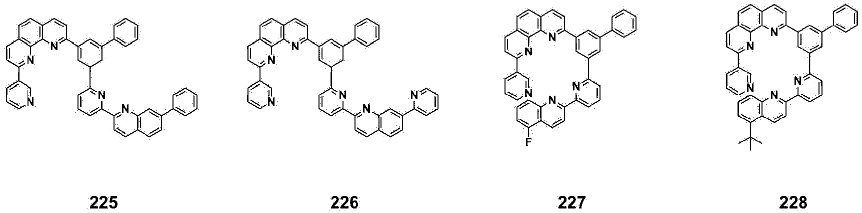

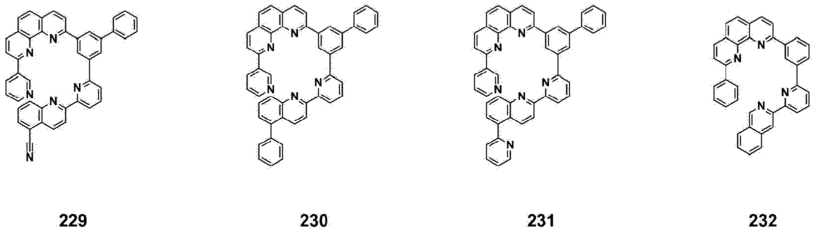

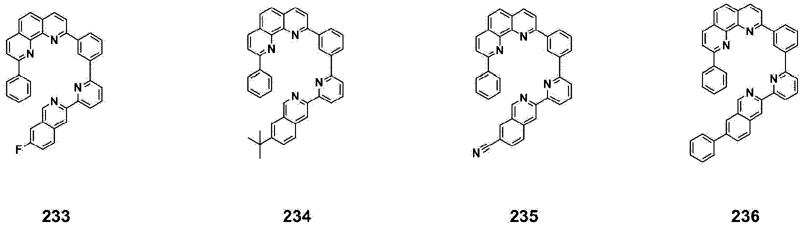

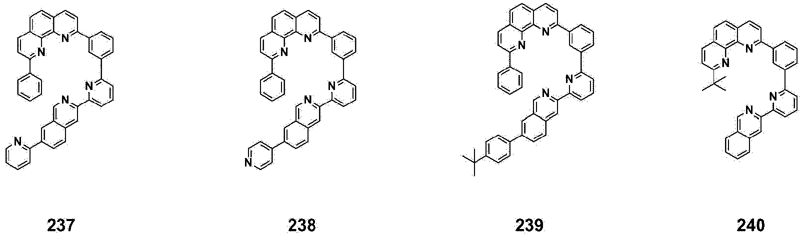

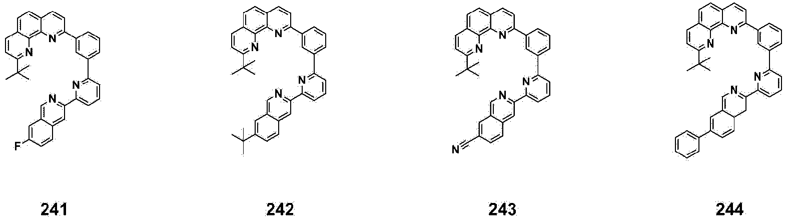

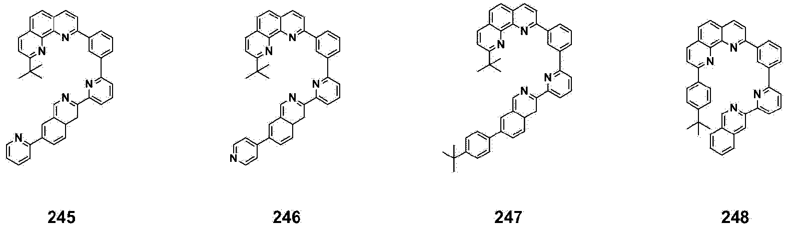

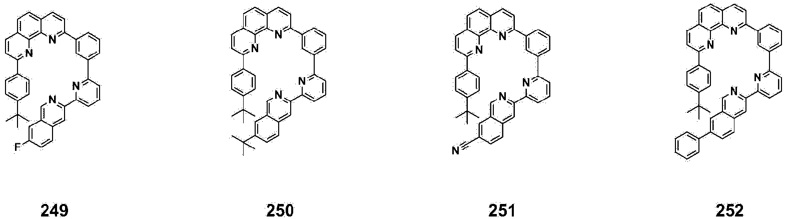

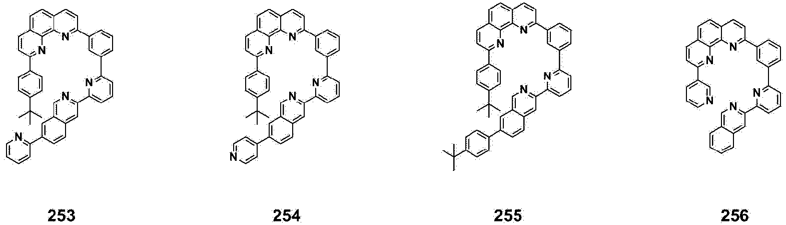

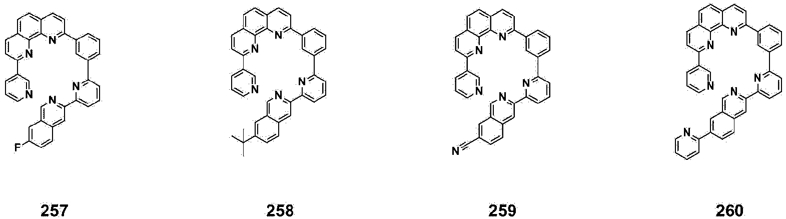

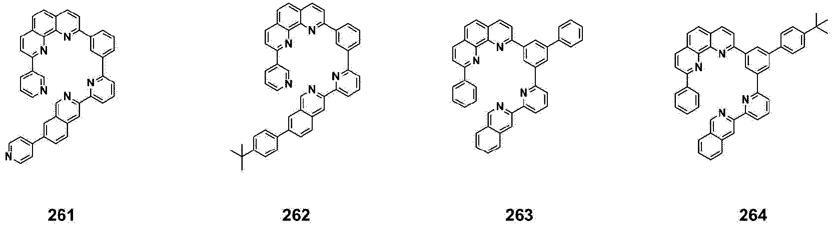

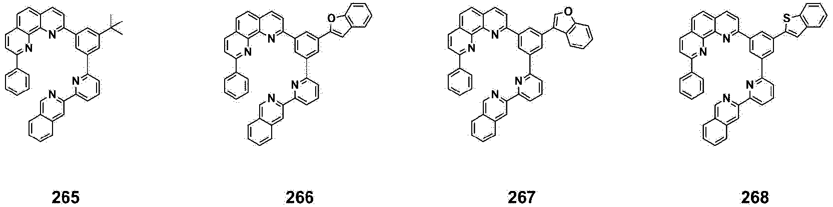

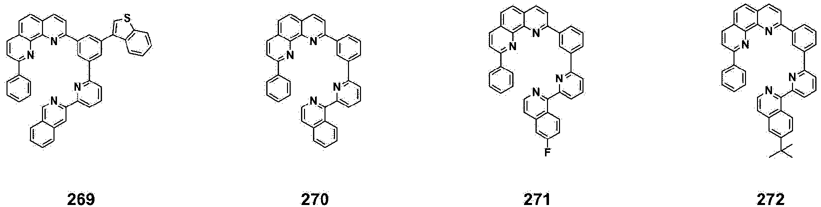

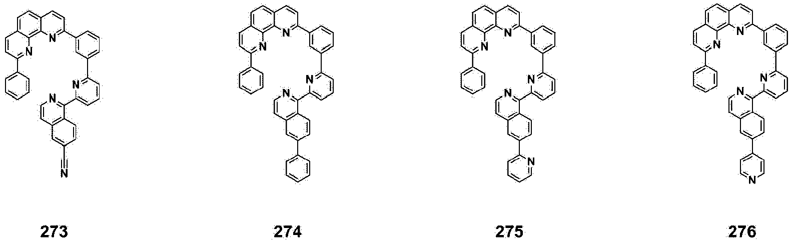

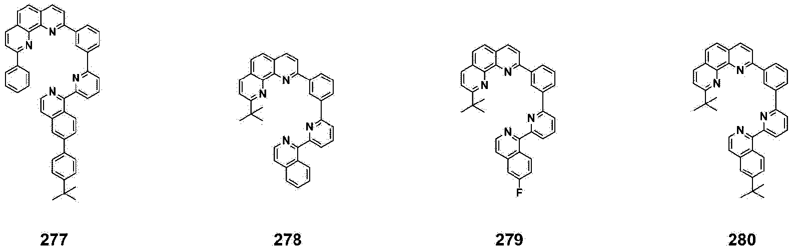

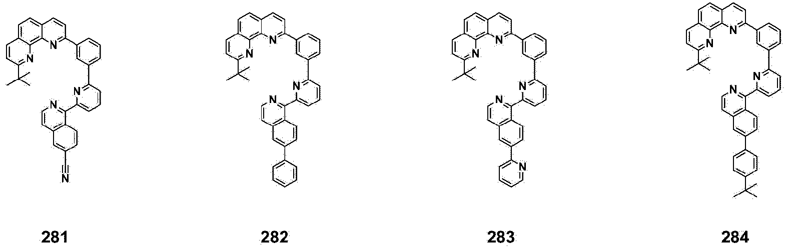

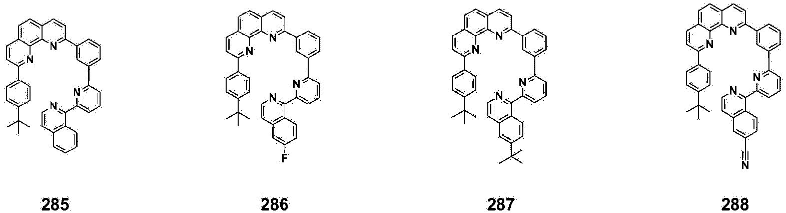

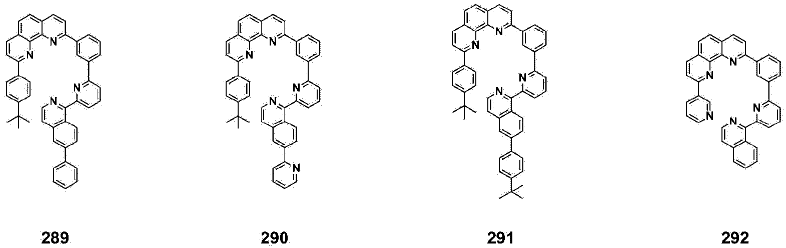

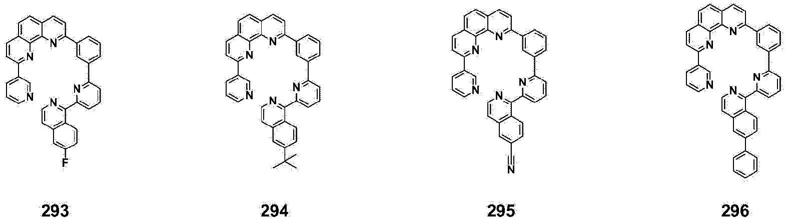

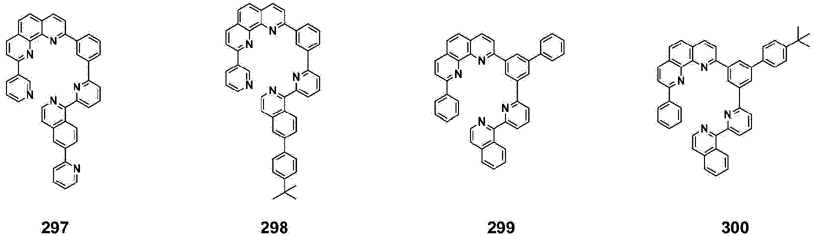

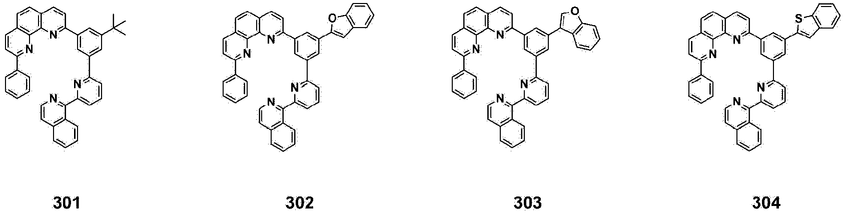

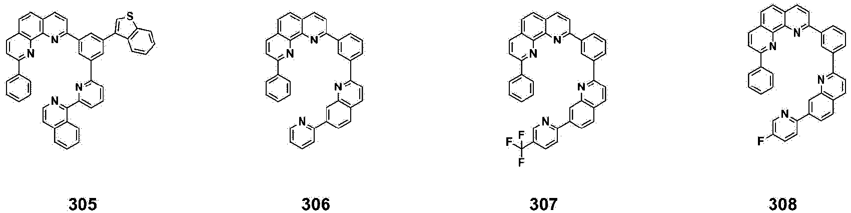

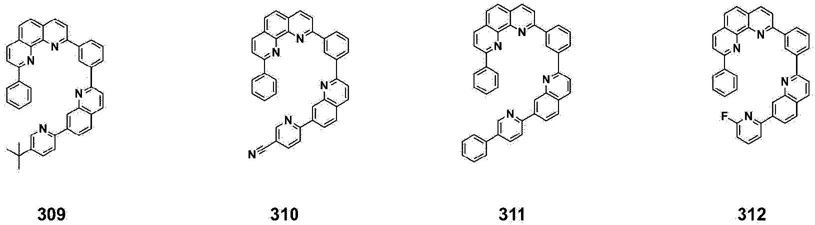

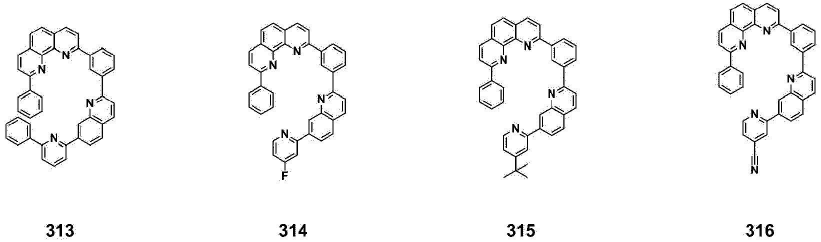

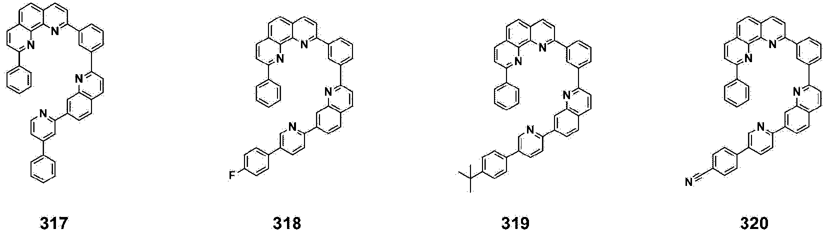

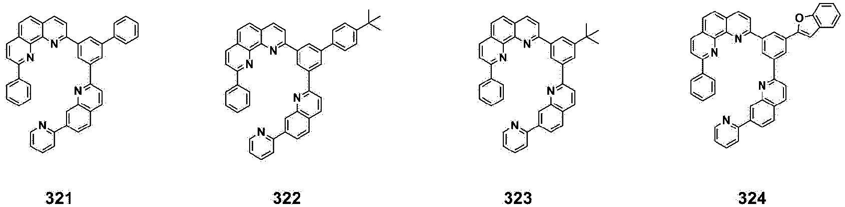

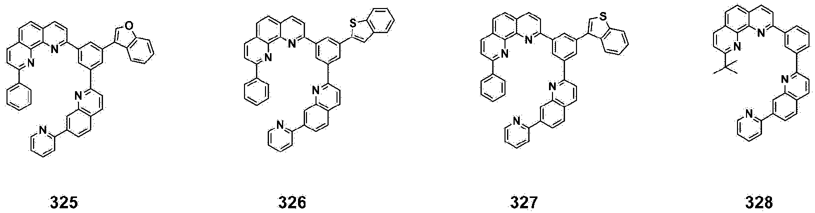

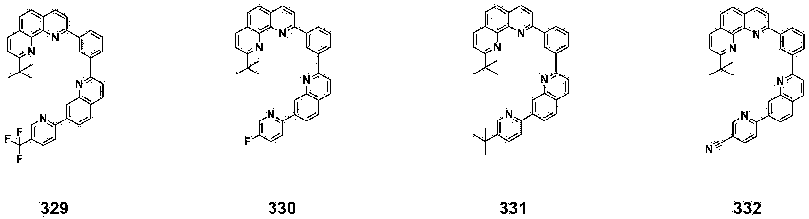

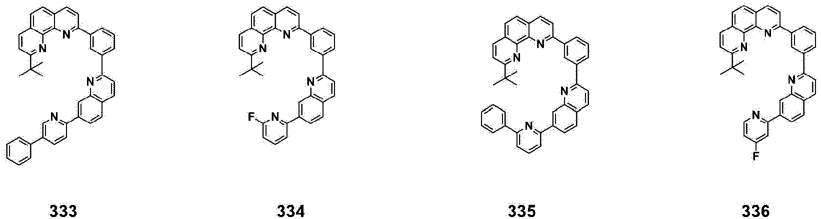

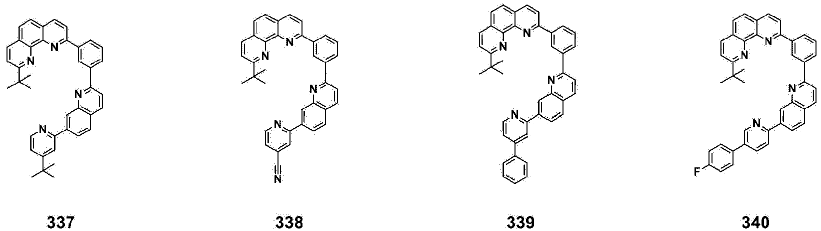

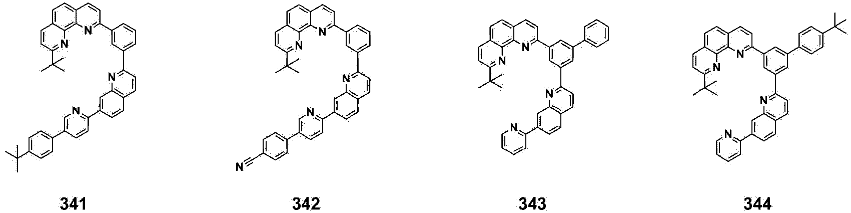

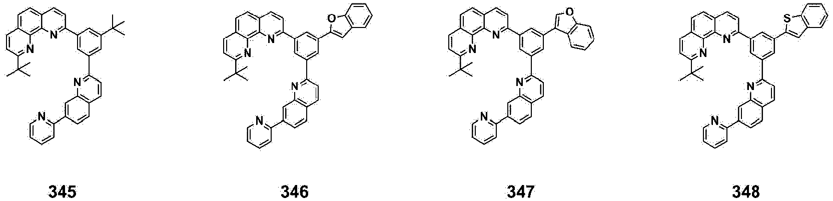

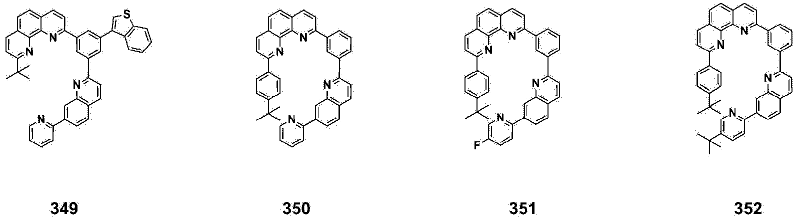

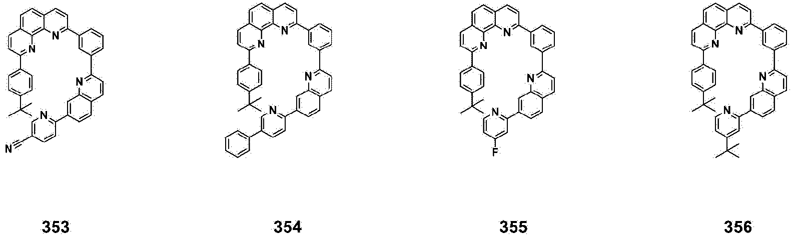

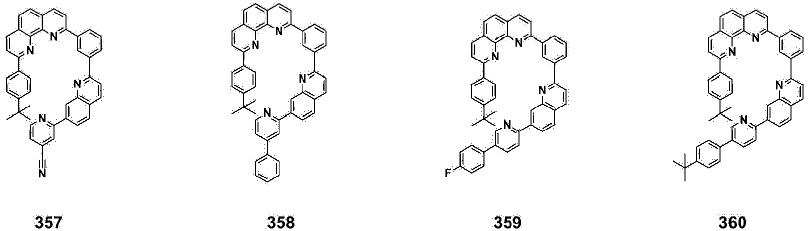

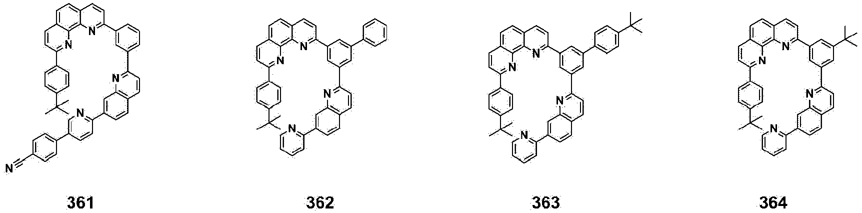

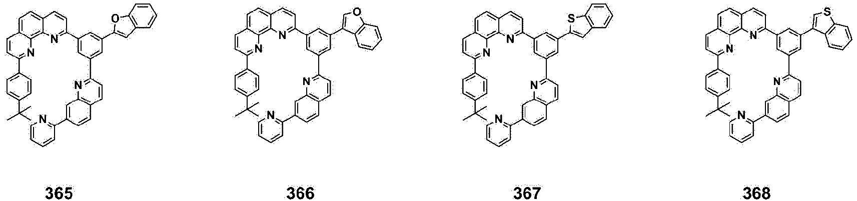

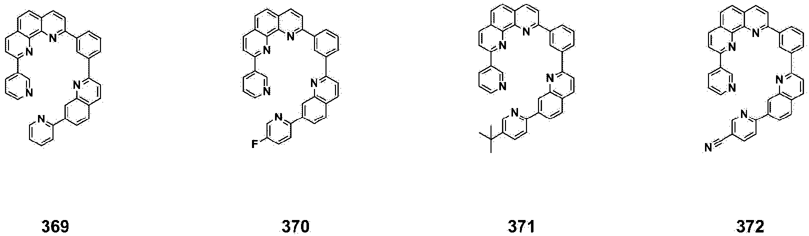

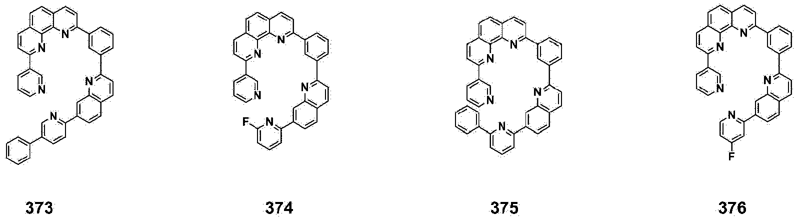

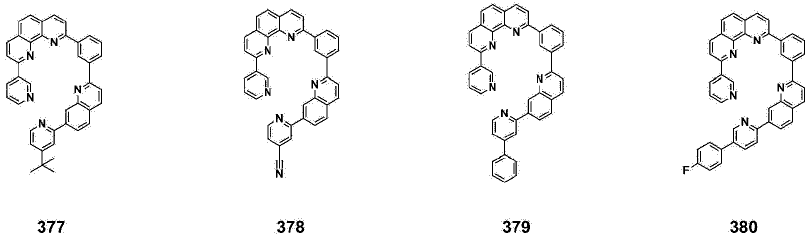

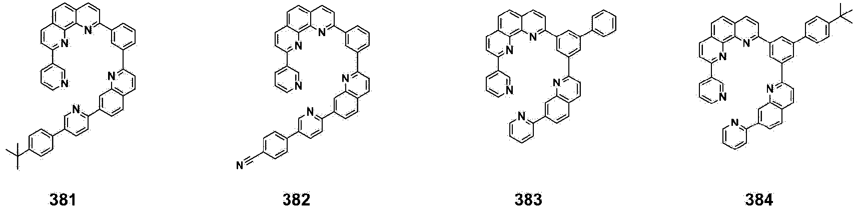

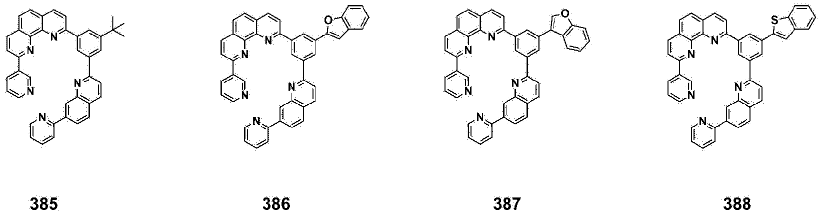

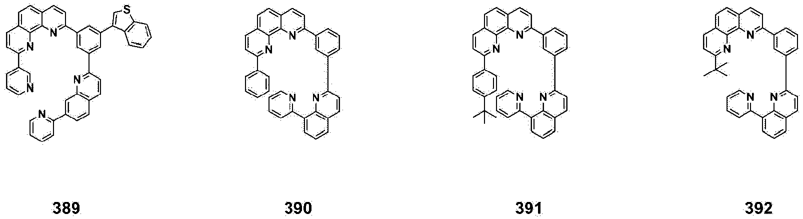

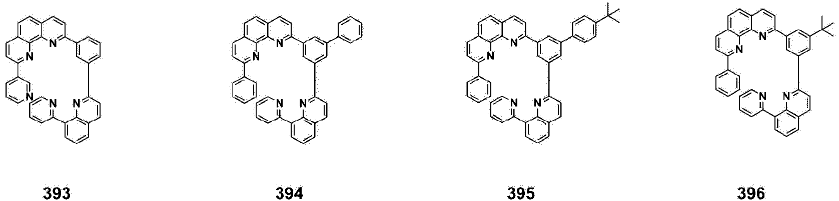

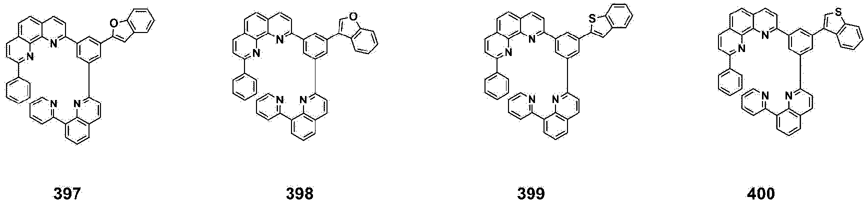

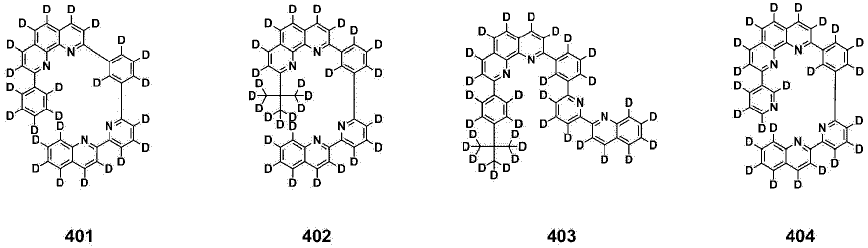

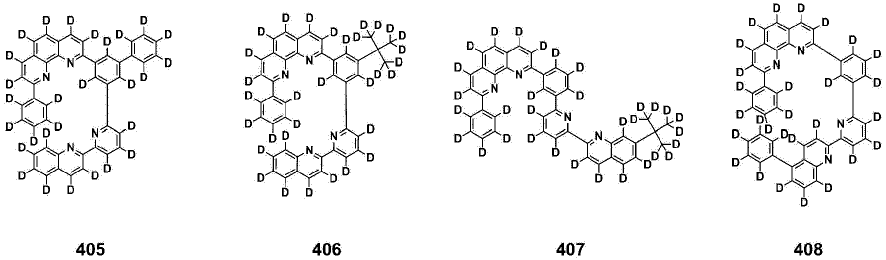

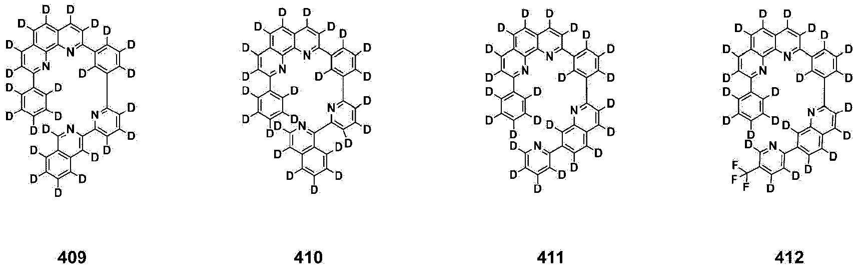

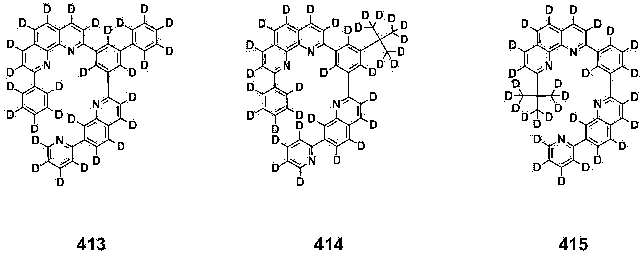

11. An organic light emitting device comprising:
a first electrode;
a second electrode; and
an organic material layer having one or more layers provided between the first electrode and the second electrode,
wherein one or more layers of the organic material layer comprise one or more of the heterocyclic compounds of any one of claims 1 to 10.

12. The organic light emitting device of claim 11, wherein the organic material layer comprises an electron transport layer, and the electron transport layer comprises one or more of the heterocyclic compounds.

13. The organic light emitting device of claim 11, wherein the organic light emitting device comprises the first electrode, a first stack provided on the first electrode and comprising a first light emitting layer, a charge generation layer provided on the first stack, a second stack provided on the charge generation layer and comprising a second light emitting layer, and a second electrode provided on the second stack, and the charge generation layer comprises one or more of the heterocyclic compounds.

14. The organic light emitting device of claim 13, wherein the charge generation layer is an N-type charge generation layer, and the N-type charge generation layer comprises one or more of the heterocyclic compounds.
